(19) Europäisches Patentamt / European Patent Office / Office européen des brevets

(11) **EP 2 307 192 B1**

(12) **EUROPÄISCHE PATENTSCHRIFT**

(45) Veröffentlichungstag und Bekanntmachung des Hinweises auf die Patenterteilung:
**02.11.2022** Patentblatt 2022/44

(21) Anmeldenummer: **09772464.5**

(22) Anmeldetag: **01.07.2009**

(51) Internationale Patentklassifikation (IPC):
**B30B 15/02** (2006.01)    **B22F 3/03** (2006.01)
**B22F 7/08** (2006.01)    **C04B 35/495** (2006.01)
**B01J 23/00** (2006.01)    **C22C 29/08** (2006.01)
**B01J 35/02** (2006.01)    **B01J 37/08** (2006.01)
**C07D 307/60** (2006.01)    **B01J 37/00** (2006.01)
**C04B 35/626** (2006.01)    **C04B 35/64** (2006.01)
**B30B 11/00** (2006.01)

(52) Gemeinsame Patentklassifikation (CPC):
(C-Sets verfügbar)
**B01J 37/0009; B01J 23/002; B01J 35/026;
B01J 37/08; B22F 3/03; B22F 7/08; B30B 11/005;
B30B 15/022; C04B 35/495; C04B 35/6261;
C04B 35/62635; C04B 35/62655; C04B 35/62685;
C04B 35/62695; C04B 35/64;**      (Forts.)

(86) Internationale Anmeldenummer:
**PCT/EP2009/058233**

(87) Internationale Veröffentlichungsnummer:
**WO 2010/000764 (07.01.2010 Gazette 2010/01)**

(54) **VERFAHREN ZUR HERSTELLUNG EINES RINGÄHNLICHEN OXIDISCHEN FORMKÖRPERS**

METHOD FOR PRODUCING A RING-SHAPED OXIDIC MOLDED BODY

PROCÉDÉ DE FABRICATION D'UN CORPS MOULÉ DE TYPE ANNULAIRE CONTENANT DES OXYDES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **02.07.2008 DE 102008040093
02.07.2008 DE 102008040094
02.07.2008 US 77638
02.07.2008 US 77601**

(43) Veröffentlichungstag der Anmeldung:
**13.04.2011** Patentblatt 2011/15

(73) Patentinhaber: **BASF SE
67056 Ludwigshafen am Rhein (DE)**

(72) Erfinder:
• **EGER, Knut**
  **67117 Limburgerhof (DE)**
• **FAUST, Jens Uwe**
  **67346 Speyer (DE)**
• **BORCHERT, Holger**
  **67591 Offstein (DE)**
• **STREIBERT, Ralf**
  **67126 Hochdorf-Assenheim (DE)**
• **MÜLLER-ENGEL, Klaus Joachim**
  **76297 Stutensee (DE)**
• **RAICHLE, Andreas**
  **01109 Dresden (DE)**

(74) Vertreter: **BASF IP Association
BASF SE
G-FLP-C006
67056 Ludwigshafen (DE)**

(56) Entgegenhaltungen:
EP-A2- 0 873 855    DE-C- 638 053
FR-A1- 2 766 743    GB-A- 1 374 952
US-A1- 2005 263 926

(52) Gemeinsame Patentklassifikation (CPC): (Forts.)
**C07D 307/60; C22C 29/08;** B01J 37/0045;
B01J 2523/00; C04B 2235/3201; C04B 2235/3256;
C04B 2235/3258; C04B 2235/3272;
C04B 2235/3275; C04B 2235/3298;
C04B 2235/3418; C04B 2235/425; C04B 2235/44;
C04B 2235/443; C04B 2235/5409;
C04B 2235/5436; C04B 2235/5463;
C04B 2235/6021; C04B 2235/604; C04B 2235/606;
C04B 2235/6562; C04B 2235/6565;
C04B 2235/6567; C04B 2235/6581;
C04B 2235/6586; C04B 2235/80; C04B 2235/94;
C22C 2204/00

C-Sets
B01J 2523/00, B01J 2523/13, B01J 2523/41,
B01J 2523/54, B01J 2523/68, B01J 2523/69,
B01J 2523/842, B01J 2523/845;
B01J 2523/00, B01J 2523/13, B01J 2523/41,
B01J 2523/54, B01J 2523/68, B01J 2523/842,
B01J 2523/845;
B01J 2523/00, B01J 2523/15, B01J 2523/17,
B01J 2523/51, B01J 2523/53, B01J 2523/55,
B01J 2523/62, B01J 2523/68

**Beschreibung**

Vorliegende Erfindung betrifft ein Verfahren zur Herstellung eines ringähnlichen oxidischen Formkörpers, umfassend das mechanische Verdichten eines in den Füllraum einer Matrize eingebrachten pulverförmigen Haufwerks aus Bestandteilen, die wenigstens eine Metallverbindung, die durch thermische Behandlung bei einer Temperatur $\geq$ 100 °C in ein Metalloxid überführbar ist, oder wenigstens ein Metalloxid, oder wenigstens ein Metalloxid und wenigstens eine solche Metallverbindung umfassen, zu einem ringähnlichen Vorläuferformkörper, bei dem sich der Füllraum in einer mit einer vertikalen Bohrachse B von oben nach unten durch das Matrizenmaterial hindurchgeführten Matrizenbohrung befindet und durch

- die Innenwand der Matrizenbohrung,

- die obere Stirnfläche eines von unten entlang der Bohrachse B in die Matrizenbohrung hub- und senkbeweglich eingeführten unteren Stempels, auf der das in den Füllraum eingebrachte pulverförmige Haufwerk aufliegt,

- die längs der Bohrachse B in einem axialen Ausgangsabstand A oberhalb der oberen Stirnfläche des unteren Stempels befindliche untere Stirnfläche eines entlang der Bohrachse B hub- und senkbeweglich angebrachten oberen Stempels, dessen untere Stirnfläche das in den Füllraum eingebrachte pulverförmige Haufwerk von oben berührt, und

- die Mantelfläche eines aus der geometrischen Mitte der oberen Stirnfläche des unteren Stempels heraus entlang der Bohrachse B in der Matrizenbohrung von unten nach oben geführten Mittelstiftes MF, der wenigstens bis zur geometrischen Mitte der unteren Stirnfläche des oberen Stempels hinaufreicht, begrenzt wird,

indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt, wobei

- die geometrische Form der Mantelfläche des unteren Stempels derjenigen der Mantelfläche eines Kreiszylinders I entspricht;

- die geometrische Form der Mantelfläche des oberen Stempels derjenigen der Mantelfläche eines Kreiszylinders II entspricht;

- in der geometrischen Mitte der oberen Stirnfläche des unteren Stempels eine von oben nach unten durch den unteren Stempel hindurchgeführte Mittelbohrung $MB^U$ ausgebildet ist;

- im Ausgangsabstand A der beiden Stirnflächen der Mittelstift MF von unten durch die Mittelbohrung $MB^U$ hindurch bis wenigstens zur geometrischen Mitte der unteren Stirnfläche des oberen Stempels hinaufragt;

- der Mittelstift MF von unten nach oben die geometrische Form eines Kreiszylinders Z mit einer kreiszylindrischen Mantelfläche MZ aufweist;

- die Länge der Umrisslinie des Kreiszylinders Z kleiner als die Länge der Umrisslinie des Kreiszylinders I sowie kleiner als die Länge der Umrisslinie des Kreiszylinders II ist;

- die Position des Mittelstiftes MF und die Position der Matrize einschließlich der Matrizenbohrung während des Verfahrens längs der Bohrachse B relativ zueinander fixiert sind;

- in der geometrischen Mitte der unteren Stirnfläche des oberen Stempels eine in den oberen Stempel hineinführende und mit wenigstens einem Auslass aus dem oberen Stempel (gasdurchlässig) in Verbindung stehende Mittelbohrung MB° ausgebildet ist, die den Mittelstift MF bei der Verringerung des Ausgangsabstands A auf den Endabstand E im erforderlichen Umfang aufzunehmen vermag und in die der Mittelstift MF bereits im Ausgangsabstand A hineinragen kann;

- die Symmetrieachsen der Matrizenbohrung, des Kreiszylinders I, des Kreiszylinders II, der Mittelbohrung MB°, des Mittelstiftes MF und der Mittelbohrung $MB^U$ auf einer gemeinsamen, durch die Matrizenbohrung vertikal verlaufenden geraden Linie L liegen;

- die Matrizenbohrung längs ihrer Bohrachse einen Längsabschnitt I aufweist, auf dessen Länge I die geometrische Form der Innenwand der Matrizenbohrung derjenigen der Mantelfläche eines Kreiszylinders KZ entspricht, und an dessen oberem Ende sich unmittelbar ein nach oben gerichteter Längsabschnitt II der Matrizenbohrung anschließt, der die Länge II aufweist;

- die Ausmaße des Längsabschnitts I der Matrizenbohrung und des Kreiszylinders I so beschaffen sind, dass der untere Stempel während des Verfahrens stets jeweils wenigstens auf einer Teillänge des Längsabschnitts I mit seiner Mantelfläche auf der Innenwand der Matrizenbohrung gleitend in die Matrizenbohrung geführt ist; und

- die Ausmaße der Mittelbohrung $MB^U$ und des Kreiszylinders Z so beschaffen sind, dass der untere Stempel während des Verfahrens stets wenigstens im Bereich des Eingangs seiner Mittelbohrung $MB^U$ in seine obere Stirnfläche mit der Innenwand der Mittelbohrung $MB^U$ auf der kreiszylindrischen Mantelfläche MZ des Mittelstiftes MF gleitend in die Matrizenbohrung geführt ist; und

nach beendeter Verdichtung der obere Stempel vom gebildeten ringähnlichen Vorläuferformkörper abgehoben und der ringähnliche Vorläuferformkörper durch Anheben des unteren Stempels aus der Matrizenbohrung entfernt wird.

Der Begriff "untere (obere) Stirnfläche" eines Stempels meint in dieser Schrift die Oberfläche der Stirn des Stempels an seinem unteren (oberen) Ende. Ist der Stempel z. B. ein Kreisringzylinder, ist sowohl seine untere Stirnfläche als auch seine obere Stirnfläche ein Kreisring.

Der Begriff "Kreiszylinder" meint in dieser Schrift stets einen "geraden Kreiszylinder".

Verbindet man die Endpunkte paralleler Radien zweier in parallelen Ebenen liegender gleich großer Kreise miteinander durch Strecken, so entsteht ein Kreiszylinder. Die Verbindungsstrecken heißen Mantellinien des Zylinders. Stehen sie senkrecht auf den parallelen Kreisebenen, so heißt der Zylinder "gerade" oder Rotationszylinder. Die Verbindungsstrecke der Kreismittelpunkte ist die Symmetrieachse des geraden Kreiszylinders (häufig auch einfach "Kreiszylinderachse" oder "Achse des Kreiszylinders" genannt). Die Gesamtheit aller Mantellinien bildet die Mantelfläche des Zylinders.

In analoger Weise meint der Begriff "Kegelstumpf" in dieser Schrift einen anderen speziellen Rotationskörper. Dieser (der Kegelstumpf) entsteht dadurch, dass man von einem geraden Kreiskegel parallel zur Grundfläche einen kleineren geraden Kegel abschneidet. Die größere der beiden durch das Abschneiden erzeugten parallelen Kreisflächen wird in dieser Schrift auch als Grundfläche und die kleinere als Deckfläche bezeichnet. Der Abstand von Grund- und Deckfläche wird als Höhe des Kegelstumpfes bezeichnet. Die Dritte der einen Kegelstumpf begrenzenden Flächen wird als Mantelfläche desselben bezeichnet. Die Verbindungslinie der Mittelpunkte von Grund- und Deckfläche bildet die Symmetrieachse des Kegelstumpfs (häufig auch einfach "Achse des Kegelstumpfs" genannt). Unter einem Kegel versteht man einen Körper, der durch einen Kreis (Grundkreis oder Basiskreis) und einen Punkt außerhalb der Ebene des Kreises (Spitze des Kegels) festgelegt ist und dadurch entsteht, dass man die Punkte auf der Umrisslinie des Kreises mit dem einen Punkt außerhalb der Ebene des Kreises verbindet. Steht die Verbindungslinie der Spitze des Kegels mit dem Mittelpunkt des Basiskreises des Kegels senkrecht zur Basisebene, so liegt ein gerader Kreiskegel oder Drehkegel vor.

Der Begriff "Kreisring" meint in dieser Schrift die Fläche zwischen zwei konzentrischen Kreisen, d.h., zwischen zwei Kreisen mit gemeinsamem Mittelpunkt.

Verbindet man von zwei in parallelen Ebenen liegenden kongruenten (deckungsgleichen) Kreisringen (dem Grundkreisring und dem Deckkreisring) die jeweiligen Endpunkte paralleler Radien auf den beiden äußeren Kreisen sowie die jeweiligen Endpunkte paralleler Radien auf den beiden inneren Kreisen durch Strecken, so entsteht ein Kreisringzylinder. Die Verbindungsstrecken der Endpunkte auf den beiden inneren Kreisen heißen innere Mantellinien des Kreisringzylinders (ihre Gesamtheit bildet den inneren Mantel des Kreisringzylinders) und die Verbindungsstrecken der Endpunkte auf den beiden äußeren Kreisen heißen äußere Mantellinien des Kreisringzylinders (ihre Gesamtheit bildet den äußeren Mantel des Kreisringzylinders). Stehen die Mantellinien senkrecht auf den beiden Kreisringen, so heißt der Kreisringzylinder gerade oder nicht schief. Der Begriff "Kreisringzylinder" soll in dieser Schrift stets für den geraden Kreisringzylinder stehen. Die Verbindungsstrecke der Kreisringmittelpunkte heißt Achse des Kreisringzylinders.

Der Begriff der "Bohrung" soll in dieser Schrift nicht so verstanden werden, dass die entsprechende Öffnung mit Hilfe von Bohrern durch Bohren erzeugt worden sein muss.

Vielmehr kann die Öffnung auch auf andere Weise (z. B. mit Hilfe eines Lasers, einer Fräse oder eines Schneidbrenners) erzeugt worden sein. Die Symmetrie der Öffnung soll jedoch so sein, wie wenn sie durch Bohren mit Hilfe eines Bohrers (oder mehrerer Bohrer) erzeugt worden wäre (selbstverständlich kann sie auch so erzeugt worden sein).

Als Mantelfläche wird die Oberfläche eines geometrischen Formkörpers ohne Boden (Grundfläche) und Deckel (Deckfläche) bezeichnet.

Das Merkmal "dass die Mantelfläche eines Kreiszylinders auf der Innenwand einer Bohrung gleitet" (oder umgekehrt) ist in dieser Schrift so zu verstehen, dass, soweit nichts anderes gesagt wird, die der Mantelfläche entsprechende Außenwand des Kreiszylinders über den Bereich der Gleitstrecke (d. h., über den Gleitbereich) auf der Innenwand der Bohrung gleichmäßig, aber gasdurchlässig sowie axial beweglich aufliegt.

Verfahren zur Herstellung eines ringförmigen (kreisringzylindrischen) oxidischen Formkörpers unter Anwendung von zu der in der Präambel dieser Schrift ausgeführten Verfahrensweise ähnlichen Verfahrensweisen sind bekannt (vgl. z. B. EP-A 184 790, US 2005/0263926 sowie die JP-A 10/29097). Das Dokument US 2005/263926 A1 offenbart ein Verfahren nach dem Oberbegriff des Anspruchs 1.

Sie werden üblicherweise angewendet, um aus pulverförmigen Gemischen (Haufwerken) von Metalloxiden und/oder von solchen Metallverbindungen (z. B. Salzen), die durch Erhitzen (thermisches Behandeln) in Metalloxide überführbar sind (wenigstens durch thermisches Behandeln in Anwesenheit von gasförmigem molekularem Sauerstoff und/oder von gasförmigen Sauerstoff freisetzenden Komponenten) kreisringzylindrische oder kurz "ringförmige" Vorläuferformkörper zu erzeugen, die nach darauffolgend durchgeführter thermischer Behandlung (in der Regel bei Temperaturen ≥ 100 °C) als Katalysatoren (in diesem Fall spricht man von ringförmigen Vollkatalysatoren) oder als Trägerformkörper (kurz auch nur "Trägerkörper") für katalytische Aktivmassen (z. B. für ringförmige Schalenkatalysatoren (enthalten die katalytische Aktivmasse auf die äußere Oberfläche des Trägerformkörpers aufgebracht) oder für ringförmige Tränkkatalysatoren (die katalytisch aktive Masse wird (z. B. durch Tränken) in das Innere des Trägerformkörpers eingebracht)) verwendet werden können. Der Begriff "oxidischer Formkörper" bringt dabei zum Ausdruck, dass der Formkörper wenigstens ein Metalloxid, häufig wenigstens ein Multimetalloxid (es enthält neben Sauerstoff wenigstens zwei voneinander verschiedene Metalle; Halbmetalle wie Phosphor, Antimon, Arsen und Silizium sollen in dieser Schrift auch den Metallen zugerechnet werden) enthält.

Anstelle der Bezeichnung Tränkkatalysatoren wird häufig auch die Bezeichnung Trägerkatalysatoren verwendet. Bei den katalytischen Aktivmassen handelt es sich dabei häufig um Multimetalloxide. Verwendung finden ringförmige Katalysatorformkörper beispielsweise zur (gegebenenfalls mit inerten Formkörpern verdünnten) Beschickung des Innenraumes der Reaktionsrohre eines Rohrbündelreaktors mit einem Katalysatorfestbett. Als inerte Formkörper zum Verdünnen kommen auch ringförmige Trägerkörper in Betracht. Ein solches Katalysatorfestbett eignet sich unter anderem zur Durchführung von heterogen katalysierten Gasphasenreaktionen (z. B. Partialoxidationen organischer Verbindungen).

Das entsprechende Reaktionsgasgemisch strömt durch das Katalysatorfestbett und während der Verweilzeit an der Katalysatoroberfläche erfolgt die gewünschte Reaktion. Die Vorteilhaftigkeit von ringförmigen Katalysatorformkörpern besteht in diesem Zusammenhang unter anderem darin, dass der Druckverlust, den das Reaktionsgasgemisch beim Durchströmen von ringförmigen Katalysatorformkörpern erfährt, besonders gering ist (vgl. z. B. EP-A 184 790).

Nachteilig an durch mechanisches Verdichten eines pulverförmigen Haufwerks erzeugten Formkörpern ist ganz generell, dass der Zusammenhalt des Pulverkorns im resultierenden Formkörper im wesentlichen nicht durch intramolekulare chemische Bindungen, sondern durch bleibende interpartikuläre Bindungen bewerkstelligt wird. Zwar resultiert aus Partikelverformungen und Bruchvorgängen beim Verdichtungsvorgang in der Regel eine Zunahme der interpartikulären Gesamtkontaktfläche, dennoch ist der Betrag der durch das Verdichten erzeugten interpartikulären Bindungskräfte ein vergleichsweise beschränkter.

Gemäß eingehenden Untersuchungen der Anmelderin ist der vorgenannte Sachverhalt insbesondere bei der Herstellung von ringförmigen Vorläuferformkörpern von Relevanz, da die Wände von ringförmigen Vorläuferformkörpern aufgrund des sie durchlaufenden inneren Hohlraums fragiler als diejenigen von entsprechenden vollzylindrischen Vorläuferformkörpern sind. Als Folgewirkung kommt es bei der Ausübung der Verfahren gemäß des in der Präambel dieser Schrift zitierten Standes der Technik in den dabei resultierenden ringförmigen Vorläuferformkörpern teilweise zur Ausbildung von optisch kaum wahrnehmbaren Rissen. Bei einer nachfolgenden thermischen Behandlung solcher ringförmiger Vorläuferformkörper, im Rahmen derer im ringförmigen Vorläuferformkörper zusätzlich Gase freigesetzt werden (normalerweise enthält das komprimierte Material sich bei der thermischen Behandlung unter Ausbildung von gasförmigen Substanzen zersetzende und/oder chemisch umsetzende Bestandteile (z. B. Porenbildner)), nimmt eine bereits vorhandene Rissbildung in der Regel ganz offensichtlich noch zu und entwickelte sich gegebenenfalls bis zum Bruch. Teilweise entwickelt sich aus einer vorhandenen Rissbildung (die, wie bereits gesagt, häufig kaum sichtbar ist) auch erst beim Einfüllen in die z. B. Reaktionsrohre und/oder im Verlauf der Durchführung der katalytischen Gasphasenreaktion der unerwünschte Bruch. In vielen Fällen wird die thermische Behandlung der Vorläuferformkörper auch bereits im Reaktor (z. B. Reaktionsrohr) befindlich vorgenommen (z. B. durch Durchleiten entsprechend erhitzter Gase durch die bereits beschickten Reaktionsrohre). Im Katalysatorbett vorhandene Bruchstücke bedingen jedoch eine Verdichtung desselben und verursachen beim das selbige durchströmenden Reaktionsgasgemisch letztlich eine Erhöhung des beim Durchströmen erlittenen Druckverlustes. D. h., im Katalysatorfestbett befindliche Bruchstücke setzen genau den Vorteil herab, zu deren Erzielung die Verwendung ringförmiger Katalysatorformkörper normalerweise erfolgt.

Eine Gegenmaßnahme, die zur Minderung des vorstehend beschriebenen Erscheinungsbildes ergriffen werden kann, besteht z. B. darin, vorab der Einbringung von oxidischen ringförmigen Formkörpern im Rahmen deren Herstellung entstandenen Bruch abzusieben (vgl. z. B. US-B 7,147,011 und die Deutsche Anmeldung mit dem Aktenzeichen 102007028332.8). Im Rahmen einer solchen Siebung zerbrechen in der Regel auch jene Ringe, die zuvor lediglich ausgeprägte Rissbildung gezeigt haben, so dass die Bruchbildung beim Befüllen der Reaktionsrohre mit dem Siebrückstand in der Regel nur noch minimal ist.

Nachteilig an einer solchen Verfahrensweise ist jedoch, dass die Rohstoffkosten für eine großtechnische Katalysatorherstellung nicht unerheblich sind, weshalb der beim Sieben anfallende Siebdurchgang einen nicht unerheblichen materiellen Verlust bedeutet.

Die Aufgabe der vorliegenden Erfindung bestand daher darin, ein verbessertes Verfahren zur Herstellung eines ringförmigen oxidischen Formkörpers zur Verfügung zu stellen, das die beschriebenen Nachteile allenfalls noch in vermindertem Umfang aufweist.

Eingehende Untersuchungen haben zum Ergebnis geführt, dass die angestrebte Verbesserung dadurch erzielt werden kann, dass man die Geometrie des Vorläuferformkörpers dahingehend abändert, dass die geometrische Form seiner äußeren Mantelfläche nicht mehr derjenigen eines Kreiszylinders, sondern (wenigstens teilweise) derjenigen eines Kegelstumpfes entspricht. Auf diese Weise wird im Endergebnis zwar nur noch ein ringähnlicher oxidischer Formkörper hergestellt, dies beeinträchtigt den erwünschten Druckverlustvorteil jedoch allenfalls unwesentlich. Die mit der erfindungsgemäßen Verfahrensweise erzielte deutliche Bruchminderung wird darauf zurückgeführt, dass aufgrund der veränderten geometrischen Verhältnisse beim Entfernen des gebildeten Vorläuferformkörpers aus der Matrizenbohrung durch Anheben des unteren Stempels die Rollreibung zwischen der Innenwand der Matrizenbohrung und der äußeren Mantelfläche des Vorläuferformkörpers im wesentlichen zum Verschwinden gebracht werden kann.

Demgemäß wird mit der vorliegenden Patentanmeldung ein Verfahren zur Herstellung eines ringähnlichen oxidischen Formkörpers, umfassend das mechanische Verdichten eines in den Füllraum einer Matrize eingebrachten pulverförmigen Haufwerks aus Bestandteilen, die wenigstens eine Metallverbindung, die durch thermische Behandlung bei einer Temperatur $\geq$ 100 °C in ein Metalloxid überführbar ist, oder wenigstens ein Metalloxid, oder wenigstens ein Metalloxid und wenigstens eine solche Metallverbindung umfassen, zu einem ringähnlichen Vorläuferformkörper, bei dem sich der Füllraum in einer mit einer vertikalen Bohrachse B von oben nach unten durch das Matrizenmaterial (durch die Matrize) hindurchgeführten Matrizenbohrung befindet und durch

- die Innenwand der Matrizenbohrung,

- die obere Stirnfläche eines von unten entlang der Bohrachse B in die Matrizenbohrung hub- und senkbeweglich eingeführten unteren Stempels (in dieser Schrift auch "Unterstempel" genannt), auf der das in den Füllraum eingebrachte pulverförmige Haufwerk aufliegt,

- die längs der Bohrachse B in einem axialen Ausgangsabstand A oberhalb der oberen Stirnfläche des unteren Stempels befindliche untere Stirnfläche eines entlang der Bohrachse B hub- und senkbeweglich angebrachten oberen Stempels (in dieser Schrift auch "Oberstempel" genannt), dessen untere Stirnfläche das in den Füllraum eingebrachte pulverförmige Haufwerk von oben berührt, und

- die Mantelfläche eines aus der geometrischen Mitte der oberen Stirnfläche des unteren Stempels heraus entlang der Bohrachse B in der Matrizenbohrung von unten nach oben geführten Mittelstiftes MF, der wenigstens bis zur geometrischen Mitte der unteren Stirnfläche des oberen Stempels hinaufreicht, begrenzt wird,

indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt, wobei

- die geometrische Form der (äußeren) Mantelfläche des unteren Stempels derjenigen der Mantelfläche eines Kreiszylinders I entspricht;

- die geometrische Form der (äußeren) Mantelfläche des oberen Stempels derjenigen der Mantelfläche eines Kreiszylinders II entspricht;

- in der geometrischen Mitte der oberen Stirnfläche des unteren Stempels eine von oben nach unten durch den unteren Stempel hindurchgeführte Mittelbohrung $MB^U$ ausgebildet ist;

- im Ausgangsabstand A der beiden Stirnflächen der Mittelstift MF von unten durch die Mittelbohrung $MB^U$ hindurch bis wenigstens zur geometrischen Mitte der unteren Stirnfläche des oberen Stempels hinaufragt;

- der Mittelstift MF von unten nach oben die geometrische Form eines Kreiszylinders Z mit einer kreiszylindrischen Mantelfläche MZ aufweist;

- die Länge der Umrisslinie des Kreiszylinders Z kleiner als die Länge der Umrisslinie des Kreiszylinders I sowie kleiner als die Länge der Umrisslinie des Kreiszylinders II ist;

- die Position des Mittelstiftes MF und die Position der Matrize einschließlich der Matrizenbohrung längs der Bohrachse B während des Verfahrens relativ zueinander fixiert sind;

- in der geometrischen Mitte der unteren Stirnfläche des oberen Stempels eine in den oberen Stempel hineinführende und mit wenigstens einem Auslass aus dem oberen Stempel (gasdurchlässig) in Verbindung stehende Mittelbohrung $MB°$ ausgebildet ist, die den Mittelstift MF bei der Verringerung des Ausgangsabstands A auf den Endabstand E im erforderlichen Umfang aufzunehmen vermag und in die der Mittelstift MF bereits im Ausgangsabstand A hineinragen kann;

- die Symmetrieachsen der Matrizenbohrung, des Kreiszylinders I, des Kreiszylinders II, der Mittelbohrung $MB°$, des Mittelstiftes MF und der Mittelbohrung $MB^U$ auf einer gemeinsamen, durch die Matrizenbohrung vertikal verlaufenden geraden Linie L liegen;

- die Matrizenbohrung längs ihrer Bohrachse einen Längsabschnitt I aufweist, auf dessen Länge I die geometrische Form der Innenwand der Matrizenbohrung derjenigen der Mantelfläche eines Kreiszylinders KZ entspricht, und an dessen oberem Ende sich unmittelbar ein nach oben gerichteter Längsabschnitt II der Matrizenbohrung anschließt, der die Länge II aufweist;

- die Ausmaße des Längsabschnitts I der Matrizenbohrung und des Kreiszylinders I so beschaffen sind, dass der untere Stempel während des Verfahrens (der Verringerung des Ausgangsabstands A auf den Endabstand E) stets jeweils wenigstens auf einer Teillänge (vorzugsweise auf einer Teillänge von wenigstens 10 %, oder von wenigstens 20 %, oder von wenigstens 30 % (in der Regel jedoch $\leq 90\%$, oder $\leq 80\%$) der Länge I) des Längsabschnitts I mit seiner (äußeren) Mantelfläche auf der Innenwand der Matrizenbohrung gleitend in die Matrizenbohrung geführt ist;

- die Ausmaße der Mittelbohrung $MB^U$ und des Kreiszylinders Z so beschaffen sind, dass der untere Stempel während des Verfahrens (der Verringerung des Ausgangsabstands A auf den Endabstand E) stets wenigstens im Bereich des Eingangs seiner Mittelbohrung $MB^U$ in seine obere Stirnfläche mit der Innenwand der Mittelbohrung $MB^U$ auf der kreiszylindrischen Mantelfläche MZ des Mittelstiftes MF gleitend in die Matrizenbohrung geführt ist; und

nach beendeter Verdichtung der obere Stempel vom gebildeten ringähnlichen Vorläuferformkörper abgehoben und der ringähnliche Vorläuferformkörper durch Anheben des unteren Stempels aus der Matrizenbohrung entfernt wird,

dadurch gekennzeichnet, dass ein sich daran anschließendes Verfahren der thermischen Behandlung des ringähnlichen Vorläuferformkörpers bei einer Temperatur $\geq 100\ °C$, bei dem sich wenigstens eine Teilmenge seiner Bestandteile unter Ausbildung wenigstens einer gasförmigen Verbindung zersetzt und/oder chemisch umsetzt und der ringähnliche oxidische Formkörper sich ausbildet zur Verfügung gestellt, und

dass die geometrische Form der Innenwand der Matrizenbohrung auf der Länge II des Längsabschnitts II von unten nach oben derjenigen der Mantelfläche eines sich von unten nach oben erweiternden Kegelstumpfes KS entspricht, dessen Querschnittsfläche an seinem unteren Ende der Querschnittsfläche des Kreiszylinders KZ an dessen oberem Ende entspricht, mit der Maßgabe, dass beim Erreichen des Endabstands E die untere Stirnfläche des oberen Stempels sich im Längsabschnitt II und die obere Stirnfläche des unteren Stempels sich nicht unterhalb des Längsabschnitts I befindet, so dass sich der durch das mechanische Verdichten des pulverförmigen Haufwerks zwischen den beiden Stirnflächen ausgebildete ringähnliche Vorläuferformkörper beim Erreichen des Endabstands E wenigstens teilweise im Längsabschnitt II befindet. D.h., das erfindungsgemäße Verfahren ist dadurch gekennzeichnet, dass sich beim Erreichen des Endabstands E zwischen der oberen Stirnfläche des unteren Stempels und der unteren Stirnfläche des oberen Stempels wenigstens ein Teil der Abstandsstrecke zwischen den beiden Stirnflächen im Längsabschnitt II befindet.

Erfindungsgemäß vorteilhaft befinden sich beim Erreichen des Endabstands E zwischen der oberen Stirnfläche des unteren Stempels und der unteren Stirnfläche des oberen Stempels wenigstens 20 % oder wenigstens 30 %, vorzugsweise wenigstens 40 % oder wenigstens 50 %, besonders bevorzugt wenigstens 60 % oder wenigstens 70 % und ganz besonders bevorzugt wenigstens 80 % bzw. wenigstens 90 % der Abstandsstrecke (oder 100 % der Abstandsstrecke, d.h., die gesamte Abstandsstrecke zwischen der oberen Stirnfläche des unteren Stempels und der unteren Stirnfläche des oberen Stempels beim Erreichen des Endabstands E) zwischen den beiden Stirnflächen im Längsabschnitt II der Matrizenbohrung. Im vollen Umfang macht man von der Vorteilhaftigkeit des erfindungsgemäßen Verfahrens dann Gebrauch, wenn sich beim Erreichen des Endabstands E sowohl die untere Stirnfläche des oberen Stempels als auch die obere Stirnfläche des unteren Stempels im Längsabschnitt II der Matrizenbohrung befinden, so dass sich der gesamte durch das mechanische Verdichten des pulverförmigen Haufwerks zwischen den beiden Stirnflächen ausgebildete ringähnliche Vorläuferformkörper beim Erreichen des Endabstands E im Längsabschnitt II befindet. Dabei erweist es sich im vorgenannten Fall als günstig, wenn sich bereits im Zustand des Ausgangsabstands A sowohl die untere Stirnfläche des oberen Stempels als auch die obere Stirnfläche des unteren Stempels im Längsabschnitt II befinden.

Die Umrisslinie des Kreiszylinders II ist beim erfindungsgemäßen Verfahren anwendungstechnisch zweckmäßig normalerweise länger oder gleich lang wie die Umrisslinie des Kreiszylinders I. In der Regel sind die beiden vorgenannten Umrisslinien gleich lang.

Darüber hinaus liegen die obere Stirnfläche des unteren Stempels und die untere Stirnfläche des oberen Stempels erfindungsgemäß vorteilhaft in zueinander parallelen Ebenen, auf denen die Bohrachse B senkrecht steht.

Die thermische Behandlung der ringähnlichen Vorläuferformkörper kann beim erfindungsgemäßen Verfahren grundsätzlich sowohl in einer dafür ausgestalteten speziellen Vorrichtung (z. B. in einem Bandcalcinierer) als auch erst in dem Reaktor, in welchem sie zur Anwendung kommen sollen (z. B. in den Reaktionsrohren eines Rohrbündelreaktors) vorgenommen werden. Im letzteren Fall wird man zweckmäßiger Weise heiße Gase durch die Reaktionsrohre leiten.

Das erfindungsgemäße Verdichtungsverfahren ist insbesondere zur Herstellung solcher ringähnlicher Vorläuferformkörper von Interesse, bei denen der Endabstand E (eine eventuelle Krümmung der Stirnflächen soll bei der Bestimmung der Abstände A und E keine Berücksichtigung finden; d.h., gemeint ist immer der Abstand der oberen/unteren Umrisslinien der zylindrischen Mantelfläche der Stempel) 2 bis 10 mm, oder 2 bis 8 mm, oder 3 bis 8 mm, oder 3 bis 7 mm beträgt. Sie alle sollen in dieser Schrift im Speziellen als ringähnliche Vorläuferformkörper F bezeichnet werden.

Häufig beträgt dabei der Quotient Q aus der Länge der Umrisslinie des Kreiszylinders Z (Zähler) und der Länge der Umrisslinie des Kreiszylinders I (Nenner) 0,3 bis 0,7 oder 0,4 bis 0,6. D. h., die Differenz, gebildet durch Subtraktion des Radius der Umrisslinie des Kreiszylinders Z vom Radius der Umrisslinie des Kreiszylinders I, beträgt im Fall von ringähnlichen Formkörpern F vielfach 1 bis 3 mm, oder 1 bis 2 mm, oder 1,5 bis 2 mm, oder 1 bis 1,5 mm. Der Durchmesser der Umrisslinie des Kreiszylinders I beträgt im Fall von ringähnlichen Formkörpern F in vielen Fällen ebenfalls 2 bis 10 mm, oder 2 bis 8 mm, oder 4 bis 8 mm, oder 5 bis 7 mm.

Im Unterschied zum Kreiszylinder ist die Querschnittsfläche eines Kegelstumpfs über die Höhe des Kegelstumpfs nicht konstant, sondern nimmt von der Deckfläche zur Grundfläche hin zu. Dies trifft selbstredend auch auf den Kegelstumpf KS zu, der beim erfindungsgemäßen Verfahren auf der Länge des Längsabschnitts II in die Matrizenbohrung einbeschrieben werden kann und dessen Querschnittsfläche von unten nach oben zunimmt ("auf den Kopf gestellter Kegelstumpf").

Ist H die Höhe des Kegelstumpfes KS, ist es für das erfindungsgemäße Verfahren vorteilhaft, wenn die Aufweitung des Kegelstumpfes KS von unten (von der Deckfläche) nach oben (zur Grundfläche) so beschaffen ist, das zwischen dem Durchmesser DD der Deckfläche, dem Durchmesser DG der Grundfläche und der Höhe H des Kegelstumpfs KS die nachfolgende Beziehung erfüllt ist:

$$0,003 \bullet H \leq DG - DD \leq 0,050 \bullet H \qquad (I).$$

Vorzugsweise gilt beim erfindungsgemäßen Verfahren

$$0,005 \bullet H \leq DG - DD \leq 0,025 \bullet H \qquad (II).$$

Besonders bevorzugt gilt beim erfindungsgemäßen Verfahren

$$0,007 \bullet H \leq DG - DD \leq 0,015 \bullet H \qquad (III).$$

Das Vorgenannte trifft insbesondere im Fall einer erfindungsgemäßen Herstellung von ringähnlichen Vorläuferformkörpern F zu.

Normalerweise weist beim erfindungsgemäßen Verfahren sowohl die (für das pulverförmige Haufwerk zugängliche) obere Stirnfläche des unteren Stempels als auch die (für das pulverförmige Haufwerk zugängliche) untere Stirnfläche des oberen Stempels die geometrische Form der Stirnfläche eines Kreisringzylinders auf. D.h., beide Stirnflächen sind normalerweise Kreisringe, die vorzugsweise kongruent sind. Aus verschiedenen Gründen (vgl. z. B. EP-A 184 790) kann es jedoch zweckmäßig sein, eine oder beide der vorgenannten Stirnflächen (die beiden äußeren und die beiden inneren Kreise bleiben dabei vorzugsweise kongruent) z.B. konkav zu gestalten (d.h., der Kreisring ist ins Stempelinnere einwärts gewölbt). In diesem Fall weist die entsprechende Stirn des zugehörigen Stempels die geometrische Form einer kreisförmigen Rille (36) (= eine kreisförmige Vertiefung; bei einer Herstellung von ringähnlichen Vorläuferformkörpern F liegt die Rillentiefe in der Regel bei ≤ 2 mm) auf. Die daraus jeweils resultierende Stirnfläche des erfindungsgemäß hergestellten ringähnlichen Vorläuferformkörpers ist dann in entsprechender Weise nicht plan, sondern nach außen (konvex) gewölbt. Eine derartige Ausgestaltung erweist sich insbesondere im Fall von erfindungsgemäß hergestellten Trägerformkörpern als vorteilhaft. Durch die gekrümmten Stirnflächen kommt es bei der Herstellung daraus resultierender Träger- oder Schalenkatalysatoren in einem geringeren Umfang zur Ausbildung von unerwünschten Zwillingen oder Drillingen der resultierenden Katalysatorformkörper. Der Radius einer solchen Krümmung beträgt in der Regel das 0,4- bis 5-fache des Außendurchmessers des Kreiszylinders I. Im übrigen gilt das in der EP-A 184 790 bezüglich der Vorteilhaftigkeit gekrümmter Stirnflächen von Hohlzylindern Gesagte in entsprechender Weise.

Grundsätzlich kann das Profil der oberen Stirn des unteren Stempels und/oder das Profil der unteren Stirn des oberen Stempels beim erfindungsgemäßen Verfahren aber auch in jedweder anderen für Tabletten (insbesondere pharmazeutische) bekannten Weise gestaltet sein. Beispielsweise können eine oder beide der oben genannten Stirnflächen auch konvex gestaltet werden. Auch kann eine der beiden Stirnflächen konkav und die andere konvex gestaltet werden. Im Fall einer Herstellung von ringähnlichen Vollkatalysatoren sind beide Stirnflächen jedoch vorzugsweise plan gestaltet.

Der Außendurchmesser des unteren Stempels ist beim erfindungsgemäßen Verfahren üblicherweise marginal kleiner als der Innendurchmesser der Matrizenbohrung im Längsabschnitt I, so dass der untere Stempel mit seiner Außenwand auf der Innenwand des Längsabschnitts I der Matrizenbohrung axial gleitend in die Matrizenbohrung eingeführt werden kann. Da sich beim Erreichen des Endabstandes E darüber hinaus nicht nur die untere Stirnfläche des oberen Stempels sondern vorzugsweise auch die obere Stirnfläche des unteren Stempels im Längsabschnitt II der Matrizenbohrung befindet, ist der Außendurchmesser des unteren Stempels beim erfindungsgemäßen Verfahren somit regelmäßig kleiner als der Innendurchmesser der Matrizenbohrung auf der Höhe der oberen Stirnfläche des unteren Stempels beim Erreichen des Endabstands E. In entsprechender Weise ist beim erfindungsgemäßen Verfahren der Außendurchmesser des oberen Stempels anwendungstechnisch zweckmäßig normalerweise etwas kleiner als der Innendurchmesser der Matrizenbohrung auf der Höhe der unteren Stirnfläche des oberen Stempels beim Erreichen des Endabstands E. Auf vorgenannte Art und Weise wird gewährleistet, dass sich sowohl der untere Stempel als auch der obere Stempel im erfindungsgemäß erforderlichen Rahmen vergleichsweise frei in den relevanten Längsabschnitten der Matrizenbohrung bewegen können. Darüber hinaus bilden die so zwischen der unteren (bzw. oberen) Umrisslinie des oberen Stempels (bzw. unteren Stempels) und der Innenwand der Matrizenbohrung im Zustand des Ausgangsabstands A sowie im Zustand des Endabstands E bestehenden Ringspalte Auslässe für die beim Verdichtungsvorgang (Kompressionsvorgang) durch Verringerung des Matrizenfüllraums komprimierte Gasphase (normalerweise Luft oder Stickstoff). Um einen möglichst gleichmäßigen Ringspalt zu gewährleisten, kann z. B. wie in der DE-A 197 14 430 bezüglich der Herstellung von kreiszylinderförmigen Formkörpern durch Tablettieren von pulverförmigem Haufwerk beschrieben vorgegangen werden. Das Gleiten des unteren Stempels auf der Innenwand der Matrizenbohrung des Längsabschnittes I erweist sich in diesem Zusammenhang als ein wesentlicher Vorteil der erfindungsgemäßen Verfahrensweise.

Die vorstehend beschriebenen Ringspalte sind aber auch ursächlich dafür, dass am erfindungsgemäß hergestellten Pressling sowohl im Bereich der Grundfläche als auch im Bereich der Deckfläche in geringem Umfang ein Grat entstehen kann. Die Verdichtung des pulverförmigen Haufwerks innerhalb eines Grats ist weniger stark ausgeprägt als im Bulk des erfindungsgemäß erzeugten Komprimats. Die Entfernung des Grats vom ringähnlichen Vorläuferformkörper ist daher im weiteren Verlauf der Bearbeitung desselben vergleichsweise leicht möglich. In der Regel brechen die Grate z. B. im Rahmen einer wie in der Deutschen Anmeldung mit dem Aktenzeichen 102007028332.8 beschrieben durchzuführenden Bruchsiebung von alleine ab und werden abgetrennt.

Im Übrigen muss sich die Breite der vorbeschriebenen Ringspalte unter anderem an der Körnung des erfindungsgemäß zu verdichtenden pulverförmigen Haufwerks orientieren. D. h., die Breite der Ringspalte sollte in der Regel so limitiert sein, dass sie nicht größer ist als das Zweifache (besser nicht größer ist das als Einfache) der im zu verdichtenden pulverförmigen Haufwerk am häufigsten vertretenen Längstausdehnung des Pulverkorns (die Längstausdehnung eines Pulverkorn ist die längste direkte geradlinige Verbindung zweier auf der Oberfläche des Pulverkorns befindlicher Punkte; besteht das pulverförmige Haufwerk aus durch Agglomeration von Primärkorn erzeugtem Sekundärkorn, ist es in der Regel zweckmäßig, zur Bemessung der noch tolerierbaren Ringspaltbreite die Längstausdehnung des Primärkorns heranzuziehen).

Im Rahmen der erfindungsgemäßen Herstellung von ringähnlichen Vorläuferformkörpern F betragen die vorgenannten Ringspaltbreiten in der Regel wenige (normalerweise weniger als zehn, meist weniger als fünf) Hundertstel Millimeter, und dies auch dann, wenn sich beim Erreichen des Endabstands E beide Stirnflächen im Längsabschnitt II befinden. Vorzugsweise ist auch in diesem Fall die Umrisslinie des Kreiszylinders II gleich lang wie die Umrisslinie des Kreiszylinders I.

Prinzipiell kann die Matrizenbohrung beim erfindungsgemäßen Verfahren nur aus den Längsabschnitten I (31) und II (32) bestehen (nur die Längsabschnitte I und II aufweisen).

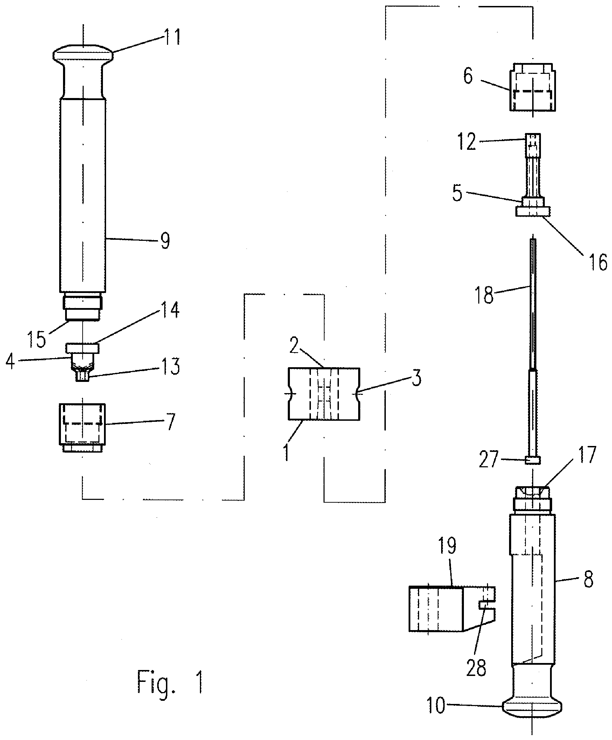

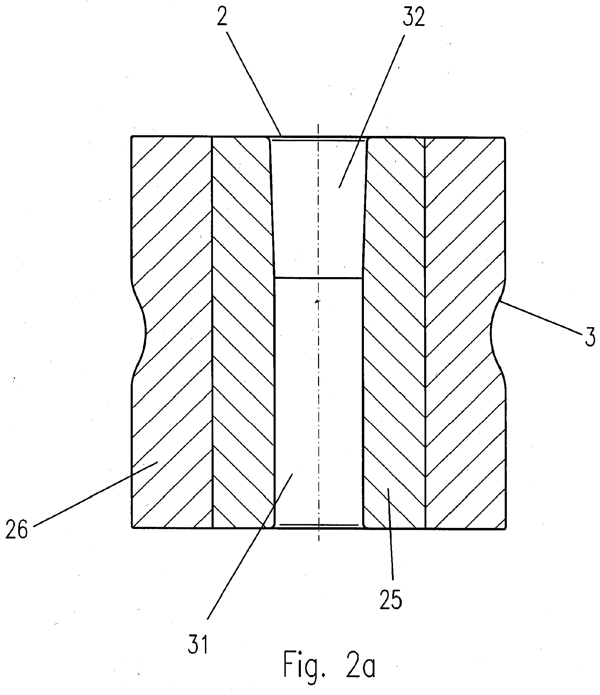

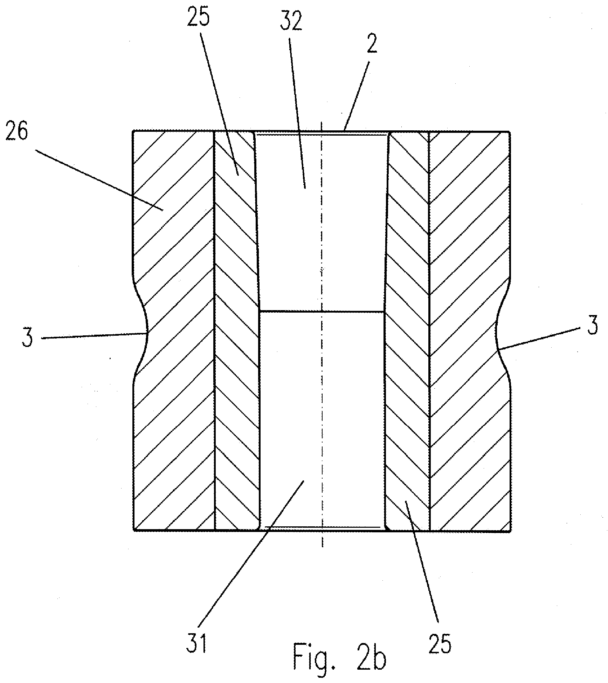

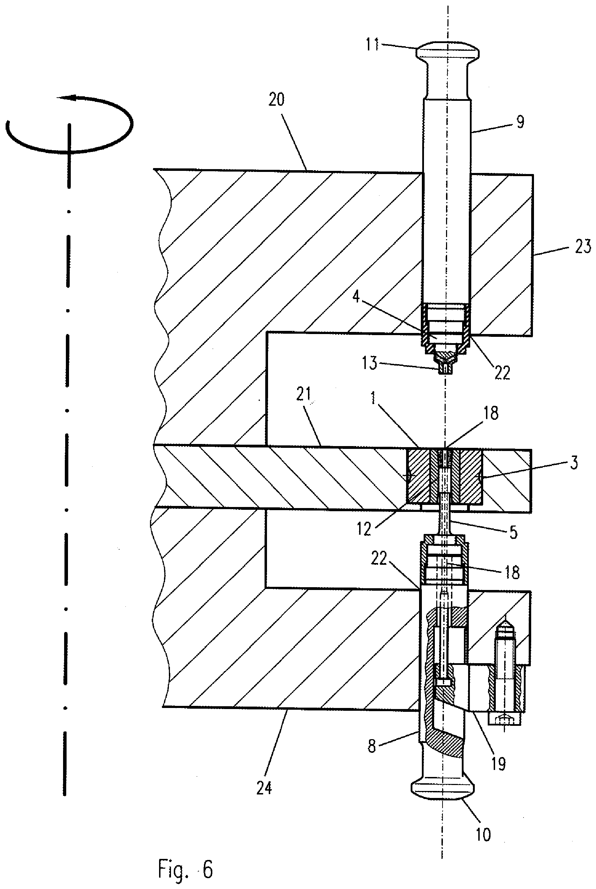

Matrizen mit derartigen Matrizenbohrungen sollen in dieser Schrift als "Matrizen mit einfachem Kegelstumpf" bezeichnet werden. Einen Längsabschnitt durch Matrizen dieser Art zeigen beispielhaft die Figuren 2a und 2b dieser Schrift (an ihrem oberen und an ihrem unteren Ende ist die Matrizenbohrung anwendungstechnisch zweckmäßig leicht abgerundet, um die Verletzungsgefahr durch scharfe Kanten zu minimieren; generell folgen die Figuren 1 bis 8 dieser Schrift in ihrer zeichnerischen Darstellung den Vorgaben im "Tabellenbuch Metall", Verlag Europa Lehrmittel, 41. Auflage, 1999 (D-42781-Haan Gruiten); aus Gründen der Übersichtlichkeit wurden in Figur 6 nicht alle Schnitte vollständig dargestellt; es wird diesbezüglich auf die Einzeldarstellungen verwiesen). Selbstverständlich können sich an die Längsabschnitte I und II der Matrizenbohrung einer Matrize sowohl nach oben als auch nach unten unmittelbar weitere Längsabschnitte anschließen.

Erfindungsgemäß wesentlich ist dabei lediglich, dass der unter Stempel (der obere Stempel) durch sich an den Längsabschnitt I (den Längsabschnitt II) der Matrizenbohrung gegebenenfalls nach unten (oben) anschließende weitere Längsabschnitte in den Längsabschnitt I (in den Längsabschnitt II) eingeführt werden kann.

Insbesondere aus ökonomischen Gründen besonders vorteilhaft ist beim erfindungsgemäßen Verfahren die Verwendung von Matrizen, deren Matrizenbohrung so beschaffen ist, dass sich an ihren Längsabschnitt I nicht nur an dessen oberem Ende unmittelbar ein nach oben gerichteter Längsabschnitt II, sondern auch an dessen unterem Ende ein nach unten gerichteter Längsabschnitt anschließt (in dieser Schrift als Längsabschnitt II* (33) bezeichnet), bei dem die geometrische Form der Innenwand der Matrizenbohrung auf der Länge II* des Längsabschnitts II* ebenfalls der Mantelfläche eines Kegelstumpfes entspricht, dessen Querschnittsfläche an seinem oberen Ende der Querschnittsfläche des Kreiszylinders KZ an dessen unterem Ende entspricht (in dieser Schrift als Kegelstumpf KS* bezeichnet), jedoch mit einem sich nach unten erweiternden Querschnitt (vorzugsweise erfüllen auch der Durchmesser der Deckfläche, der Durchmesser der Grundfläche und die Höhe des Kegelstumpfes KS* wenigstens eine der Beziehungen (I), (II) oder (III)). Matrizen mit Matrizenbohrungen, die nur die Längsabschnitte II*, I und II aufweisen, werden in dieser Schrift als "Matrizen mit doppeltem Kegelstumpf" bezeichnet (selbstredend können sich auch an die Längsabschnitte II und II* grundsätzlich unmittelbar weitere Längsabschnitte anschließen, so lange der jeweilige Stempel in selbige eingeführt werden kann). Erfindungsgemäß vorteilhaft entsprechen dabei die geometrischen Ausmaße des Längsabschnitts II* denen des Längsabschnitts II. Matrizen der vorgenannten Art sind insofern besonders vorteilhaft, als die erfindungsgemäße Verdichtung z. B. zunächst in der oberen Hälfte des Längsabschnitts I und/oder im Längsabschnitt II der Matrizenbohrung durchgeführt werden kann. Ist die Innenwand der Matrizenbohrung im vorgenannten Bereich aufgrund vielfacher Verfahrensdurchführung in selbigem abgenutzt, kann die Matrize in einfacher Weise auf den Kopf gestellt (um eine senkrecht zur Matrizenbohrung liegende Achse um 180° gedreht werden) und die erfindungsgemäße Verdichtung anschließend in der anderen Hälfte des Längsabschnitts I und/oder im Längsabschnitt II* der Matrizenbohrung durchgeführt werden. Für das erfindungsgemäße Verfahren ganz besonders vorteilhaft ist die Durchführung desselben mit Matrizen, deren Matrizenbohrung nur aus einem Längsabschnitt I und einem sich an dessen oberes Ende unmittelbar anschließenden Längsabschnitt II sowie einem sich an dessen unteres Ende unmittelbar anschließenden Längsabschnitt II* besteht und wobei die Geometrie des Längsabschnitts II der Matrizenbohrung zur Geometrie des Längsabschnitts II* der Matrizenbohrung kongruent ist (= "Matrize mit kongruentem doppeltem Kegel stumpf").

Ferner ist es anwendungstechnisch geschickt, wenn die Länge II (Höhe) des Längsabschnitts II (sowie die Länge II* des Längsabschnitts II*) der Matrizenbohrung der zur Durchführung des erfindungsgemäßen Verfahrens verwendeten Matrize (d. h., die Höhe H des Kegelstumpfes KS (sowie des Kegelstumpfes KS*)) bis zum Vierfachen, vorzugsweise bis zum Dreifachen oder bis zum Zwei- oder Eineinhalbfachen des axialen Endabstands E beträgt. D. h., erfindungsgemäß vorteilhaft sind erfindungsgemäße Verfahren für die zutrifft:

$$4 \bullet \text{Endabstand E} \geq H \geq 1 \bullet \text{Endabstand E} \qquad (IV);$$

oder

$$3 \bullet \text{Endabstand E} \geq H \geq 1 \bullet \text{Endabstand E} \qquad (V);$$

oder

$$1{,}5 \bullet \text{Endabstand E} \geq H \geq 1 \bullet \text{Endabstand E} \qquad (VI);$$

oder

$$3 \bullet \text{Endabstand } E \geq H \geq 1{,}5 \bullet \text{Endabstand } E \quad \text{(VII)};$$

oder

$$2 \bullet \text{Endabstand } E \geq H \geq 1{,}5 \bullet \text{Endabstand } E \quad \text{(VIII)}.$$

In der Regel wird beim erfindungsgemäßen Verfahren die Länge I des Längsabschnitts I größer sein als die Länge II des Längsabschnitts II (sowie größer als die Länge II* des Längsabschnitts II*). Die Länge I des Längsabschnitts I kann aber auch kleiner sein als die Länge II des Längsabschnitts II (sowie kleiner als die Länge II* des Längsabschnitts II*).

Üblicherweise wird die Länge I jedoch nicht mehr als das Dreifache der Länge II (als das Dreifache der Länge II*) betragen.

Häufig beträgt die Länge I nicht mehr als das Zweifache (oder Einfache) der Länge II (als das Zweifache (oder Einfache) der Länge II*).

Normalerweise beträgt die Länge I nicht weniger als das 0,1-fache (bzw. nicht weniger als das 0,2-fache) der Länge II (als das 0,1-fache bzw. 0,2-fache der Länge II*).

Vielfach beträgt die Länge I das 0,1- bis 1-fache oder das 0,5 bis 1-fach der Länge II (bzw. der Länge II*).

Alles Vorgenannte gilt, wie sonst auch in dieser Schrift, insbesondere für den Fall einer erfindungsgemäßen Herstellung von ringähnlichen Vorläuferformkörpern F. Im Besonderen gilt alles in dieser Schrift Gesagte für eine erfindungsgemäße Herstellung von ringähnlichen Vorläuferformkörpern F, bei deren Herstellung beim Erreichen des Endabstands E sowohl die obere Stirnfläche des unteren Stempels als auch die untere Stirnfläche des oberen Stempels sich im Längsabschnitt II (oder im Längsabschnitt II*) der Matrizenbohrung befinden. Eine derartige Herstellung von ringähnlichen Vorläuferformkörpern F wird in dieser Schrift im engeren Sinn als eine Herstellung von ringähnlichen Vorläuferformkörpern $F^{LII}$ bezeichnet (unabhängig von den quantitativen Ausmaßen der erfindungsgemäß hergestellten ringähnlichen Vorläuferformkörper, sollen diejenigen, bei deren Herstellung beim Erreichen des Endabstands E sowohl die obere Stirnfläche des untern Stempels als auch die unter Stirnfläche des oberen Stempels sich im Längsabschnitt II (oder im Längsabschnitt II*) der Matrizenbohrung befindet, in dieser Schrift als "ringähnliche Vorläuferformkörper LII" bezeichnet werden.

Die Vorteilhaftigkeit von erfindungsgemäßen Verfahren, auf die wenigstens eine der Beziehungen (IV) bis (VIII) zutrifft, liegt unter anderem darin begründet, dass insbesondere im Rahmen der Herstellung einer größeren Charge von ringähnlichen Vorläuferformkörpern LII mit der erfindungsgemäßen Verdichtung zunächst im oberen Teil des Längsabschnitts II begonnen werden kann (d. h., im Zustand des Ausgangsabstands A befinden sich sowohl die obere Stirnfläche des unteren Stempels als auch die untere Stirnfläche des oberen Stempels im oberen Teil des Längsabschnitts II; erfindungsgemäß vorteilhaft wird man am Beginn des Verfahrens im Zustand des Ausgangsabstands A die unter Fläche des oberen Stempels so positionieren, dass sie mit dem oberen Ende des Längsabschnitts II bündig abschließt). Mit zunehmender Abnutzung der Innenwand des oberen Teils des Längsabschnitts II der Matrizenbohrung wird man dann im Zustand des Ausgangsabstands A sowohl die untere Stirnfläche des oberen Stempels als auch die obere Stirnfläche des unteren Stempels innerhalb der Matrizenbohrung nach unten verschieben. Die im Rahmen einer solchen Vorgehensweise resultierenden ringähnlichen Vorläuferformkörper (z. B. ringähnlichen Vorläuferformkörper LII oder ringähnlichen Vorläuferformkörper $F^{LII}$ ) sind einander geometrisch so ähnlich, dass sie äquivalent zu geometrisch einheitlichen Vorläuferformkörpern (z. B. als Katalysatoren oder Katalysatorträger) verwendet werden können. In bestimmten Fällen (vgl. z. B. die Deutsche Anmeldung mit dem Aktenzeichen 102007017080.9) kann eine definierte Varianz der Formkörpergeometrie über eine Produktionscharge sogar vorteilhaft sein. Dabei ist zu beachten, dass beim Übergang vom ringähnlichen Vorläuferformkörper zum oxidischen Formkörper durch thermische Behandlung des ersteren in der Regel eine Veränderung der Formkörpergeometrie einhergeht.

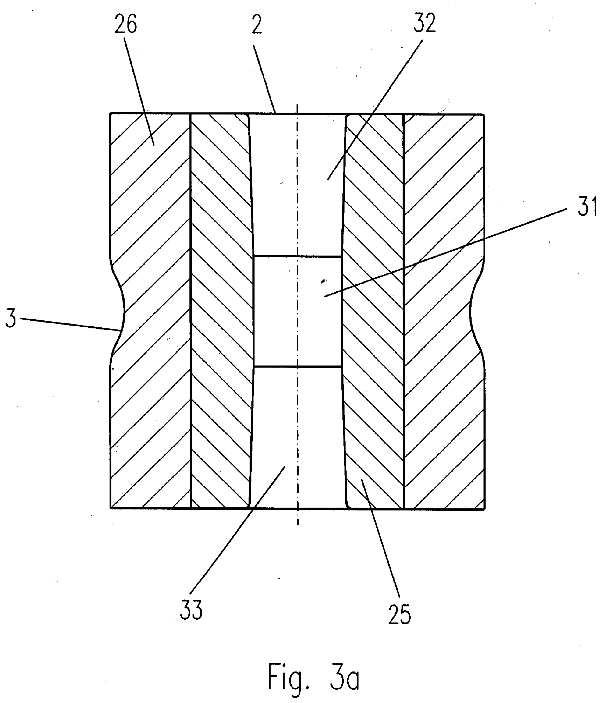

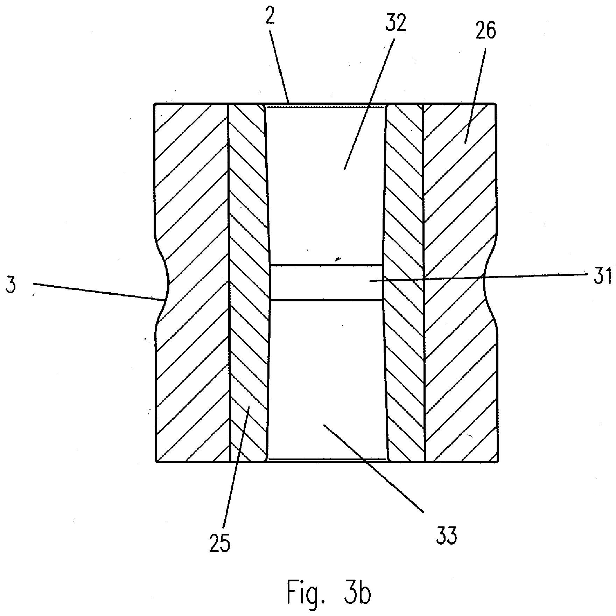

Einen Längsschnitt durch erfindungsgemäß geeignete Matrizen mit kongruentem doppeltem Kegelstumpf zeigen beispielhaft die Figuren 3a und 3b.

Wesentlich für das erfindungsgemäße Verfahren ist, dass in der geometrischen Mitte der unteren Stirnfläche des oberen Stempels eine in den oberen Stempel hineinführende und mit wenigstens einem Auslass aus dem oberen Stempel in Verbindung stehende Mittelbohrung $MB^O$ ausgebildet ist, die den Mittelstift MF bei der Verringerung des Ausgangsabstands A auf den Endabstand E im erforderlichen Umfang aufzunehmen vermag und in die der Mittelstift MF bereits im Ausgangsabstand A hineinragen kann. Der Mittelstift MF wird beim erfindungsgemäßen Verfahren insbesondere dann im Zustand des Ausgangsabstands A bereits in die Mittelbohrung MB° hineinragen, wenn, wie vorstehend beschrieben, im oberen Teil des Längsabschnitts II die Innenwand der Matrizenbohrung bereits abgenutzt ist, und die erfindungsgemäße Verdichtung aus diesem Grund in den weiter unten gelegenen Teil des Längsabschnitts II verlagert wird.

Wird beim erfindungsgemäßen Verfahren im Rahmen des Verdichtungsvorgangs der obere Stempel abgesenkt, muss die Mittelbohrung MB° (35) den Mittelstift MF jedoch in jedem Fall in dem Umfang aufnehmen, indem die Absenkung des oberen Stempels erfolgt.

Da die Mittelbohrung MB° (im Unterschied zur Mittelbohrung $MB^U$ (37), die durch den unteren Stempel hindurchgeführt ist) normalerweise nicht durch den oberen Stempel hindurchgeführt ist, bedarf es anwendungstechnisch zweckmäßig wenigstens eines Auslasses (34), mit dem die Mittelbohrung MB° in Verbindung steht und über den diejenige Gasphase entweichen kann (ausgelassen wird), die der Mittelstift MF bei seiner Aufnahme in die Mittelbohrung MB° im Rahmen eines Absenkens des oberen Stempels verdrängt. In der Regel ist der wenigstens eine Auslass ebenfalls als Bohrung ausgestaltet, die schräg auf die Mittelbohrung MB° zuläuft.

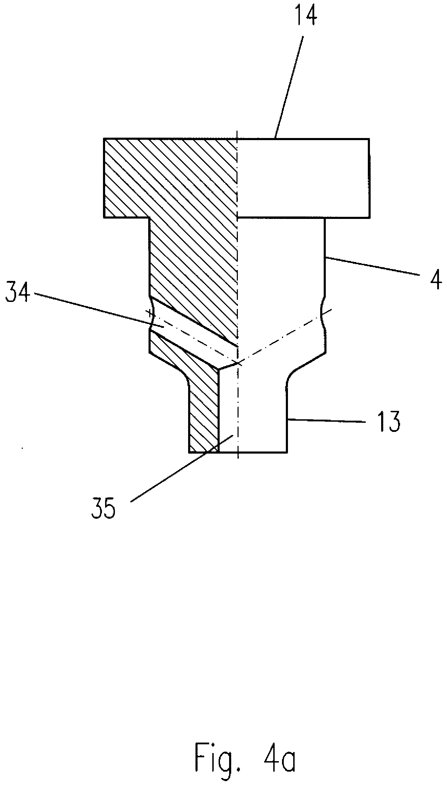

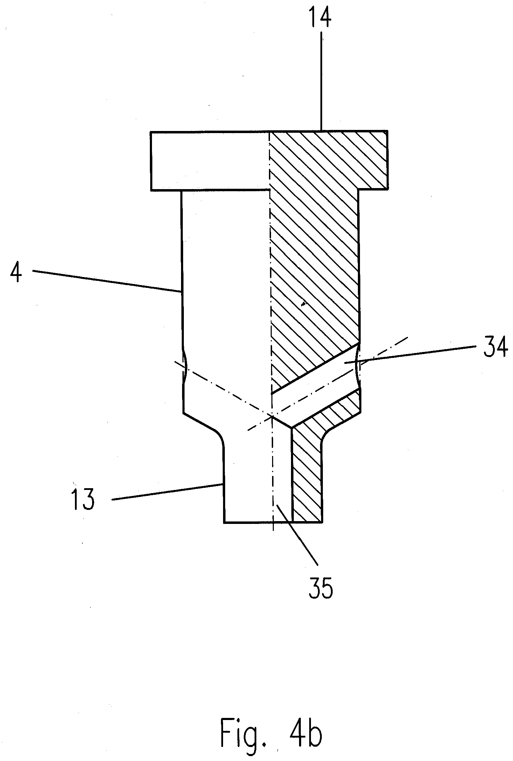

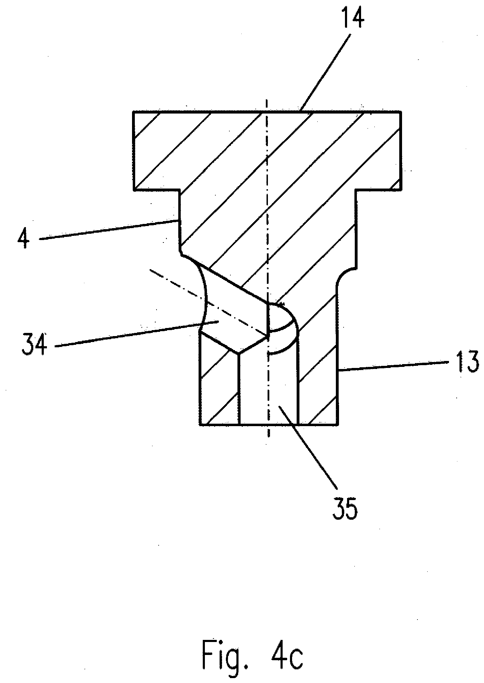

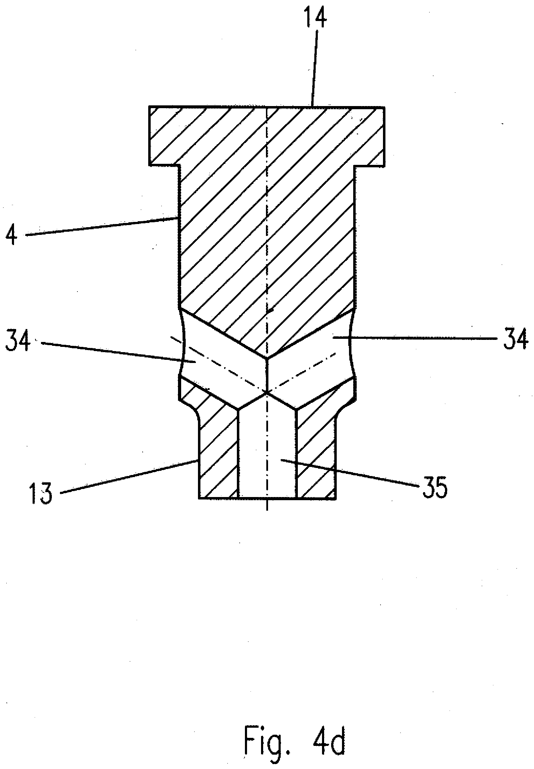

Die Figuren 4a, 4b, 4c und 4d zeigen Längsschnitte durch entsprechende mit wenigstens einem Auslass versehene Oberstempel, wobei der eigentliche obere Stempel im Sinne dieser Erfindung lediglich den nach unten kreiszylindrisch (bzw. ringförmig) auslaufenden Hals in diesen Figuren meint. Die Gesamtfigur zeigt jeweils die Ausgestaltung des erfindungsgemäßen oberen Stempels als sogenannter oberer "Einlegestempel", auf den im weiteren Verlauf dieser Schrift noch Bezug genommen werden wird.

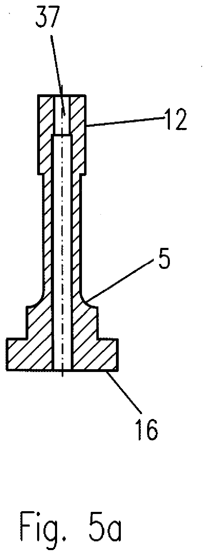

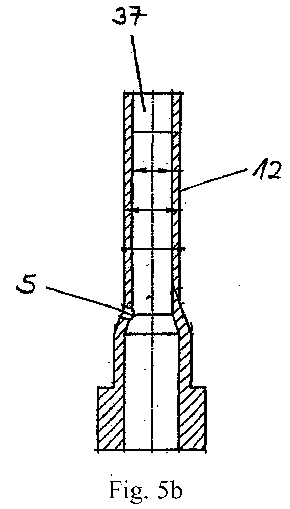

Die Verbindung der Mittelbohrung MB° zu wenigstens einem Auslass ist insbesondere dann von besonderer Bedeutung, wenn wenigstens der Eingang in die Mittelbohrung MB° vorzugsweise kreiszylindrisch so gestaltet ist, dass die Mantelfläche des Kreiszylinders Z (des kreiszylindrischen Mittelstiftes MF) bei seiner Aufnahme in die Mittelbohrung MB° wenigstens im Eingangsbereich derselben auf ihrer Innenwand gleitet. Erfindungsgemäß bevorzugt ist die Mittelbohrung MB° so gestaltet, dass die Geometrie ihrer Innenwand entlang ihrer gesamten Längsachse der Mantelfläche eines Kreiszylinders entspricht. In diesem Fall sind die Ausmaße des kreiszylindrischen Mittelstiftes MF und der Mittelbohrung MB° vorzugsweise so gestaltet, dass die Mantelfläche des kreiszylindrischen Mittelstiftes MF (des Kreiszylinders Z) im gesamten Umfang seiner Aufnahme in die Mittelbohrung MB° auf der Innenwand der Mittelbohrung MB° gleitet. Im Unterschied dazu ist die Mittelbohrung $MB^U$ unterhalb des Bereichs ihres Eingangs in die obere Stirnfläche des unteren Stempels häufig geringfügig erweitert gestaltet, wie es z. B. im Längsschnitt durch einen unteren Stempel in der Figur 5a gezeigt wird. Im Unterschied dazu zeigt die Figur 5b einen Längsschnitt durch einen unteren Stempel, bei dem die Mittelbohrung $MB^U$ über die gesamte Länge des unteren Stempels mit konstantem zylindrischem Querschnitt gestaltet ist. Auch in den Figuren 5a und 5b ist mit dem unteren Stempel im Sinne dieser Erfindung lediglich der nach oben kreiszylindrisch (bzw. ringförmig) auslaufende Hals in diesen Figuren gemeint. Die Gesamtfigur zeigt jeweils die Ausgestaltung des erfindungsgemäßen unteren Stempels als sogenannter "unterer Einlegestempel", auf den im weiteren Verlauf dieser Schrift noch Bezug genommen werden wird. Die Öffnung der Mittelbohrung $MB^U$ in der oberen Stirnfläche des unteren Stempels und die Öffnung der Mittelbohrung MB° in der unteren Stirnfläche des oberen Stempels sind normalerweise kongruent gestaltet.

Ist die Matrize eine solche mit kongruentem doppeltem Kegelstumpf, ist die Länge einer Mantellinie des Kreiszylinders I in der Regel nicht größer als die Summe aus der Länge II und dem 0,7-fachen der Länge I (und dem 0,5-fachen der Länge I). Ist der untere Stempel dabei als ein unterer Einlegestempel ausgestaltet, ist es erfindungsgemäß vorteilhaft, wenn der Querschnitt des Einlegestempels dort, wo er das untere Ende des unteren Stempels hält, im Vergleich zum Querschnitt des Kreiszylinders I, einen kleineren Querschnitt aufweist. Eine derartige Ausgestaltung ermöglicht den Austrag von feinteiligem Korn, das sich zwischen der Wand der Matrizenbohrung und der Mantelfläche des Kreiszylinders I verfangen hat, im Zuge einer Absenkung des unteren Stempels in den Längsabschnitt II* hinein.

Erfindungsgemäß weist der Mittelstift MF von unten nach oben die geometrische Form eines Kreiszylinders Z mit einer kreiszylindrischen Mantelfläche MZ auf. Des weiteren ist es erfindungsgemäß wesentlich, dass die Position des Mittelstiftes MF und die Position der Matrize einschließlich der Matrizenbohrung während des Verfahrens längs der Bohrachse B relativ zueinander fixiert sind. Die Fixierung der Matrize wird in der Praxis in der Regel so vorgenommen, dass die Matrize passgenau in eine entsprechende Aufnahmeöffnung innerhalb einer Matrizenscheibe eingelegt wird.

Zusätzlich wird sie normalerweise mittels einer Befestigungsschraube, die z. B. vom Matrizenscheibenrand horizontal zur Aufnahmeöffnung für die Matrize führen kann, fixiert. Weist die Matrizenscheibe mehrere, z.B. auf einem Kreisumfang äquidistant platzierte Aufnahmeöffnungen auf, kann die zusätzliche Fixierung von in selbige eingelegten Matrizen auch so erfolgen, dass die Befestigungsschraube auf einem Teilkreis zwischen zwei Aufnahmeöffnungen platziert ist, die die zwei in diese eingelegten Matrizen gegeneinander fixiert.

Zur Fixierung des Mittelstiftes MF werden in der Regel Mittelstifthalter eingesetzt. Um die Fixierung zu erleichtern, ist der Mittelstift MF an seinem unteren Ende normalerweise mit einem Kopf (27) ausgestattet, der von einem passgenau gefertigten Zwischenraum (28) (Schlitz) des Mittelstifthalters aufgenommen wird. Zum vorgenannten Kopf hin kann sich an den eigentlichen Mittelstift ein verbreiterter Querschnitt anschließen, der die Fixierung des Mittelstiftes erleichtert (vgl. z. B. in Figur 6 und in Figur 1). Der Mittelstifthalter selbst wird anwendungstechnisch zweckmäßig in der Regel ebenfalls an der Matrizenscheibe festgeschraubt.

Die vorliegende Erfindung soll jedoch auch diejenige Ausführungsform umfassen, bei der der Mittelstift MF von unten nach oben zunächst die geometrische Form eines Kreiszylinders Z mit einer kreiszylindrischen Mantelfläche MZ aufweist, sich daran anschließend jedoch nach oben hin konisch verjüngt. Dies gilt insbesondere dann, wenn der von unten nach oben zunächst kreiszylindrische Mittelstift MF sich innerhalb des Längsabschnitts II der Matrizenbohrung nach oben hin konisch verjüngt (und sich bis zu seinem oberen Ende nicht mehr erweitert). In diesem Fall kann der Mittelstift MF von unten nach oben die Geometrie eines Kreiszylinders Z aufweisen, dem dann innerhalb des Längsabschnitts II ein sich nach oben verjüngender Kegelstumpf KM (30) aufgesetzt ist (der Querschnitt des Kreiszylinders Z entspricht dabei dem Querschnitt der Grundfläche des Kegelstumpfs KM). Dabei kann die Höhe des Kegelstumpfs KM der Länge des Längsabschnitts II entsprechen (was erfindungsgemäß bevorzugt ist), aber auch kürzer sein (im letzteren Fall erstreckt sich der eine kreiszylindrische Geometrie aufweisende Anteil des Mittelstiftes von unten nach oben bis in den Längsabschnitt II hinein. Die Vorteilhaftigkeit eines solchen Auslaufens des Mittelstiftes MF nach oben als Kegelstumpf KM liegt, in ähnlicher Weise wie die Vorteilhaftigkeit der Geometrie des Längsabschnitts II der Matrizenbohrung selbst, darin begründet, dass aufgrund der konischen Verjüngung des Mittelstiftes MF nach oben, beim Entfernen des gebildeten ringähnlichen Formkörpers aus der Matrizenbohrung durch Anheben des unteren Stempels die Rollreibung zwischen der Außenwand (der Mantelfläche) des Mittelstiftes MF und der Mantelfläche des Hohlraums des gebildeten ringähnlichen Formkörpers (z. B. über die Länge des Längsabschnitts II) im wesentlichen zum Verschwinden gebracht werden kann (z. B. im Fall der Herstellung von ringähnlichen Vorläuferformkörpern LII bzw. $F^{LII}$). Allerdings ist der aus diesem Sachverhalt resultierende Vorteilsgewinn vergleichsweise beschränkt, da die Mantelfläche des Kegelstumpfs KM im Vergleich zur Mantelfläche des Kegelstumpfs KS bei gleicher Höhe im Normalfall wesentlich kleiner ist.

Ist H* die Höhe des Kegelstumpfs KM, ist es vorteilhaft, wenn die Verjüngung des Kegelstumpfs KM von unten (von der Grundfläche) nach oben (zur Deckfläche) so beschaffen ist, dass zwischen dem Durchmesser DG* der Grundfläche und der Höhe H* sowie dem Durchmesser DD* der Deckfläche des Kegelstumpfes KM die nachfolgende Beziehung erfüllt ist:

$$0{,}005 \bullet H^* \leq DG^* - DD^* \leq 0{,}015 \bullet H^* \qquad (IX).$$

Vorzugsweise gilt:

$$0{,}007 \bullet H^* \leq DG^* - DD^* \leq 0{,}013 \bullet H^* \qquad (X).$$

Besonders bevorzugt gilt:

$$0{,}009 \bullet H^* \leq DG^* - DD^* \leq 0{,}011 \bullet H^* \qquad (XI).$$

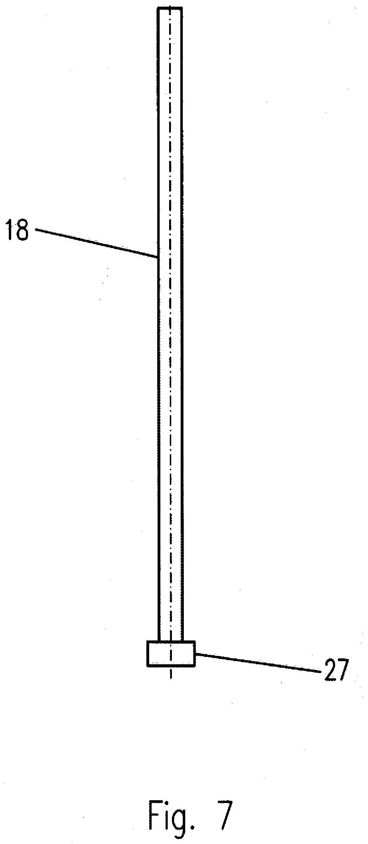

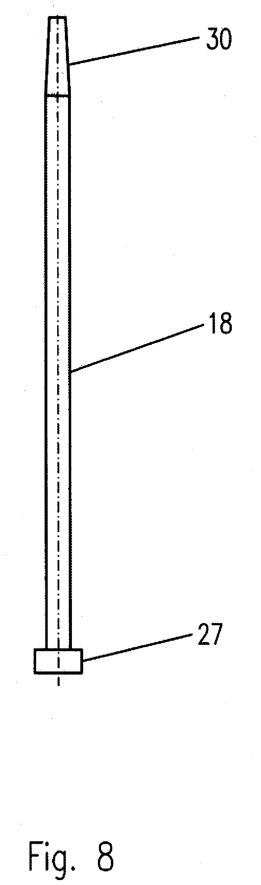

Zu beachten ist bei der Anwendung eines sich z. B. zu seinem oberen Ende hin konisch verjüngenden Mittelstiftes MF, dass aufgrund des über die Höhe H* des Kegelstumpfes KM nicht konstanten Querschnitts bei der Aufnahme des Kegelstumpfs KM in die Mittelbohrung MB° in notwendiger Weise ein Ringspalt verbleibt (die Mantelfläche des Kegelstumpfs KM nicht auf der Innenwand der Mittelbohrung MB° gleitet). Die noch tolerable Breite desselben muss sich wieder an der Korngröße des zu verdichtenden pulverförmigen Haufwerks orientieren. Im Normalfall wird man den Querschnitt der Mittelbohrung MB° im Fall eines sich nach oben konisch verjüngenden Mittelstiftes MF so bemessen, dass er bei einer Aufnahme seines kreiszylindrischen Abschnitts in die Mittelbohrung MB° mit seiner kreiszylindrischen Mantelfläche wenigstens im Bereich des Eingangs in die Mittelbohrung MB° auf deren Innenwand gleiten würde. Figur 7 zeigt beispielhaft einen Längsschnitt durch einen von unten nach oben ausschließlich die geometrische Form eines Kreiszylinders Z aufweisenden Mittelstiftes MF, während Figur 8 beispielhaft einen Längsschnitt durch einen solchen Mittelstift MF zeigt, der von unten nach oben zunächst die Geometrie eines Kreiszylinders Z aufweist, und sich anschließend bis zu seinem oberen Ende konisch verjüngt.

Ganz generell ist es beim erfindungsgemäßen Verfahren bevorzugt, wenn das obere Ende des Längsabschnitts II der Matrizenbohrung, die obere (plane) Stirnfläche des Mittelstiftes MF und die obere (plane) Stirnfläche der Matrize miteinander bündig (d. h., nicht überstehend) abschließen.

Dies gilt insbesondere für den Fall einer maschinellen Durchführung des erfindungsgemäßen Verfahrens, da vorgenannte Konstellation das maschinelle Einbringen des pulverförmigen Haufwerks in den Füllraum erleichtert (begünstigt).

In der Regel weist die Matrize eine plane obere Stirnfläche auf. Anwendungstechnisch zweckmäßig ist auch die untere Stirnfläche der Matrize plan gestaltet. Vorzugsweise weist die Matrize (sieht man von der Matrizenbohrung ab) die Form eines Kreiszylinders mit einer planen oberen und einer planen unteren Stirnfläche auf. In der äußeren Wand des Kreiszylinders verläuft auf halber Höhe vorteilhaft ein horizontal verlaufender Ring bzw. eine runde Vertiefung. Sie dient der Fixierung der Matrize in der Matrizenscheibe mittels einer oder mehreren Befestigungsschrauben.

Grundsätzlich kann beim erfindungsgemäßen Verfahren die Verringerung des Ausgangsabstands A auf den Endabstand E so erfolgen, dass beide (der untere und der obere Stempel) Stempel aktiv aufeinander zu bewegt werden. Selbstverständlich kann aber auch so vorgegangen werden, dass der untere Stempel seine Position beibehält und nur der obere Stempel bewegt (nach unten abgesenkt) wird.

In der Regel ist es für eine möglichst gleichmäßige Oberflächenhärte des beim erfindungsgemäßen Verfahren resultierenden Komprimats (ringähnlichen Vorläuferformkörpers) vorteilhaft, wenn bei der Verringerung des Ausgangsabstands A auf den Endabstand E Ober- und Unterstempel gemeinsam aktiv aufeinander zu bewegt werden (der obere Stempel wird abgesenkt, der untere Stempel wird angehoben). In diesem Fall wird der erforderliche Kompressionsdruck auf das pulverförmige Haufwerk vom oberen und vom unteren Stempel gleichmäßig ausgeübt, was eine einheitlichere Seitendruckfestigkeit des resultierenden Komprimats über dessen Höhe bedingt.

Auch resultiert dabei ein Vorläuferformkörper mit einer homogeneren Massendichte über seine gesamte Dimension. Letzteres bedingt nach der thermischen Behandlung ein homogeneres Porengefüge und auf letzterem basierend eine verbesserte Katalysatorperformance.

Grundsätzlich kann das erfindungsgemäße Verfahren sowohl manuell als auch maschinell durchgeführt werden. Aus Gründen der Wirtschaftlichkeit ist eine maschinelle Ausübung des erfindungsgemäßen Verfahrens bevorzugt. Im wesentlichen können dazu zwei Maschinentypen verwendet werden, die in der Fachliteratur als "Exzenterpresse" und als "Rundläufer" voneinander unterschieden werden. Bei der Exzenterpresse übt nur der Oberstempel den eigentlichen Kompressionsdruck durch seine Abwärtsbewegung mit Hilfe der Exzenterscheibe aus, wobei der Unterstempel während der Kompression stillsteht und sich lediglich zur abschließenden Ausstoßung des Komprimats (des ringähnlichen Vorläuferformkörpers) aufwärts bewegt (angehoben wird). Bei der Exzenterpresse steht die Matrize still. Sie ruht in der Matrizenplatte auf dem feststehenden Matrizentisch. Die Matrize kann eine oder (nebeneinander) mehrere Matrizenbohrungen (und damit Matrizenfüllräume) aufweisen. In jeder Matrizenbohrung bewegt sich im Rhythmus der Exzenterscheibe ein aus Oberstempel und Unterstempel bestehendes Stempelpaar. Der Mittelstift MF ist ebenfalls stillstehend durch die Matrizenbohrung und den unteren Stempel geführt und mit einem Mittelstifthalter an der Matrizenplatte befestigt. Je nachdem ob die Matrize eine oder mehrere Matrizenbohrungen (Matrizenfüllräume) aufweist, spricht man von einer einstempligen oder mehrstempligen Matrize. In entsprechender Weise wird zwischen Einfach- und Mehrfachwerkzeugen unterschieden. Das Einfachwerkzeug besteht aus einer Matrize mit einer Matrizenbohrung und einem Mittelstift MF sowie einem Ober- und einem Unterstempel. Ein Mehrfachwerkzeug besteht in entsprechender Weise aus einer Matrize mit zwei oder mehreren Matrizenbohrungen und mit entsprechend vielen Mittelstiften MF sowie Ober- und Unterstempeln. Ob Einfach- oder Mehrfachwerkzeuge verwendet werden, wird im wesentlichen anhand der Größe des ringähnlichen Vorläuferformkörpers sowie des Pressdrucks, den die Maschine leisten kann, entschieden. Die beim erfindungsgemäßen Verfahren anwendbare Obergrenze liegt bei einem ca. fünfzigfachen Werkzeug. Da bei der Exzenterpresse die Matrize stillsteht, gleiten normalerweise ein Fülltrichter samt Füllschuh, die das erfindungsgemäß zu verdichtende pulverförmige Haufwerk enthalten, auf dem Matrizentisch vorwärts und rückwärts, um eine gleichmäßige Füllung des Füllraums bzw. der Füllräume der Matrize zu erreichen. Füllung des Füllraums, Verdichtung (Kompression) und Auslass des ringähnlichen Formkörpers erfolgen bei der Exzenterpresse auf diese Weise zeitlich periodisch wiederkehrend nacheinander und entsprechen zusammen jeweils einer vollen Exzenterumdrehung.

Im einfachsten Fall verläuft der Arbeitsgang bei der Exzentermaschine daher wie folgt.

Der Unterstempel befindet sich innerhalb der Matrizenbohrung zunächst in seiner Füllstellung. Der Füllschuh gleitet über die Matrize, deren obere plane Stirnfläche mit der oberen planen Stirnfläche des Mittelstiftes MF bündig abschließt, wobei das Füllgut (das pulverförmige Haufwerk) in die Matrizenbohrung und auf die obere Stirnfläche des unteren Stempels gelangt. Beim Zurückgleiten des Füllschuhes bewegt sich der Oberstempel abwärts, bis er mit seiner unteren Stirnfläche das Füllgut berührt. Damit ist das pulverförmige Haufwerk in den Füllraum eingebracht und der Zustand des Ausgangsabstands A erreicht. Durch weitergehende Abwärtsbewegung des Oberstempels (bei feststehendem Unterstempel) wird das Füllgut zum ringähnlichen Vorläuferformkörper verdichtet, bis der Endabstand E samt zugehörigem Pressdruck erreicht ist. Anschließend wird der Oberstempel vom gebildeten ringähnlichen Vorläuferformkörper abgehoben und durch (in der Regel etwas verzögertes) Anheben des unteren Stempels der ringähnliche Vorläuferformkörper aus der Matrizenbohrung entfernt. In der Regel erfolgt das Anheben des unteren Stempels so weit, dass die Unterseite des gebildeten Vorläuferformkörpers das Niveau der Matrizenoberseite knapp erreicht. Während nun die Vorderkante des sich vorwärtsschiebenden Füllschuhes den Vorläuferformkörper von der Matrize schiebt, senkt sich der Unterstempel bereits wieder bis zu seiner Füllstellung, und die Matrizenbohrung wird neu gefüllt.

Beim Rundläufer steht dagegen der Fülltrichter samt Füllschuh fest, und eine Matrizenscheibe, in der die Matrizen ruhen, rotiert, wobei die Matrizenbohrungen unter dem Füllschuh vorbeigeführt werden. Bei einer Umdrehung der Matrizenscheiben werden die einzelnen Matrizen (bzw. deren Matrizenbohrungen) nacheinander gefüllt.

Dann werden die Füllungen komprimiert und nachfolgend die resultierenden Komprimate ausgestoßen.

Es wird also eine Matrizenbohrung gefüllt, während eine andere Matrizenfüllung komprimiert und gleichzeitig bei wiederum einer anderen Matrize ein ringähnlicher Vorläuferformkörper (das Komprimat) ausgestoßen wird. Bei einer Umdrehung der Matrizenscheibe werden so viele ringähnliche Vorläuferformkörper hergestellt, wie - bei Verwendung von einstempligen Werkzeugen - Werkzeugsätze vorhanden sind. Bei mehrstempligen Werkzeugen ist mit der Anzahl der Bohrungen pro Matrize zu multiplizieren. Während die Exzenterpresse diskontinuierlich arbeitet, arbeiten Rundlaufpressen kontinuierlich. Ferner wird beim Rundläufer der Kompressionsdruck mit Hilfe von Druckrollen vom Ober- und Unterstempel gleichmäßig ausgeübt.

An Rundläufern sind am Markt zur Durchführung des erfindungsgemäßen Verfahrens Modelle für 10 bis 100 (bzw. 80) Werkzeugsätze verfügbar, wobei jeder Werkzeugsatz normalerweise insgesamt bis zu sechsstempelig sein kann. Während bei einem normalen Rundläufer je Matrizenscheiben-Umdrehung so viele Komprimate gepresst werden, wie Matrizen (mal Bohrungen bei Mehrfachwerkzeug) vorhanden sind, haben sogenannte Doppelrundläufer (sie verfügen über eine besonders hohe Ausstoßleistung) zwei Pressstationen, und es werden - bei einstempeligen Werkzeugsätzen - während einer Matrizenscheiben - Umdrehung zur gleichen Zeit jeweils zwei Matrizen gefüllt, zwei Füllungen komprimiert und zwei ringähnliche Formkörper ausgestoßen. Beispielsweise können für das erfindungsgemäße Verfahren die Exzenterpressen KS, KIS und K III der Firma Kilian, D-50735 Köln, verwendet werden. Besonders geeignet sind für das erfindungsgemäße Verfahren jedoch Rundläufer der Firma Kilian (z. B. der T-Reihe, der R-Reihe, der S-Reihe und der X-Reihe).

Besonders geeignet ist für das erfindungsgemäße Verfahren der Kilian Doppelrundläufer vom Typ RX 73 sowie der Rundläufer Kilian Synthesis 700-77 A. Ferner eigenen sich für das erfindungsgemäße Verfahren die Rundläufer der Korsch AG, D-13509 Berlin, wie z. B. die Korsch Rundläufer PH800 und PH865. Die individuelle Gestaltung des oberen Stempels, des Mittelstiftes MF, des unteren Stempels und der Matrize samt Matrizenbohrung (d. h., des Werkzeugs) obliegt dabei dem Anwender. Zur Anwendung in einem Kilian Rundläufer kann deren Ausgestaltung in einer für das erfindungsgemäße Verfahren besonders vorteilhaften Weise z. B. wie folgt sein (die numerischen Adressen beziehen sich dabei (wie stets in dieser Schrift) auf die dieser Schrift beiliegenden Figuren). Die einzelne Matrize (1) ist so gefertigt, dass sie genau in die in einer Matrizenscheibe befindliche Aufnahmeöffnung passt. Sieht man von der Matrizenbohrung (2) ab, weist die Matrize anwendungstechnisch zweckmäßig die Form eines Kreiszylinders mit einer planen oberen und einer planen unteren Stirnfläche auf, in dessen äußerer Wand auf halber Höhe entweder ein horizontal verlaufender Ring oder eine runde Vertiefung (3) ausgefräst ist. Sie dient der Fixierung der Matrize in der Matrizenscheibe (z.B. mittels wenigstens einer Befestigungsschraube, die z. B. vom Matrizenscheibenrand horizontal zur Aufnahmeöffnung für die Matrize führen oder auf einem Teilkreis von einer Matrize zu der zu dieser benachbarten Matrize verlaufen kann). Erfindungsgemäß zweckmäßig ist der (zu dieser Matrize gehörige) obere Stempel als ein oberer Einlegestempel (4), und der (zu dieser Matrize gehörige) untere Stempel als ein unterer Einlegestempel (5) gefertigt. Der untere Einlegestempel (der obere Einlegestempel) kann mittels einer unteren Schraubkappe (6) (mittels einer oberen Schraubkappe (7)), in die der untere Einlegestempel (der obere Einlegestempel) eingelegt werden kann, auf einen unteren Schaft (8) (auf einen oberen Schaft (9)) zentriert aufgeschraubt werden. Der unter Schaft (der obere Schaft) läuft in einen unteren Schaftkopf (10) (läuft in einen oberen Schaftkopf (11)) aus, der in den Führungsschienen des Rundläufers gleitet. Der untere Einlegestempel (der obere Einlegestempel) läuft in den eigentlichen unteren Stempel (12) (in den eigentlichen oberen Stempel (13)) aus (d. h., der erfindungsgemäß relevante Stempel ist in beiden Fällen der Hals, in den der jeweilige Einlegestempel ausläuft).

Der Boden (14) des oberen Einlegestempels (auch "Einsetzstempel" genannt) liegt im eingeschraubten Zustand auf der kreisförmigen Druckfläche (15) des oberen Schaftes auf. Die Gestaltung als Einlegestempel eröffnet bei Verwendung derselben Schafte eine hohe Flexibilität.

Der kreisringförmige Boden (16) des unteren Einsetzstempels liegt im eingeschraubten Zustand auf der kreisringförmigen Druckfläche (17) des unteren Schaftes auf. Die Kreisringöffnung der Druckfläche (17) ist als zylindrischer Hohlraum in den unteren Schaft fortgeführt. Er kann die Fortsetzung des Mittelstiftes MF (18) aufnehmen. Über eine seitliche Öffnung (eine Nut) des unteren Schaftes kann der Mittelstift MF mittels eines Mittelstifthalters (19) positioniert (längs der Bohrachse B relativ zur Matrize und deren Matrizenbohrung fixiert werden) werden.

Der Mittelstifthalter selbst wird mittels einer Schraube an der Matrizenscheibe befestigt. Figur 1 zeigt in einer Art Explosionsdarstellung die vorstehend aufgeführten Einzelelemente im Längsschnitt.

Figur 6 zeigt einen Ausschnitt eines durch die Matrizenscheibe geführten Längsschnitts. Er zeigt die in die Aufnahmeöffnung der Matrizenscheibe (20) eingelegte Matrize (1) sowie den horizontal verlaufenden Ring (3) zu ihrer Fixierung mittels einer Befestigungsschraube. Der Teil der Matrizenscheibe, in dessen Aufnahmeöffnungen die Matrizen (1) eingelegt sind, soll in dieser Schrift auch als Matrizenscheibenzunge (21) bezeichnet werden. Desweiteren zeigt die Figur 6 die in der Matrizenscheibe oberhalb und unterhalb der Aufnahmeöffnung für die Matrize (1) angebrachten Führungsbohrungen (22) für die Schafte (8) und (9). Auf der Innenwand der jeweiligen Führungsbohrung (22) mit seiner Mantelfläche vertikal gleitend, kann der untere Schaft (8) bzw. der obere Schaft (9) angehoben oder abgesenkt werden. Der Teil der Matrizenscheiben, der die Führungsbohrungen für die oberen Schafte enthält, soll in dieser Schrift auch als Matrizenscheibenstirn (23) bezeichnet werden. Der Teil der Matrizenscheibe, der die Führungsbohrungen für die unteren Schafte enthält, soll in dieser Schrift auch als Matrizenscheibenkinn (24) bezeichnet werden. Der Mittelstifthalter (19) ist in der Figur 6 von unten mit der Matrizenscheibe (20) verschraubt. Der der zu seinem Kopf hin mit einem breiteren Querschnitt auslaufende Mittelstift MF (18) führt vom Mittelstifthalter (19) gehalten durch den unteren Schaft und den unteren Einsetzstempel hindurchgeführt bis zur planen Stirnfläche der Matrize (1), mit der die plane Stirn des Mittelstiftes MF (18) bündig abschließt. Insbesondere wenn Mehrfachwerkzeuge verwendet werden, sollten sich die Schafte in der Führungsbohrung (22) nicht drehen. Dies erreicht man durch Keilnuten im Schaft und Keile längs der Innenwand der Führungsbohrung. Der obere Schaftkopf (11) und der untere Schaftkopf (10) sitzen in einer feststehenden "Oberstempelführungsschiene" bzw. "Unterstempelführungsschiene", die die Figur 6 nicht zeigt. Die eingelegte Matrize (1) ist eine Matrize mit kongruentem doppeltem Kegelstumpf. Die Arbeitsweise der Rundlaufpresse ist nun schematisch wie folgt (dieses Arbeitsprinzip ist bei allen Rundläufern im wesentlichen gleich).

Die z. B. durch eine Schnecke oder ein Zahnrad angetriebene Matrizenscheibe dreht sich in Horizontalebene um ihre Achse. Die mit ihrem jeweiligen Schaftkopf in feststehenden Führungsschienen (in der Regel Edelstahl- oder Kunststoffschienen) sitzenden Schäfte folgen beim Drehen der Matrizenscheibe in der jeweiligen Führungsschiene gleitend deren Höhenprofil. Der den unteren Stempel tragende untere Schaft gleitet im Rahmen der Drehbewegung der Matrizenscheibe auf seiner Gleitbahn zunächst bis zum Füllschuh, wo er und damit auch der untere Stempel nach unten gezogen wird, so dass sich die obere Stirnfläche des unteren Stempels in der Matrizenbohrung auf ihrer Füllhöhe befindet. Im weiteren Verlauf der Drehbewegung wird der oberhalb der oberen Stirnfläche des unteren Stempels befindliche freie Raum der Matrizenbohrung mit dem erfindungsgemäß zu verdichtenden feinteiligen Haufwerk (dem Füllgut) aus dem Füllschuh heraus gefüllt. Beim Weiterdrehen der Matrizenscheibe wird der untere Schaft und mit ihm der untere Stempel angehoben, so dass sich die obere Stirnfläche des unteren Stempels in der Matrizenbohrung auf ihrer Füllmengenhöhe befindet. Überschüssiges Füllgut wird nach oben gedrückt und im weiteren Verlauf der Drehbewegung abgestrichen. Dann wird der untere Schaft und mit ihm der untere Stempel wieder heruntergezogen, so dass sich die obere Stirnfläche des unteren Stempels in der Matrizenbohrung in derjenigen Höhe befindet, auf die sich der Ausgangsabstand A bezieht (hier auch "Presshöhe" genannt). Während der Füllung schwebt der obere Stempel über dem Füllschuh und gleitet nun entsprechend dem Verlauf der Führungsschiene für den oberen Schaft abwärts, bis er mit seiner unteren Stirnfläche das in der Matrizenbohrung befindliche feinteilige Haufwerk berührt. Damit ist das pulverförmige Haufwerk in den Füllraum eingebracht und der Zustand des Ausgangsabstands A erreicht. Sowohl der obere Schaftkopf als auch der unter Schaftkopf werden im Rahmen der Weiterdrehung der Matrizenscheibe über jeweils eine Druckrolle gefahren, und dadurch sowohl der obere Stempel als auch der unter Stempel gegen das in den Füllraum eingebrachte Füllgut gepresst (der untere Stempel wird angehoben, der obere Stempel weiter abgesenkt), bis der Endabstand E erreicht ist. Während der Verdichtung zwischen den Druckrollen kann bedarfsgerecht eine Weghaltezeit erreicht werden, während der der Abstand von Ober- und Unterstempel konstant gehalten wird (bei Exzentertablettiermaschinen gibt es keine Zeitspanne, in der der Abstand zwischen Ober- und Unterstempel konstant bleibt; die Verdichtung wird nur durch die Eintauchtiefe des Oberstempels in das pulverförmige Haufwerk bedingt).

Die Weghaltezeit, während der der Verdichtungsdruck annähernd konstant ist, begünstigt zeitabhängige plastische Verformungsvorgänge im zu verdichtenden Haufwerk. Dann wird der obere Schaft bedingt durch den Verlauf seiner Führungsschiene wieder angehoben, um den oberen Stempel vom erzeugten ringähnlichen Formkörper abzuheben. Der untere Schaft und mit ihm der untere Stempel wird durch das Gleiten des unteren Schaftkopfs in der Aushebebahn der Führungsschiene angehoben und der auf seiner oberen Stirnfläche befindliche ringähnliche Vorläuferformkörper aus der Matrizenbohrung herausgeführt, und durch einen Abstreifer abgestreift (das erfindungsgemäße Verfahren ermöglicht in vorteilhafter Weise besonders geringe Auswurfkräfte; in der Regel liegen diese bei Verwendung von frischen Matrizen im Bereich von 0,15 bis 1,5 kN; im Verlauf der weiteren Ausübung des Verfahrens steigt die erforderliche Auswurfkraft in der Regel an; erreicht dieser Anstieg ca. 700 N, wird die Matrize normalerweise gedreht oder ausgetauscht). Über eine Rinne rutscht der ringähnliche Vorläuferformkörper anschließend in einen Lagerbehälter. Im Rahmen der weiteren Drehbewegung der Matrizenscheibe wird der obere Stempel durch das Gleiten des oberen Schaftkopfs in seiner Obergleitbahn bis zu seiner höchsten Stelle geschoben, bis er sich wieder über dem Füllschuh befindet.

Der untere Stempel wird inzwischen durch das Weitergleiten des unteren Schaftkopfes in seiner Untergleitbahn wieder nach unten gezogen (abgesenkt), so dass er sich auf der Untergleitbahn ebenfalls wieder unter dem Füllschuh und seine obere Stirnfläche sich in der Matrizenbohrung wieder auf ihrer Füllhöhe befindet. Anschließend wiederholt sich der beschriebene Vorgang mit der Periodizität der Drehbewegung der Matrizenscheibe.

Es ist vorteilhaft, wenn die Füllung der Matrizenbohrung mit Füllgut bereits während der Absenkung des unteren Stempels auf Füllhöhe erfolgt, damit beim Füllen der Matrizenbohrung nicht zuviel Luft eingeschlossen wird. Im Verlauf einer vollständigen Umdrehung der Matrizenscheibe werden die Schafte aus den Führungsbohrungen niemals vollständig herausgeführt.

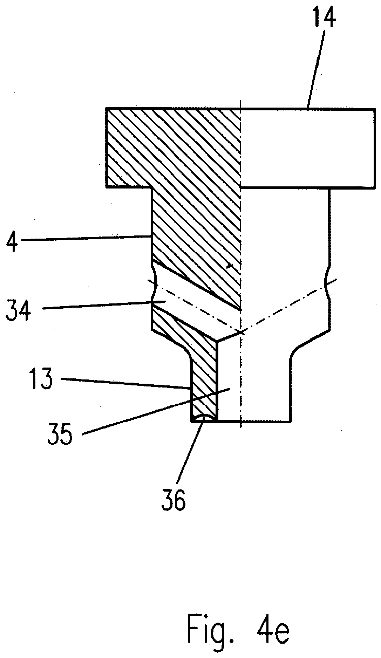

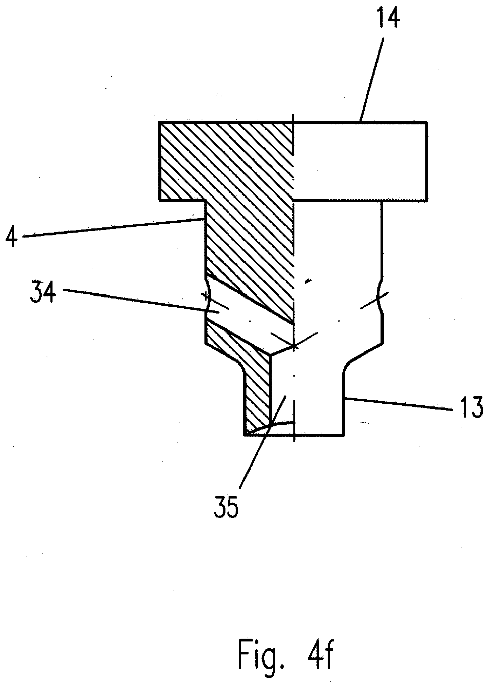

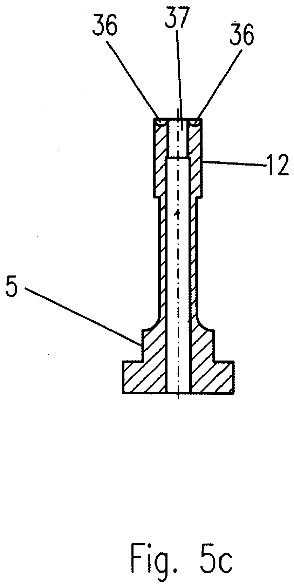

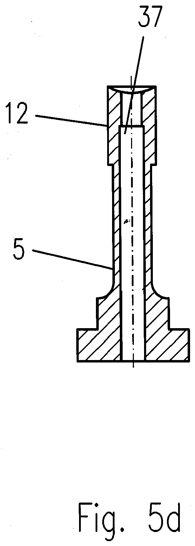

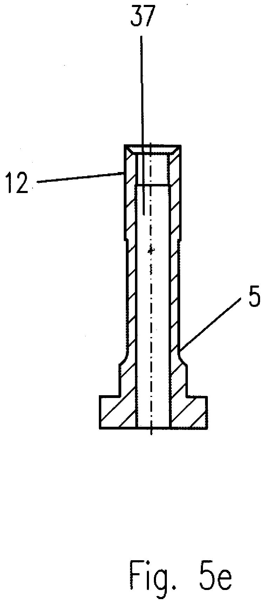

Erfindungsgemäß bevorzugt werden Rundläufer eingesetzt, bei denen nicht wie eben beschrieben durch Verwendung eines Paares von Druckrollen nur ein Verdichtungsvorgang je ringähnlichem Vorläuferformkörper, sondern durch Verwendung zweier dicht hintereinander angeordneter Druckrollenpaare eine Vor- (auf den vorläufigen Endabstand $E^V$ der beiden Stirnflächen) und eine Hauptverdichtung (auf den Endabstand E, wobei $E^V > E$) pro ringähnlichem Vorläuferformkörper durchgeführt wird (die Vordruckrolle ist in der Regel in einfacher Weise kleiner dimensioniert als die Hauptdruckrolle. Die Anwendung einer Vorverdichtung auf einen vorläufigen Endabstand $E^V$ (> E) der beiden Stirnflächen gewährleistet eine bessere Entlüftung beim Verdichten sowie eine gleichmäßigere Verdichtung des pulverförmigen Haufguts, da das Vorverdichten das Füllgut in einen vergleichsweise einheitlichen Ordnungszustand überführt. Generell ist ein langsames Verpressen vorteilhaft für eine gute Entgasung. Die Seitenwanddruckfestigkeit des resultierenden ringähnlichen Vorläuferformkörpers kann auch dadurch verbessert werden, dass man nach der Vorverdichtung zunächst eine Entlastung und sich an diese anschließend erst die Hauptverdichtung durchführt. Der Vollständigkeit halber sind in den Figuren 5c, 5d und 5e drei untere Einlegestempel dargestellt, bei denen die obere Stirnfläche des unteren Stempels nicht plan ist. Der untere Einlegestempel in Figur 5c läuft dabei in einen unteren Stempel aus, in dem die Lehre der EP-A 184790 umgesetzt ist. Die Figuren 4e und 4f zeigen entsprechend ausgestaltete obere Einlegestempel.

Als Füllschuhe kommen für das erfindungsgemäße Verfahren im Fall von Rundläufern z. B. Rüttelfüllschuhe, Vibrationsfüllschuhe und Rührwerkfüllschuhe in Betracht. Besonders bevorzugt werden jedoch Rührflügelfüllschuhe verwendet. Letztere wurden auch in allen Ausführungsbeispielen eingesetzt.

Ferner sei an dieser Stelle noch festgehalten, dass für das erfindungsgemäße Verfahren die Verwendung von solchen einfachen Rundläufern oder Doppelrundläufern besonders vorteilhaft ist, bei denen die Matrizenscheibe (20) auswechselbar gelagert ist. Ein solcher Doppelrundläufer ist z. B. der Doppelrundläufer Synthesis 700 der Firma Kilian. Günstig an diesem Doppelrundläufer ist auch, dass er mit Vorverdichtung und Hauptverdichtung arbeitet. Rundläufermaschinen sind z. B. in den Schriften DE-A 2624853, DE-A 19733969 und DE-A 2435777 beschrieben. Im übrigen sind die Werkzeuge für eine erfindungsgemäße Tablettierung sehr präzise zu fertigen und die diesbezüglich geltenden Normen des jeweiligen Landes (z. B. die DIN ISO 2768) zu erfüllen. Die jeweiligen Oberflächen der Werkzeuge sollten möglichst glatt gefertigt sein.

Insbesondere für den Fall, dass das erfindungsgemäß zu verdichtende pulverförmige Haufwerk wenigstens ein Metalloxid (z. B. aus der Gruppe umfassend Aluminiumoxid, Wolframoxid, Antimonoxid, Zirkoniumoxid, Wismutoxid, Molybdänoxid, Siliciumoxid, Magnesiumoxid sowie Mischoxide, die wenigstens zwei der in den vorgenannten Metalloxiden enthaltenen Metallelemente enthalten (z. B. Mischoxide des Wismuts und des Wolframs wie z. B. $Bi_2W_2O_9$)), ein Metallhydroxid, ein Metallhydrogenphosphat und/oder wenigstens ein Metallnitrat (unter diesem Begriff sollen auch Metallnitrathydrate subsumieren) wie z. B. Kobaltnitrat, Eisennitrat, Wismutnitrat, Nickelnitrat, Cäsiumnitrat, Kupfernitrat, Calciumnitrat und Magnesiumnitrat enthält (derartige pulverförmige Haufwerke sollen im Folgenden als pulverförmige Haufwerke HW* bezeichnet werden), ist es erfindungsgemäß vorteilhaft, wenn der obere Stempel und der untere Stempel für das erfindungsgemäße Verfahren aus dem Werkzeugstahl mit der DIN-Werkstoffnummer 1.2601 gefertigt sind (für den Fall, dass die vorgenannten Stempel als Einlegestempel gefertigt sind, ist der gesamte Einlegestempel anwendungstechnisch zweckmäßig aus dem DIN-Werkstoff 1.2601 gefertigt). Alternativ zum DIN-Werkstoff 1.2601 können die Stempel, insbesondere in den vorgenannten Fällen, auch aus dem DIN-Werkzeugstahl 1.2379 gefertigt sein.

Die Matrize ist im Unterschied zu den Stempeln erfindungsgemäß vorteilhaft hingegen aus einem Werkstoffverbund gefertigt. Dieser besteht auf seiner die Matrizenbohrung berührenden Seite erfindungsgemäß bevorzugt aus einem Hartmetall (25) und auf seiner von der Matrizenbohrung abgewandten Seite vorteilhaft aus einem Werkzeugstahl (26). Letzterer hat vorzugsweise die folgende Elementzusammensetzung WS:

| | |
|---|---|
| 1,50 bis 1,80 Gew.-% | C, |
| 0,10 bis 0,40 Gew.-% | Si, |
| 0,10 bis 0,50 Gew.-% | Mn, |
| ≥ 0 bis 0,05 Gew.-% | P, |
| ≥ 0 bis 0,05 Gew.-% | S, |
| 10 bis 13 Gew.-% | Cr, |
| 0,50 bis 0,80 Gew.-% | Mo, |
| 0,10 bis 1,10 Gew.-% | V, |
| ≥ 0 bis 0,60 Gew.-% | W, und |
| ≥ 0 bis 0,10 Gew.-% | eines oder mehrere seltene Erdmetalle, |
| und im unreini | übrigen Fe und herstellungsbedingte Vergungen, wobei die Prozentangaben jeweils |
| auf | das Gesamtgewicht bezogen sind. |

Besonders bevorzugte Werkzeugstähle WS sind die DIN-Werkstoffe 1.2601 und 1.2379. D. h., die Matrize besteht erfindungsgemäß zweckmäßig aus einem die Matrizenbohrung aufweisenden Kern aus Hartmetall und einer den Kern der Matrize umfassenden Matrizeneinfassung aus einem Werkzeugstahl (vorzugsweise aus einem solchen der Elementzusammensetzung WS). Eine Wanddicke des die Matrizenbohrung berührenden Hartmetalls von wenigen Millimetern (z.B. 1 bis 10 mm, vielfach 2 bis 8 mm, oder 2 bis 6 mm, oder 2 bis 4 mm) ist dabei in der Regel ausreichend. Dies ist insbesondere im Fall einer erfindungsgemäßen Herstellung von ringähnlichen Formkörpern F zutreffend. Die Wanddicke der Matrizeneinfassung wird normalerweise bei wenigen Zentimetern (z.B. 0,5 bis 3 cm, oder 1 bis 2 cm) liegen (insbesondere im Fall einer Herstellung von ringähnlichen Vorläuferformkörpern F, z. B. $F^{LII}$).

Unter einem Hartmetall soll in dieser Schrift ein gesinterter Verbundwerkstoff aus wenigstens einem Hartstoff, ausgewählt aus der Gruppe der Metallcarbide, Metallnitride und Metallboride (wobei das Metall jeweils bevorzugt wenigstens ein Metall aus der Gruppe bestehend aus W, Ti, Zr, Hf, V, Nb, Ta, Mo und Cr ist), und wenigstens einem weichen, zähen Metall der Gruppe Fe, Co, Ni und Cr verstanden werden. Die Herstellung erfindungsgemäß geeigneter gesinterter Hartmetalle erfolgt normalerweise so, dass man eine überwiegende Menge (in der Regel $\geq$ 80 Gew.-%, vorzugsweise $\geq$ 90 Gew.-%) der hochschmelzenden Hartstoffe (bevorzugt wenigstens ein Metallcarbid) in Pulverform mit einer geringen Menge (in der Regel $\leq$ 20 Gew.-%, vorzugsweise $\leq$ 10 Gew.-%) an tiefer schmelzendem Metallpulver (vorzugsweise möglichst homogen) mischt und auf Temperaturen unterhalb des Schmelzpunktes der hochschmelzenden Hartstoffe erhitzt, wobei die Temperatur und Zeitdauer der Erhitzung so gewählt werden, dass der Hartstoff zu einem Skelett (der Hartphase) zusammensintert, in welches das Metall als Bindephase (Bindemittel) eingelagert ist. Die Korngröße (insbesondere des Hartstoffanteils) im Hartmetallpulver kann dabei z.B. 0,2 $\mu$m bis 15 $\mu$m, vorteilhaft 0,5 bis 3 $\mu$m, besonders vorteilhaft 1 bis 1,5 $\mu$m betragen. Vorteilhaft sollte die Rockwellhärte des Hartmetalls nicht unter 80 liegen, seine Vickers Härte $\geq$ 1500 betragen und die Biegefestigkeit bei $\geq$ 2000 N/mm$^2$ liegen. Die Herstellung von gesinterten Hartmetallen ist z.B. in den Schriften AT-PS 358833, EP-A 1364732, AT-PS 362943 sowie in der Studienarbeit "Ermüdungsverhalten des Hartmetalls G55 Co bei Raumtemperatur", von Frank Hebner aus Erlangen, 7. September 2003, an der Friedrich-Alexander-Universität, Erlangen-Nürnberg, Institut für Werkstoffwissenschaften, Lehrstuhl I - Allgemeine Werkstoffeigenschaften, Prof. Dr. Mughrabi und dem in diesen Schriften zitierten Stand der Technik beschrieben.

Für das erfindungsgemäße Verfahren besonders bevorzugt ist die Verwendung eines Hartmetalls als Matrizenkern, das, bezogen auf sein Gewicht, zu $\geq$ 90 Gew.-% aus Wolframcarbid besteht. Ferner ist es erfindungsgemäß günstig, wenn es zusätzlich zu wenigstens 5 Gew.-% aus Ni oder aus Ni und Cr besteht. Noch besser ist für das erfindungsgemäße Verfahren die Verwendung eines Hartmetalls als Matrizenkern, das, bezogen auf sein Gewicht, zu 90 bis 95 % Gew.-% aus Wolframcarbid (WC), zu $\geq$ 0 bis 1 Gew.-% aus wenigstens einem (in der Regel als kornwachstumshemmender Zusatz wirkendes) Metallcarbid aus der Gruppe bestehend aus Titancarbid (TiC), Tantalcarbid (TaC), Niobcarbid (NbC), Vanadiumcarbid (VC), Chromcarbid ($Cr_3C_2$) und Mischmetallcarbiden (z.B. Tantal-Niob-Carbid (TaNbC)), die wenigstens zwei der in den vorgenannten Metallcarbiden enthaltenen Metalle enthalten, sowie zu 5 bis 10 Gew.-% aus Fe, Co, Ni und/oder Cr besteht, wobei Ni oder Ni und Cr als Bindephase bevorzugt sind.

Ganz besonders bevorzugt wird beim erfindungsgemäßen Verfahren als Hartmetall für den Matrizenkern ein solches verwendet, das aus

| | |
|---|---|
| 90 bis 95 Gew.-% | WC, |
| $\geq$ 0 bis 1 Gew.-% | TiC und/oder TaNbC, und |
| 5 bis 10 Gew.-% | Ni oder Ni und Cr |

besteht.

Unter den vorgenannten Hartmetallen sind wiederum jene erfindungsgemäß bevorzugt,

| | | |
|---|---|---|
| die aus | 89,2 bis 94,8 Gew.-% | WC, |
| | 0,2 bis 0,8 Gew.-% | TiC und TaNbC, |
| sowie | 5 bis 10 Gew.-% | Ni |

bestehen. Zu diesen Hartmetallen gehört unter anderem das erfindungsgemäß für den Matrizenkern besonders günstig verwendbare Hartmetall G 10-Ni der Hartmetall® Gesellschaft in D-70572 Stuttgart.

Alle vorstehenden Ausführungen sind insbesondere für Hartmetallkorngrößen, (d. h. Hartstoffkorngrößen im Hartmetall) von 0,5 $\mu$m bis 2 $\mu$m, bevorzugt von 1 bis 1,5 $\mu$m zutreffend.

Ferner gilt das in dieser Schrift zum Werkstoff (bzw. Werkstoffverbund) für die Matrize Gesagte insbesondere dann, wenn das erfindungsgemäß zu verdichtende pulverförmige Haufwerk ein pulverförmiges Haufwerk HW* ist. Letzteres nicht zuletzt dann, wenn in dem pulverförmigen Haufwerk HW* noch Salpetersäure enthalten ist (dann soll es in dieser Schrift als pulverförmiges Haufwerk HW** bezeichnet werden).

Der Mittenrauhwert $R_a$ (nach DIN 4768) der Innenwand der Matrizenbohrung (insbesondere im Längsabschnitt I und II) sollte beim erfindungsgemäßen Verfahren vorzugsweise nicht mehr als 0,2 $\mu$m und besonders bevorzugt nicht mehr als 0,1 $\mu$m und noch besser nicht mehr als 0,05 $\mu$m betragen.

Im übrigen sollte der Mittenrauhwert $R_a$ der Werkzeuge beim erfindungsgemäßen Verfahren nicht mehr als 1 $\mu$m, vorzugsweise nicht mehr als 0,8 $\mu$m und besonders bevorzugt nicht mehr als 0,4 $\mu$m betragen (der Mittenrauhwert ist der arithmetische Mittelwert der absoluten Beträge der Abstände des Rauheitsprofils von der mittleren Linie innerhalb der Messstrecke). Entsprechend geringe Rauheiten sind durch Polieren zu erreichen.

Die Herstellung einer erfindungsgemäß zu verwendenden Matrize mit z. B. einem Kern aus dem Hartmetall G10-Ni und z. B. einer Matrizeneinfassung aus dem DIN-Werkstoff 1.2379 ist in einfacher Weise z. B. durch sogenanntes Einschrumpfen möglich.

Es wird dabei zunächst die Matrizeneinfassung aus dem Werkzeugstahl hergestellt. Diese wird dann erhitzt, wobei sie sich ausdehnt. In die ausgedehnte Matrizeneinfassung kann der Hartmetallkern eingefügt werden. Beim Abkühlen zieht sich die Matrizeneinfassung wieder zusammen und bildet mit dem Hartmetallkern einen quasi nahtlosen Verbund.

Um den in der WO 2005/115733 beschriebenen Problemen Rechnung zu tragen, werden die Mittelstifte MF mit Vorteil aus einem DIN-Werkzeugstahl 1.2343 gefertigt.

Dies gilt insbesondere im Fall einer Verdichtung von pulverförmigen Haufwerken HW* und HW** (insbesondere im Fall einer Herstellung von ringähnlichen Vorläuferformkörpern F; vor allem im Fall von ringähnlichen Vorläuferformkörpern $F^{LII}$). Die Matrizenscheibenzunge besteht beim erfindungsgemäßen Verfahren, insbesondere im Fall von pulverförmigem Haufwerk HW* oder HW**, vorteilhaft aus dem DIN Kugelgraphitgrauguß GGG 50 mit einer dünnen Auflage aus dem DIN Werkzeugstahl 1.0425, während die Matrizenscheibenstirn und das Matrizenscheibenkinn anwendungstechnisch zweckmäßig aus dem DIN Werkzeugstahl 1.6850 (nitriert) gefertigt sind. Der untere Schaft, der obere Schaft und die zugehörigen Schaftköpfe können beim erfindungsgemäßen Verfahren ebenso wie die Mittelstifthalter in an sich bekannter Weise z. B. aus dem DIN Werkzeugstahl 1.25550 (gehärtet und angelassen, Rockwellhärtegrad HRC 58 + 2) gefertigt sein.

Die bei der Durchführung des erfindungsgemäßen Verfahrens im Endabstand E von beiden Stempeln (bzw. wenigstens vom Oberstempel) in typischer Weise ausgeübten Pressdrucke liegen üblicherweise im Bereich von 50 bis 5000 kg/cm², vorzugsweise bei 200 bis 3500 kg/cm², besonders bevorzugt 500 bis 2500 kg/cm² und besonders bevorzugt 500 bis 1500 kg/cm².

Die Vordrucke (sie werden im vorläufigen Endabstand $E^V$ ausgeübt) betragen typisch 5 - 500 kg/cm² und die Hauptdrucke liegen meist bei 1000 - 2000 kg/cm². Je höher der angewandte Hauptdruck ist, als desto vorteilhafter erweist sich das erfindungsgemäße Verfahren.

Das erfindungsgemäße Verfahren entfaltet besondere Vorteilhaftigkeit, wenn das zu verdichtende pulverförmige Haufwerk gegenüber Stählen korrosiv wirkende Bestandteile wie z.B. Salpetersäure, Ammoniumsalze und/oder Nitratsalze enthält.

Durch die korrosive Einwirkung kommt es in diesen Fällen bei der Verfahrensausübung zu einer vergleichsweise raschen Aufrauhung der Innenwand der Matrizenbohrung und damit zu einer ebenso raschen Zunahme der Gleitreibung beim Entfernen des gebildeten Formkörpers im Falle einer ausschließlich kreiszylindrischen Matrizenbohrung.

Insbesondere im Fall einer erfindungsgemäßen Herstellung von ringähnlichen Vorläuferformkörpern LII (z.B. $F^{LII}$), wird diesem Problem im wesentlichen vollständig abgeholfen. Letzteres gilt insbesondere dann, wenn die Matrize aus einem Werkstoffverbund gefertigt ist, wie er im Vorstehenden ausgeführt wurde.

Wie bereits verschiedentlich ausgeführt, umfasst das erfindungsgemäße Verfahren die Herstellung von ring-ähnlichen Vorläuferformkörpern aus pulverförmigem Haufwerk, das bereits (bei Normalbedingungen (25 °C, 1 atm) in der Regel festes) wenigstens ein Metalloxid enthält, und/oder wenigstens eine solche Metallverbindung (z. B. ein Metallsalz), die durch thermische Behandlung bei einer Temperatur ≥ 100 °C in ein (bei Normalbedingungen in der Regel festes) Metalloxid überführbar ist (wenigstens durch thermisches Behandeln in Anwesenheit von gasförmigem molekularem Sauerstoff und/oder von gasförmigen Sauerstoff freisetzenden Komponenten; prinzipiell kann die Sauerstoffquelle z. B. in Form eines Peroxids auch Bestandteil des pulverförmigen Haufwerks und damit des erfindungsgemäß resultierenden ringähnlichen Vorläuferkörpers sein). Derartige pulverförmige Haufwerke sollen in dieser Schrift auch als pulverförmige Haufwerke O bezeichnet werden.

Das feste Metalloxid kann dabei ein solches sein, das neben Sauerstoff nur ein oder aber auch mehr als ein (z. B. zwei oder drei) Metallelemente enthält. Ebenso kommen als Metallverbindungen grundsätzlich auch solche in Betracht, die nur ein, oder aber auch mehr als ein (z. B. zwei oder drei) Metallelement enthält.

Ferner umfasst das erfindungsgemäße Verfahren die thermische Behandlung der erfindungsgemäß hergestellten ringähnlichen Vorläuferformkörper. Im Rahmen dieser thermischen Behandlung kommt es dann zur Ausbildung der erfindungsgemäß erwünschten ringähnlichen oxidischen Formkörper. Erfindungsgemäß erfolgt die thermische Behandlung bei Temperaturen ≥ 100 °C, häufig ≥ 150 °C, oder ≥ 200 °C (z. B. 300 bis 800 °C). Insbesondere im Fall einer Herstellung von ringähnlichen oxidischen Trägerformkörpern kann die thermische Behandlung eine solche bei Temperaturen ≥ 600 °C, oder ≥ 1000 °C einbeziehen. 1500 °C werden in den meisten Fällen nicht überschritten.

Erfindungsgemäß wesentlich ist dabei ferner, dass das erfindungsgemäß zu verdichtende pulverförmige Haufwerk, und mit diesem die resultierenden ringähnlichen Vorläuferformkörper, Substanzen (Bestandteile) enthalten, die sich unter den bei ihrer nachfolgenden erfindungsgemäßen thermischen Behandlung angewandten Bedingungen unter Ausbildung von unter den Bedingungen der thermischen Behandlung gasförmigen Verbindungen zersetzen und/oder chemisch umsetzen (z. B. unter Ausbildung von Ammoniak, Wasserdampf, $CO_2$, CO und/oder Stickstoffoxiden). In der Regel beträgt der mit der thermischen Behandlung der ringähnlichen Vorläuferformkörper einhergehende und auf deren Ausgangsgewicht bezogene Gewichtsverlust (infolge des vorgenannten Ausgasens) 0,5 bis 40 Gew.-%, häufig 0,8 bis 35 Gew.-% oder 2 bis 30 Gew.-%.

Eine Ausbildung (Freisetzung) von gasförmigen Verbindungen im Rahmen einer erfindungsgemäßen thermischen Behandlung der erfindungsgemäß erzeugten ringähnlichen Vorläuferformkörper ist normalerweise z.B. dann gegeben, wenn die Bestandteile des erfindungsgemäß zu verdichtenden pulverförmigen Haufwerks (eines Haufwerks O) wenigstens teilweise organischer Natur sind bzw. Hydroxidionen, Carbonationen, Hydrogencarbonationen, Ammoniumionen, Hydrogenphosphationen und/oder Nitrationen enthalten, die sich bei der erfindungsgemäßen thermischen Behandlung in der Regel wenigstens teilweise zersetzen. Dabei können die Hydroxidionen, Carbonationen, Hydrogencarbonationen, Ammoniumionen, Hydrogenphosphationen und/oder Nitrationen grundsätzlich bereits Bestandteil der nicht Oxid seienden Metallverbindungen in dem erfindungsgemäß zu verdichtenden pulverförmigen Haufwerk sein. Sie können dem erfindungsgemäß zu verdichtenden pulverförmigen Haufwerk aber auch zusätzlich (oder nur) als Bestandteil von bei der nachfolgenden thermischen Behandlung der ringähnlichen Vorläuferformkörper Poren bildenden, keine Metallverbindungen seienden, Substanzen zugegeben werden.

Als solche porenbildende Substanzen kann das erfindungsgemäß zu verdichtende pulverförmige Haufwerk (ein Haufwerk O) z.B. $NH_4OH$, $(NH_4)_2CO_3$, $NH_4HCO_3$, $NH_4NO_3$, Harnstoff, $NH_4CHO_2$, $NH_4CH_3CO_2$, $NH_4HSO_4$, $(NH_4)_2SO_4$, Ammoniumoxalat und/oder Hydrate der vorgenannten Ammoniumsalze zugesetzt enthalten. Selbstverständlich ist das erfindungsgemäße Verfahren aber auch dann von Vorteil, wenn das erfindungsgemäß zu verdichtende pulverförmige Haufwerk (ein Haufwerk O) als sich im Rahmen der nachfolgenden thermischen Behandlung gasförmig zersetzende und/oder sich in gasförmige Verbindungen chemisch umsetzende organische Substanzen wie z.B. Stärken (wie Kartoffelstärke, Maisstärke), gemahlene Nussschale, feinteiliges Kunststoffgranulat (wie z.B. Polyethylen, Polypropylen), Cellulose, Graphit, Stearinsäure, Malonsäure, Salze der Stearinsäure, Salz der Malonsäure u.a. zugesetzt enthält.

Des weiteren kann das erfindungsgemäß zu verdichtende pulverförmige Haufwerk (ein Haufwerk O) als weitere Hilfsmittel Gleitmittel zugesetzt enthalten, die z.B. die Haftreibung zwischen dem erfindungsgemäß hergestellten ringähnlichen Vorläuferformkörper und der Innenwand der Matrizenbohrung herabsetzen. Als solche Gleit- bzw. Schmiermittel können z.B. Graphit, Ruß, Polyethylenglykol, Stearinsäure, Salze der Stearinsäure, Stärke, Polyacrylsäure, Mineralöl, Pflanzenöl, Wasser, Bornitrid, Bortrifluorid, Glycerin, feinteiliges Teflonpulver und/oder Celluloseether verwendet werden (es sei an dieser Stelle festgehalten, dass das erfindungsgemäße Verfahren auch dadurch besticht, dass es die Minimierung des Bedarfs derartiger Hilfsmittel ermöglicht). Vorgenannte Gleitmittel können sich im Rahmen einer sich an die erfindungsgemäße Formgebung anschließenden thermischen Behandlung der ringähnlichen Vorläuferformkörper gegebenenfalls auch unter Bildung gasförmiger Substanzen zersetzen bzw. chemisch umsetzen.

Als weitere Formgebungshilfsmittel kann das erfindungsgemäß zu verdichtende pulverförmige Haufwerk (ein Haufwerk O) sogenannte Verstärkungsmittel zugesetzt enthalten, die den Zusammenhalt im resultierenden Komprimat fördern. Solche Verstärkungsmittel können beispielsweise Mikrofasern aus Glas, Asbest, Siliciumcarbid und/oder Kaliumtitanat sein.

Bezogen auf die Gesamtmenge des erfindungsgemäß zu verdichtenden pulverförmigen Haufwerks (eines Haufwerks O) wird die Gesamtmenge an Formgebungshilfsmitteln in der Regel nicht mehr als 30 Gew.-%, meist nicht mehr als 20 Gew.-% und vielfach nicht mehr als 10 Gew.-% betragen.

Üblicherweise wird das pulverförmige Haufwerk (ein Haufwerk O) beim erfindungsgemäßen Verfahren anfasstrocken eingesetzt. Es kann jedoch bis zu 10 % seines Gesamtgewichts Substanzen zugesetzt enthalten, die bei Normalbedingungen (25°C, 1 atm) flüssig sind. Das erfindungsgemäße Verfahren ist aber auch dann anwendbar, wenn das pulverförmig Haufwerk (ein Haufwerk O) überhaupt keine solchen flüssigen Substanzen mehr enthält. Selbstverständlich kann das pulverförmige Haufwerk (ein pulverförmiges Haufwerk O) auch feste Solvate (z.B. Hydrate) enthalten, die solche flüssigen Substanzen in chemisch und/oder physikalisch gebundener Form aufweisen. Derartige Solvate zersetzen sich bei der thermischen Behandlung in der Regel ebenfalls unter gasförmiger Freisetzung der Solvatphase. Erfindungsgemäß vorteilhaft liegt die Restfeuchte des erfindungsgemäß zu verdichtenden Haufwerks bei < 10 Gew.-%, in Abwesenheit von Kristallwasser (bzw. Kristallsolvatphase) in der Regel bei < 5 Gew.-%.

Die Partikeldurchmesser des erfindungsgemäß zu verdichtenden feinteiligen pulverförmigen Haufwerks (eines pulverförmigen Haufwerks O) werden (zugesetzte Formgebungs- und Porosierungsmittel ausgenommen) für wenigstens 90 % des Gewichts des pulverförmigen Haufwerks (zugesetzte Formgebungs- und Porasierungsmittel nicht miteinbezogen) anwendungstechnisch zweckmäßig im Bereich von 10 bis 2000 $\mu$m, vielfach im Bereich von 20 bis 1800 $\mu$m, oder von 30 bis 1700 $\mu$m, oder von 40 bis 1600 $\mu$m, oder von 50 bis 1500 $\mu$m liegen. Besonders häufig werden vorgenannte Partikeldurchmesser im Bereich von 100 bis 1500 $\mu$m oder 150 bis 1500 $\mu$m liegen.

Grundsätzlich kann eine erfindungsgemäße thermische Behandlung von erfindungsgemäß hergestellten ringähnlichen Vorläuferformkörpern sowohl unter Vakuum, unter inerter Atmosphäre (z.B. $N_2$, Edelgase, Wasserdampf, $CO_2$ etc.), unter reduzierender Atmosphäre (z.B. $H_2$ oder $NH_3$) oder unter oxidierender Atmosphäre erfolgen. In der Regel werden oxidierende Atmosphären molekularen Sauerstoff enthalten. Typische oxidierende Atmosphären sind Gemische aus Inertgas ($N_2$, Edelgase, Wasserdampf, $CO_2$ etc.) und molekularem Sauerstoff, Üblicherweise wird der Gehalt an molekularem Sauerstoff dabei wenigstens 0,1 Vol.-%, häufig wenigstens 0,2 Vol.-%, vielfach wenigstens 0,5 Vol.-%, oft wenigstens 1 Vol.-%, oder wenigstens 10 Vol.-%, oder wenigstens 20 Vol.-% betragen. Selbstverständlich kann der Gehalt an molekularem Sauerstoff in solchen Gemischen aber auch 30 Vol.-%, oder 40 Vol.-%, oder 50 Vol.-%, oder mehr betragen. Selbstverständlich kommt als oxidierende Atmosphäre für eine solche thermische Behandlung aber auch reiner molekularer Sauerstoff in Betracht. Häufig wird eine oxidierende thermische Behandlung unter Luft erfolgen. Generell kann die thermische Behandlung unter stehender oder unter fließender Gasatmosphäre (z.B. im Luftstrom) erfolgen.

Der Begriff der Atmosphäre (bzw. der Gasatmosphäre) in welcher die thermische Behandlung erfolgt, ist dabei in dieser Schrift so zu verstehen, dass er sich aus den erfindungsgemäß hergestellten ringähnlichen Vorläuferformkörpern im Rahmen der thermischen Behandlung aufgrund von Zersetzungsvorgängen und/oder chemischen Reaktionsvorgängen sich entwickelnde Gase nicht umfasst. Selbstverständlich kann die Gasatmosphäre, in welcher die thermische Behandlung erfolgt, aber auch ausschließlich oder teilweise aus diesen Gasen bestehen. Auch können sowohl die Behandlungstemperatur als auch die Behandlungsatmosphäre über die Dauer der thermischen Behandlung zeitlich konstant oder zeitlich variabel gestaltet sein. Für den Fall, dass als Ergebnis der anschließenden thermischen Behandlung von erfindungsgemäß erhältlichen ringähnlichen Vorläuferformkörpern ringähnliche (oxidische) Vollkatalysatoren angestrebt werden, deren Aktivmasse wenigstens ein Multimetalloxid ist, erfolgt die thermische Behandlung häufig bei Temperaturen von 150 bis 650°C, vielfach 200 bis 600°C, oft 250 bis 550°C und vielfach 300 bis 500°C. Der Begriff "Multimetalloxid" meint in dieser Schrift kein einfaches Gemisch verschiedener Metalloxide, sondern eine komplexe Polyoxyverbindung, die neben Sauerstoff wenigstens zwei voneinander verschiedene Metalle (metallische Konstituenten) enthält.

Wie in dieser Schrift bereits mehrfach angesprochen, eignet sich das erfindungsgemäße Verfahren insbesondere zur Herstellung von ringähnlichen Vorläuferformkörpern, aus denen durch thermische Behandlung ringähnliche (oxidische) Katalysatorformkörper bzw. vereinfacht ausgedrückt, ringähnliche Katalysatoren erhältlich sind (z.B. dann, wenn die katalytisch aktive Komponente des ringähnlichen Katalysatorformkörpers ein Multimetalloxid ist). Derartige erfindungsgemäß erhältliche ringähnliche Vorläuferformkörper sollen in dieser Schrift auch als ringähnliche Katalysatorvorläuferformkörper bezeichnet werden.

Dabei kann der ringähnliche Katalysatorformkörper im einfachsten Fall nur aus der katalytisch aktiven Komponente (z.B. dem Multimetalloxid) bestehen. Er kann die katalytisch aktive Komponente (z.B. das Multimetalloxid) aber auch mit inertem Material verdünnt enthalten. In beiden Fällen spricht man von ringähnlichen Vollkatalysatorformkörpern. Ist die aktive Komponente ein Multimetalloxid soll in dieser Schrift von ringähnlichen Multimetalloxidvollkatalysatoren gesprochen werden.

Ringähnliche Multimetalloxidvollkatalysatoren eignen sich insbesondere für die heterogene Katalyse von partiellen Gasphasenoxidationen (vgl. z.B. DE-A 102005037678, die Deutsche Anmeldung mit dem Aktenzeichen 102007028332.8, die Deutsche Anmeldung mit dem Aktenzeichen 102007025869.2, die Deutsche Anmeldung mit dem Aktenzeichen 102007017080.9 und die Deutsche Anmeldung mit dem Aktenzeichen 102007003778.5) organischer Verbindungen mit molekularem Sauerstoff.

Unter einer vollständigen Oxidation einer organischen Verbindung mit molekularem Sauerstoff soll in dieser Schrift verstanden werden, dass die organische Verbindung unter reaktiver Einwirkung von molekularem Sauerstoff so umgesetzt wird, dass der in der organischen Verbindung insgesamt enthaltene Wasserstoff in Oxide des Wasserstoffs umgewandelt wird. Alle davon verschiedenen exothermen Umsetzungen einer organischen Verbindung unter reaktiver Einwirkung von molekularem Sauerstoff werden hier als Partialoxidationen einer organischen Verbindung zusammengefasst (z.B. einschließlich der Ammoxidationen und Oxychlorierungen, die im gleichzeitigen Beisein von Ammoniak bzw. Chlorwasserstoff durchgeführt werden). Im besonderen sollen in dieser Schrift unter Partialoxidationen solche exothermen Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält, als vor Durchführung der Partialoxidation.

Als Beispiele für heterogen katalysierte partielle Gasphasenoxidationen seien an dieser Stelle beispielhaft diejenige von Propylen zu Acrolein, diejenige von iso-Buten zu Methacrolein, diejenige von Methacrolein zu Methacrylsäure und diejenige von $C_4$-Kohlen-wasserstoffen zu Maleinsäureanhydrid genannt. Üblicherweise werden heterogen katalysierte partielle Gasphasenoxidationen in z.B. mit Salzschmelzen gekühlten Rohrbündelreaktoren durchgeführt. Die Katalysatoren befinden sich dabei, gegebenenfalls mit inerten Formkörpern verdünnt, in den vom Reaktionsgasgemisch durchströmten Reaktionsrohren.

Zur Herstellung von ringähnlichen Multimetalloxidvollkatalysatoren kann nun so vorgegangen werden, dass man aus Quellen der elementaren Konstituenten des katalytisch aktiven Multimetalloxids sowie nach Bedarf mitzuverwendenden Formgebungshilfsmitteln (z.B. Porosierungsmittel, Gleitmittel und Verstärkungsmittel) ein feinteiliges Haufwerk erzeugt, und aus diesem nach dem erfindungsgemäßen Verfahren zunächst ringähnliche Multimetalloxid-Vollkatalysatorvorläuferformkörper herstellt. Als Quellen für die elementaren Konstituenten des Multimetalloxids können dabei (bei Normalbedingungen in der Regel im festen Aggregatzustand befindliche) Metalloxide und/oder solche Metallverbindungen verwendet werden, die durch Erhitzen (thermisches Behandeln) in (bei Normalbedingungen in der Regel im festen Aggregatzustand befindliche) Oxide überführbar sind (wenigstens durch thermisches Behandeln in Anwesenheit von gasförmigem molekularem Sauerstoff und/oder von gasförmigen Sauerstoff freisetzenden Komponenten).

Durch nachfolgende thermische Behandlung der ringähnlichen Multimetalloxid-Vollkatalysatorvorläuferformkörper (z.B. im Temperaturbereich von 200 bis 800°C, oder 300 bis 600°C) sind dann die ringähnlichen Multimetalloxidvollkatalysatoren erhältlich.

Die zur erfindungsgemäßen Herstellung von ringähnlichen Multimetalloxid-Vollkatalysatorvorläuferformkörpern zu verwendenden pulverförmigen Haufwerke werden daher in der Regel Haufwerke HW oder Haufwerke HW** sein. Insbesondere aber werden sie pulverförmige Haufwerke O sein. Alle bezüglich der erfindungsgemäßen Verdichtung von pulverförmigen Haufwerken O, HW* und HW** in dieser Schrift gemachten Ausführungen gelten daher in entsprechender Weise. Bei erfindungsgemäß bevorzugten ringähnlichen Multimetalloxid-Vollkatalysatorvorläuferformkörpern handelt es sich in der Regel um ringähnliche Formkörper F, vorzugsweise um ringähnliche Formkörper $F^{LII}$.

Unter anderem eignet sich das erfindungsgemäße Verfahren zur Herstellung der Vorläuferformkörper von solchen ringähnlichen Multimetalloxidvollkatalysatoren, die als katalytisch aktive Komponente wenigstens ein Multimetalloxid enthalten, in welchem das Element Mo, oder das Element V, oder das Element P dasjenige von Sauerstoff verschiedene Element ist, das numerisch (molar gerechnet) am häufigsten enthalten ist (Multimetalloxid meint dabei, dass das Oxid wenigstens zwei von Sauerstoff verschiedene Elemente enthält).

Insbesondere eignet sich das erfindungsgemäße Verfahren zur Herstellung der Vorläuferformkörper (insbesondere von ringähnlichen Vorläuferformkörpern F bzw. $F^{LII}$) von solchen ringähnlichen Multimetalloxidvollkatalysatoren, die als katalytisch aktive Komponente wenigstens ein Multimetalloxid enthalten, das die Elemente Mo und Fe, oder die Elemente Mo, Fe und Bi, oder die Elemente Mo und V, oder die Elemente Mo, V und P, oder die Elemente V und P enthält (vor allem, wenn gleichzeitig die vorgenannte Häufigkeitsbedingung erfüllt ist). Die ersteren ringähnlichen Multimetalloxidvollkatalysatoren in der vorgenannten Reihe eignen sich vor allem für heterogen katalysierte partielle Gasphasenoxidationen von Methanol zu Formaldehyd. Die Zweitgenannten eignen sich vor allem für die heterogen katalysierte partielle Gasphasenoxidation von Propylen zu Acrolein. Die Drittgenannten eignen sich vor allem für heterogen katalysierte partielle Gasphasenoxidationen von Acrolein zu Acrylsäure, die Viertgenannten eignen sich vor allem für die heterogen katalysierte partielle Gasphasenoxidation von Methacrolein zu Methacrylsäure und die Letztgenannten in der vorstehenden Reihe eignen sich vor allem für heterogen katalysierte partielle Gasphasenoxidationen von n-Butan zu Maleinsäureanhydrid.

Zur erfindungsgemäßen Herstellung von ringähnlichen Multimetalloxid-Vollkatalysatorvorläuferformkörpern wird vorzugsweise ein unterer Stempel mit einer planen oberen Stirnfläche und ein oberer Stempel mit einer planen unteren Stirnfläche verwendet (vorzugsweise sind dabei die beiden Stirnflächen zueinander kongruent). Selbstverständlich können ringähnliche Multimetalloxid-Vollkatalysatorvorläuferformkörper aber auch, wie in dieser Schrift beschrieben, mit gekrümmten Stirnflächen hergestellt werden.

Katalytisch aktive Multimetalloxide der vorgenannte Art, einschließlich zur erfindungsgemäßen Herstellung von zugehörigen ringähnlichen Multimetalloxid-Vollkatalysatorvorläuferformkörpern verwendbarer pulverförmiger Haufwerke, finden sich unter anderem in den Schriften WO 2005/030393, EP-A 467 144, EP-A 1 060 792, DE-A 198 55 913, WO 01/68245, EP-A 1060792, Research Disclosure RD 2005- 497012, DE-A 102005035978, DE-A 102005037678, WO 03/78059, WO 03/078310, DE-A 199 22 113, WO 02/24620, WO 02/062737, die Deutsche Anmeldung mit dem Aktenzeichen 102007028332.8, die Deutsche Anmeldung mit dem Aktenzeichen 102007025869.2, die Deutsche Anmeldung mit dem Aktenzeichen 102007017080.9 und US-A 2005/0131253.

Die erfindungsgemäß zu verdichtenden pulverförmigen (Vorläufer)Haufwerke sind dabei in einfachster Weise dadurch erhältlich, dass man von Quellen der elementaren Konstituenten des angestrebten katalytisch aktiven Multimetalloxids ein feinteiliges, möglichst inniges, der Stöchiometrie des gewünschten Multimetalloxids entsprechend zusammengesetztes, formbares Gemisch erzeugt, dem noch die bereits erwähnten Formgebungshilfsmittel (einschließlich Verstärkungshilfsmittel) zugesetzt werden können (und/oder von Anfang an eingearbeitet werden können).

Als Quellen für die elementaren Konstituenten des gewünschten Multimetalloxids kommen, wie bereits gesagt, grundsätzlich solche Metallverbindungen in Betracht, bei denen es sich bereits um Oxide handelt und/oder um solche Metallverbindungen, die durch Erhitzen, wenigstens in Anwesenheit von gasförmigem molekularem Sauerstoff und/oder von gasförmigen Sauerstoff freisetzenden Komponenten, in Oxide überführbar sind. Prinzipiell kann die Sauerstoffquelle auch z. B. in Form eines Peroxids Bestandteil des Vorläufergemisches (des pulverförmigen Haufwerks) selbst sein. Auch kann das pulverförmige (Vorläufer)Haufwerk Verbindungen wie $NH_4OH$, $(NH_4)_2CO_3$, $NH_4NO_3$, Harnstoff, $NH_4CHO_2$, $NH_4CH_3CO_2$, Ammoniumoxalat, Hydrate der vorgenannten Verbindungen und/oder organische Komponenten wie z. B. Stearinsäure zugesetzt enthalten, die bei der thermischen Behandlung als Porenbildner zu vollständig gasförmig entweichenden Verbindungen zerfallen und/oder zersetzt werden können.

Das, vorzugsweise innige, Vermischen der Ausgangsverbindungen (Quellen) zur Herstellung des feinteiligen erfindungsgemäß formbaren pulverförmigen (Vorläufer)Haufwerks, kann in trockener oder in nasser Form erfolgen. Erfolgt es in trockener Form, so werden die Ausgangsverbindungen zweckmäßigerweise als feinteilige Pulver (mit einem

Partikeldurchmesser $d_{50}$ (Zur Bestimmung von Partikeldurchmesserverteilungen sowie den aus diesen entnommenen Partikeldurchmessern $d_{10}$, $d_{50}$ und $d_{90}$ (allgemein $d_x$) wurde das jeweilige feinteilige Pulver über eine Dispergierrinne in den Trockendispergierer Sympatec RODOS (Sympatec GmbH, System-Partikel-Technik, Am Pulverhaus 1, D-38678 Clausthal-Zellerfeld) geführt und dort mit Druckluft trocken dispergiert und im Freistrahl in die Messzelle geblasen. In dieser wird dann nach ISO 13320 mit dem Laserbeugungsspektrometer Malvern Mastersizer S (Malvern Instruments, Worcestshire WR14 1AT, United Kingdom) die volumenbezogene Partikeldurchmesserverteilung bestimmt. Die als Messergebnis angegebenen Partikeldurchmesser $d_x$ sind dabei so definiert, dass X % des Gesamtpartikelvolumens aus Partikeln mit diesem oder einem kleineren Durchmesser bestehen.) zweckmäßig im Bereich 1 bis 200 $\mu$m, vorzugsweise 2 bis 180 $\mu$m, besonders bevorzugt 3 bis 170 $\mu$m und ganz besonders bevorzugt 4 bis 160 $\mu$m, oder 5 bis 150 $\mu$m bzw. 10 bis 150 $\mu$m, oder 15 bis 150 $\mu$m liegend) eingesetzt. Nach Zusatz der Formgebungshilfsmittel kann anschließend die erfindungsgemäße Formgebung erfolgen. Solche Hilfsmittel können beispielsweise Graphit als Gleitmittel sowie Mikrofasern aus Glas, Asbest, Siliciumcarbid und/oder Kaliumtitanat sein. Ganz generell kann eine Ausgangsverbindung Quelle für mehr als einen elementaren Konstituenten sein.

Anstatt das durch Mischen von pulverförmigen Quellen erzeugte Gemisch als solches unmittelbar zur gewünschten Vorläufergeometrie zu formen, ist es häufig zweckmäßig, als einen ersten Formgebungsschritt zunächst eine Zwischenkompaktierung desselben durchzuführen, um das Pulver zu vergröbern (in der Regel auf Partikeldurchmesser $d_{50}$ von 100 bis 2000 $\mu$m, bevorzugt 150 bis 1500 $\mu$m, besonders bevorzugt 400 bis 1000 $\mu$m).

Dabei kann bereits vor der Zwischenkompaktierung z. B. Graphit als Kompaktierhilfsmittel zugesetzt werden. Anschließend erfolgt mit dem vergröberten Pulver die erfindungsgemäße Formgebung, wobei bei Bedarf zuvor nochmals z. B. feinteiliger Graphit (sowie gegebenenfalls weitere Formgebungshilfsmittel (einschließlich Verstärkungsmittel)) zugegeben werden kann.

Vorzugsweise erfolgt das innige Vermischen der Quellen jedoch in nasser Form. Üblicherweise werden dabei die Ausgangsverbindungen z. B. in Form einer wässrigen (aber auch Flüssigkeiten wie iso-Butanol kommen als Lösungs- und/oder Dispergiermedium in Betracht) Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt (aber auch Flüssigkeiten wie iso-Butanol kommen als Lösungsmittel in Betracht). Anschließend wird die erhaltene Lösung oder Suspension getrocknet, wobei der Trocknungsprozess vorzugsweise durch Sprühtrocknung mit Austrittstemperaturen von 100 bis 150 °C erfolgt (in manchen Fällen kann die Trocknung aber auch durch Filtration und anschließende Trocknung des Filterkuchens erfolgen). Der Partikeldurchmesser $d_{50}$ des resultierenden Sprühpulvers beträgt in typischer Weise 10 bis 50 $\mu$m. War Wasser die Basis des flüssigen Mediums, wird das resultierende Sprühpulver normalerweise nicht mehr als 20 % seines Gewichtes, vorzugsweise nicht mehr als 15% seines Gewichtes und besonders bevorzugt nicht mehr als 10 Gew.-% seines Gewichtes an Wasser enthalten. Diese Prozentsätze gelten in der Regel auch bei Anwendung anderer flüssiger Lösungs- bzw. Suspendierhilfsmittel. Nach Zusatz (oder auch ohne einen solchen Zusatz) der gewünschten Formgebungshilfsmittel zur jeweiligen pulverförmig gestalteten Trockenmasse kann das pulverförmige Gemisch als feinteiliges Vorläufergemisch (pulverförmiges Haufwerk) erfindungsgemäß zum gewünschten Multimetalloxid-Vollkatalysatorvorläuferformkörper verdichtet (geformt) werden. Die feinteiligen Formgebungshilfsmittel können aber auch bereits vorab in die Sprühmaische (teilweise oder vollständig) zugesetzt werden.

Eine nur teilweise Entfernung des Lösungs- bzw. Suspendiermittels kann auch dann zweckmäßig sein, wenn seine Mitverwendung als Formgebungshilfsmittel beabsichtigt ist.

Vorab einer Zugabe von z. B. feinteiligem Graphit als Gleitmittel kann auch bereits eine erste thermische Behandlung des Trockenpulvers erfolgen. Nach Zugabe des z. B. Graphits erfolgt dann die erfindungsgemäße Formgebung sowie die sich daran anschließende thermische Weiterbehandlung.

Anstatt das auf dem Sprühpulver fußende feinteilige Vorläufergemisch als solches unmittelbar zur gewünschten Geometrie zu formen, ist es häufig zweckmäßig, als einen ersten Formgebungsschritt zunächst eine Zwischenkompaktierung durchzuführen, um das Pulver zu vergröbern (in der Regel auf Partikeldurchmesser von 100 bis 2000 $\mu$m, bevorzugt 150 bis 1500 $\mu$m, besonders bevorzugt 400 bis 1000 $\mu$m).

Dabei kann bereits vor der Zwischenkompaktierung z. B. Graphit als Kompaktierhilfsmittel zugesetzt werden. Anschließend erfolgt auf der Grundlage des vergröberten Pulvers die endgültige (eigentliche), die erfindungsgemäße Formgebung, wobei bei Bedarf zuvor nochmals feinteiliger Graphit (sowie gegebenenfalls weitere Formgebungshilfsmittel wie z. B. Verstärkungsmittel) zugegeben werden kann.

Selbstverständlich können als Quellen der elementaren Konstituenten auch Ausgangsverbindungen eingesetzt werden, die ihrerseits durch thermische Behandlung von Vorläuferverbindungen (Elementquellen) erzeugt wurden, und multimetalloxidischer Natur sind. Insbesondere können die Ausgangsverbindungen der elementaren Konstituenten multimetallischer Natur sein.

Alles bisher in dieser Schrift Gesagte hat vor allem dann Gültigkeit, wenn das katalytisch wirksame (aktive) Multimetalloxid des ringähnlichen Multimetalloxid-Vollkatalysators eine Stöchiometrie der allgemeinen Formel XII,

$$Mo_{12}Bi_aFe_bX^1_cX^2_dX^3_eX^4_fO_n \qquad (XII),$$

mit

$X^1 =$ Nickel und/oder Kobalt,

$X^2 =$ Thallium, Samarium, ein Alkalimetall und/oder ein Erdalkalimetall,

$X^3 =$ Zink, Phosphor, Arsen, Bor, Antimon, Zinn, Cer, Blei, Vanadium, Chrom, Niob und/oder Wolfram,

$X^4 =$ Silicium, Aluminium, Titan und/oder Zirkonium,

$a =$ 0,2 bis 5,

$b =$ 0,01 bis 5,

$c =$ 0 bis 10,

$d =$ 0 bis 2,

$e =$ 0 bis 8,

$f =$ 0 bis 10, und

$n =$ eine Zahl, die durch die Wertigkeit und Häufigkeit der von Sauerstoff verschiedenen Elemente in XII bestimmt wird,

oder eine Stöchiometrie der allgemeinen Formel XIII,

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (XIII),$$

mit

$Y^1 =$ nur Wismut oder Wismut und wenigstens eines der Elemente Tellur, Antimon, Zinn und Kupfer,

$Y^2 =$ Molybdän oder Wolfram, oder Molybdän und Wolfram,

$Y^3 =$ ein Alkalimetall, Thallium und/oder Samarium,

$Y^4 =$ ein Erdalkalimetall, Nickel, Kobalt, Kupfer, Mangan, Zink, Zinn, Cadmium und/oder Quecksilber,

$Y^5 =$ Eisen oder Eisen und wenigstens eines der Elemente Vanadium, Chrom und Cer,

$Y^6 =$ Phosphor, Arsen, Bor und/oder Antimon,

$Y^7 =$ ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

$Y^8 =$ Molybdän oder Wolfram, oder Molybdän und Wolfram,

$a' =$ 0,01 bis 8,

$b' =$ 0,1 bis 30,

$c' =$ 0 bis 4,

$d' =$ 0 bis 20,

$e' >$ 0 bis 20,

$f' =$ 0 bis 6,

$g' =$ 0 bis 15,

$h' =$ 8 bis 16,

$x', y' =$ Zahlen, die durch die Wertigkeit und Häufigkeit der von Sauerstoff verschiedenen Elemente in XIII bestimmt werden, und

$p, q =$ Zahlen, deren Verhältnis $p/q$ 0,1 bis 10 beträgt,

aufweist.

Solche ringähnlichen Multimetalloxid-Vollkatalysatoren eignen sich vor allem als Katalysatoren mit erhöhter Selektivität und Aktivität für die gasphasenkatalytische Partialoxidation von Propen zu Acrolein sowie von iso-Buten bzw. tert.-Butanol bzw. dessen Methylether zu Methacrolein.

Zur erfindungsgemäßen Herstellung der zugehörigen ringähnlichen Multimetalloxid-Vollkatalysatorvorläuferformkörper wird man aus Quellen der elementaren Konstituenten des aktiven Multimetalloxids ein feinteiliges, durch Verdichten formbares Vorläufergemisch erzeugen (ein pulverförmiges Haufwerk) und dieses Gemisch, nach Zugabe von Formgebungshilfsmittel (vgl. dazu z.B. DE-A 10 2005 037 678, DE-A 10 2007 003 778, DE-A 10 2007 028 332 und den in diesen Schriften zitierten Stand der Technik), das gegebenenfalls auch Verstärkungsmittel umfasst, in erfindungsgemäßer Weise verdichten (vorzugsweise zu ringähnlichen Vollkatalysatorvorläuferformkörpern F bzw. $F^{LII}$).

Die erfindungsgemäße Formgebung erfolgt dabei vorteilhaft so, dass die Seitendruckfestigkeit des resultierenden ringähnlichen Multimetalloxid-Vollkatalysatorvorläuferformkörpers $\geq$ 10 und $\leq$ 40 N, besser $\geq$ 10 und $\leq$ 35 N, noch besser $\geq$ 12 N und $\leq$ 30 N beträgt. Vorzugsweise beträgt die Seitendruckfestigkeit der ringähnlichen Multimetalloxid-Vollkatalysatorvorläuferformkörper ≥ 13 N und ≤ 27 N, bzw. ≥ 14 N und ≤ 25 N. Ganz besonders bevorzugt beträgt die Seitendruckfestigkeit der ringähnlichen Multimetalloxid-Vollkatalysatorvorläuferformkörper ≥ 15 N und ≤ 22 N.

Die Körnung (der Partikeldurchmesser) des erfindungsgemäß zu verdichtenden pulverförmigen Haufwerks (ausgenommen die zuzusetzenden Hilfsmittel) wird dabei vorteilhaft auf 200 bis 1500 μm, besonders vorteilhaft auf 400 bis 1000 μm eingestellt (z. B. durch Zwischenkompaktierung). In günstiger Weise liegen wenigstens 80 Gew.-%, besser wenigstens 90 Gew.-% und besonders vorteilhaft 95 oder 98 oder mehr Gew.-% des pulverförmigen Haufwerks in diesem Körnungsbereich.

Als Seitendruckfestigkeit wird dabei in dieser Schrift die Druckfestigkeit bei Stauchung des ringähnlichen Multimetalloxid-Vollkatalysatorvorläuferformkörpers senkrecht zur Symmetrieachse (d.h., parallel zur Fläche der Öffnung) verstanden. Dabei beziehen sich alle Seitendruckfestigkeiten dieser Schrift auf eine Bestimmung mittels einer Material-Prüfmaschine der Firma Zwick GmbH & Co (D-89079 Ulm) des Typs Z 2.5/TS15. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wird dabei entsprechend der DIN EN ISO 7500-1 kalibriert und ist für den Messbereich 1-500 N einsetzbar (relative Messunsicherheit: ± 0,2 %).

Die Messungen werden mit folgenden Parametern durchgeführt:

Vorkraft: 0,5 N.
Vorkraft-Geschwindigkeit: 10 mm/min.
Prüfgeschwindigkeit: 1,6 mm/min.

Dabei wird der obere Stempel zunächst langsam bis kurz vor die Mantelfläche des ringähnlichen Vollkatalysatorvorläuferformkörpers abgesenkt. Dann wird der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Vollkatalysatorvorläuferformkörper Rissbildung zeigt, ist die Seitendruckfestigkeit (SDF).

Betreffend die Aktivmassen der Stöchiometrie XII betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Wolfram, Zink und/oder Phosphor und $X^4$ ist bevorzugt Si. Besonders bevorzugt weisen die Variablen $X^1$ bis $X^4$ gleichzeitig die vorgenannten Bedeutungen auf.

Besonders bevorzugt weisen alle stöchiometrischen Koeffizienten a bis f und alle Variablen $X^1$ bis $X^4$ gleichzeitig ihre vorgenannten vorteilhaften Bedeutungen auf.

Innerhalb der Stöchiometrien der allgemeinen Formel XIII sind jene bevorzugt, die der allgemeinen Formel XIV

$$[Bi_{a''}Z^2_{b''}O_{x''}]_{p''}[Z^8_{12}Z^3_{c''}Z^4_{d''}Fe_{e''}Z^5_{f''}Z^6_{g''}Z^7_{h''}O_{y''}]_{q''} \qquad (XIV),$$

mit

$Z^2 =$ Molybdän oder Wolfram, oder Molybdän und Wolfram,
$Z^3 =$ Nickel und/oder Kobalt,
$Z^4 =$ Thallium, ein Alkalimetall und/oder ein Erdalkalimetall, vorzugsweise K, Cs und/oder Sr,
$Z^5 =$ Phosphor, Arsen, Bor, Antimon, Zinn, Cer, Vanadium, Chrom und/oder Bi,
$Z^6 =$ Silicium, Aluminium, Titan und/oder Zirkonium, vorzugsweise Si,
$Z^7 =$ Kupfer, Silber und/oder Gold,
$Z^8 =$ Molybdän oder Wolfram, oder Wolfram und Molybdän,
$a'' =$ 0,1 bis 1,
$b'' =$ 0,2 bis 2,
$c'' =$ 3 bis 10,
$d'' =$ 0,02 bis 2,
$e'' =$ 0,01 bis 5, vorzugsweise 0,1 bis 3,
$f'' =$ 0 bis 5,
$g'' =$ 0 bis 10, vorzugsweise > 0 bis 10, besonders bevorzugt 0,2 bis 10 und ganz besonders bevorzugt 0,4 bis 3,
$h'' =$ 0 bis 1,
$x'', y'' =$ Zahlen, die durch die Wertigkeit und Häufigkeit der von Sauerstoff verschiedenen Elemente in XIV bestimmt werden, und

p", q" = Zahlen, deren Verhältnis p" / q" 0,1 bis 5, vorzugsweise 0,5 bis 2 beträgt,

entsprechen.

Ferner sind katalytisch aktive Multimetalloxide der Stöchiometrie XIII bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$ enthalten, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte katalytisch aktive Multimetalloxide der Stöchiometrie XIII sind solche, in denen $Y^1$ nur Wismut ist.

Innerhalb der katalytisch aktiven Multimetalloxide der Stöchiometrie XIV sind diejenigen erfindungsgemäß bevorzugt, in denen $Z^2_{b''}$ = (Wolfram)$_{b''}$ und $Z^8_{12}$ = (Molybdän)$_{12}$ ist.

Ferner sind katalytisch aktive Multimetalloxide der Stöchiometrie XIV bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Bi_{a''}Z^2_{b''}O_{x''}$ enthalten, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Ferner ist es von Vorteil, wenn wenigstens 25 mol-% (bevorzugt wenigstens 50 mol-% und besonders bevorzugt 100 mol-%) des gesamten Anteils $[Y^1_aY^2_{b'}O_{x'}]_p$
($[Bi_aZ^2_{b''}O_{x''}]_p$) der wie beschrieben erhältlichen katalytisch aktiven Multimetalloxide der Stöchiometrie XIII (der Stöchiometrie XIV) in den katalytisch aktiven Multimetalloxiden der Stöchiometrie XIII (der Stöchiometrie XIV) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ ($[Bi_{a''}Z^2_{b''}O_{x''}]$) vorliegen, deren Größtdurchmesser im Bereich 1 nm bis 100 $\mu$m liegt.

Im Fall eines katalytisch aktiven Multimetalloxids einer der Stöchiometrien XII bis XIV kommen für das erfindungsgemäße Verfahren der Herstellung ringähnlicher Vorläuferformkörper als Gleitmittel neben Graphit auch Ruß, Polyethylenglykol, Stearinsäure, Stärke, Polyacrylsäure, Mineral- oder Pflanzenöl, Wasser, Bortrifluorid und/oder Bornitrid in Betracht. Auch Glycerin und Celluloseether können als weitere Gleitmittel eingesetzt werden. Erfindungsgemäß bevorzugt wird Graphit als alleiniges Formgebungshilfsmittel zugesetzt. Bezogen auf die erfindungsgemäß zum ringähnlichen Vollkatalysatorvorläuferformkörper zu formende Masse werden insgesamt in der Regel $\leq$ 15 Gew.-%, meist $\leq$ 9 Gew.-%, vielfach $\leq$ 5 Gew.-%, oft $\leq$ 4 Gew.-% an Graphit zugesetzt. Üblicherweise beträgt die vorgenannte Zusatzmenge $\geq$ 0,5 Gew.-%, meist $\geq$ 2,5 Gew.-%. Bevorzugt zugesetzte Graphite sind Asbury 3160 und Asbury 4012 der Firma Asbury Graphite Mills, Inc. New Jersey 08802, USA und Timrex® T44 der Firma Timcal Ltd., 6743 Bodio, Schweiz.

Bei Bedarf können noch feinteilige Verstärkungsmittel wie Mikrofasern aus Glas, Asbest, Siliciumcarbid oder Kaliumtitanat zugesetzt werden.

Die thermische Behandlung von wie eben beschrieben erfindungsgemäß erhältlichen ringähnlichen Multimetalloxid-Vollkatalysatorvorläuferformkörpern erfolgt in der Regel bei Temperaturen, die 350 °C überschreiten. Normalerweise wird im Rahmen der thermischen Behandlung die Temperatur von 650 °C jedoch nicht überschritten. Erfindungsgemäß vorteilhaft wird im Rahmen der thermischen Behandlung die Temperatur von 600 °C, bevorzugt die Temperatur von 550 °C und besonders bevorzugt die Temperatur von 510 °C nicht überschritten. Ferner wird im Rahmen der thermischen Behandlung des ringähnlichen Vollkatalysatorvorläuferformkörpers vorzugsweise die Temperatur von 380 °C, mit Vorteil die Temperatur von 400 °C, mit besonderem Vorteil die Temperatur von 420 °C und ganz besonders bevorzugt die Temperatur von 440 °C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350 °C, vorzugsweise 220 bis 290 °C, und daran anschließend eine thermische Behandlung bei einer Temperatur von 400 bis 600 °C, vorzugsweise 430 bis 550 °C durchgeführt werden.

Normalerweise nimmt die thermische Behandlung des ringähnlichen Multimetalloxid-(XII bis XIV)-Vollkatalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des ringähnlichen Vollkatalysatorvorläuferformkörpers Behandlungsdauern von 45 h bzw. 25 h nicht überschritten. Oft liegt die Gesamtbehandlungsdauer unterhalb von 20 h. Erfindungsgemäß vorteilhaft werden im Rahmen der thermischen Behandlung des relevanten ringähnlichen Vollkatalysatorvorläuferformkörpers 500°C (460°C) nicht überschritten und die Behandlungsdauer im Temperaturfenster von $\geq$ 400°C ($\geq$ 440°C) erstreckt sich auf 5 bis 20 h.

Die thermische Behandlung (auch die nachfolgend angesprochene Zersetzungsphase) der vorstehend ausgeführten ringähnlichen Multimetalloxid (XII bis XIV)-Vollkatalysatorvorläuferformkörper kann sowohl unter Inertgas als auch unter einer oxidativen Atmosphäre wie z. B. Luft (Gemisch aus Inertgas und Sauerstoff) sowie auch unter reduzierender Atmosphäre (z.B. Gemisch aus Inertgas, $NH_3$, CO und/oder $H_2$ oder Methan, Acrolein, Methacrolein) erfolgen.

Selbstredend kann die thermische Behandlung auch unter Vakuum ausgeführt werden.

Prinzipiell kann die thermische Behandlung von ringähnlichen Multimetalloxid-Vollkatalysatorvorläuferformkörpern in den unterschiedlichsten Ofentypen wie z. B. beheizbare Umluftkammern, Hordenöfen, Drehrohröfen, Bandcalcinierern oder Schachtöfen durchgeführt werden. Bevorzugt erfolgt die thermische Behandlung der ringähnlichen Multimetalloxid-Vollkatalysatorvorläuferformkörper in einer Bandcalciniervorrichtung, wie sie die DE-A 100 46 957 und die WO 02/24620 empfehlen.

Die thermische Behandlung von ringähnlichen Multimetalloxid- Vollkatalysatorvorläuferformkörpern unterhalb von 350 °C verfolgt in der Regel das Ziel der thermischen Zersetzung der in den Vollkatalysatorvorläuferformkörpern enthaltenen Quellen der elementaren Konstituenten des angestrebten ringähnlichen Multimetalloxid-Vollkatalysators. Häufig erfolgt diese Zersetzungsphase im Rahmen des Aufheizens auf Temperaturen $\geq$ 350 °C.

Insbesondere zur Herstellung von katalytisch aktiven Multimetalloxiden der Stöchiometrie der allgemeinen Formel XIII oder XIV ist es vorteilhaft, ein Mischoxid $Y^1{}_{a'}Y^2{}_{b'}O_{x'}$ bzw. $Bi_{a''}Z^2{}_{b''}O_{x''}$ als Quelle der Elemente $Y^1$, $Y^2$ bzw. Bi, $Z^2$ in Abwesenheit der übrigen Konstituenten des Multimetalloxids vorzubilden und damit nach seiner Vorbildung, wie bereits beschrieben, mit Quellen der übrigen Konstituenten des Multimetalloxids ein feinteiliges formbares Gemisch zu erzeugen, um daraus, nach Zusatz von Formgebungshilfsmittel, den ringähnlichen Multimetalloxid-Vollkatalysatorvorläuferformkörper erfindungsgemäß zu formen.

Bei einer solchen Vorgehensweise ist lediglich darauf zu achten, dass für den Fall, dass die Herstellung des feinteiligen formbaren Gemischs nass erfolgt (in Suspension), die vorgebildeten Mischoxide $Y^1{}_{a'}Y^2{}_{b'}O_{x'}$ bzw. $Bi_{a''}Z^2{}_{b''}O_{x''}$ nicht in nennenswertem Umfang in Lösung gehen.

Ausführlich wird eine wie vorstehend beschriebene Herstellweise in den Schriften DE-A 44 07 020, EP-A 835, EP-A 575 897 und DE-C 33 38 380 sowie in der Deutschen Anmeldung mit dem Aktenzeichen 102007003778.5 beschrieben.

Beispielsweise kann man wasserlösliche Salze von $Y^1$ wie Nitrate, Carbonate, Hydroxide oder Acetate mit $Y^2$-Säuren oder deren Ammoniumsalzen in Wasser mischen, die Mischung trocknen (vorzugsweise sprühtrocknen) und die getrocknete Masse anschließend thermisch behandeln. Die thermisch behandelte Masse wird nachfolgend zweckmäßig zerkleinert (z. B. in einer Kugelmühle oder durch Strahlmahlen) und aus dem dabei erhältlichen, in der Regel aus im wesentlichen kugelförmigen Partikeln bestehenden, Pulver die Kornklasse mit einem im für das aktive Multimetalloxid der Stöchiometrie der allgemeinen Formel XIII bzw. XIV gewünschten Größtdurchmesserbereich liegenden Korngrößtdurchmesser durch in an sich bekannter Weise durchzuführendes Klassieren (z. B. Nass- oder Trockensiebung) abgetrennt und vorzugsweise mit, bezogen auf die Masse dieser abgetrennten Kornklasse, 0,1 bis 3 Gew.-%, feinteiligem $SiO_2$ (der Partikeldurchmesser $d_{50}$ der üblicherweise im wesentlichen kugelförmigen $SiO_2$-Partikel beträgt zweckmäßigerweise 100 nm bis 15 $\mu$m) vermischt und so eine Ausgangsmasse 1 hergestellt. Die thermische Behandlung erfolgt zweckmäßig bei Temperaturen von 400 bis 900 °C, vorzugsweise bei 600 bis 900 °C. Letzteres gilt insbesondere dann, wenn es sich bei dem vorgebildeten Mischoxid um ein solches der Stöchiometrie $BiZ^2{}_2O_6$, $Bi_2Z^2{}_2O_9$ und/oder $Bi_2Z^2{}_3O_{12}$ handelt, unter denen das $Bi_2Z^2{}_2O_9$ bevorzugt ist, insbesondere wenn $Z^2$ = Wolfram.

Üblicherweise erfolgt die thermische Behandlung im Luftstrom (z. B. in einem Drehrohrofen, wie er in der DE-A 103 25 487 beschrieben ist). Die Dauer der thermischen Behandlung erstreckt sich in der Regel auf einige Stunden.

Von den übrigen Bestandteilen des gewünschten aktiven Multimetalloxids der allgemeinen Formel XIII bzw. XIV wird normalerweise ausgehend von in an sich bekannter Weise geeigneten Quellen (vgl. EP-A 835 und DE-C 33 38 380 sowie DE-A 44 07 020 und die Deutsche Anmeldung mit dem Aktenzeichen 102007003778.5) in erfindungsgemäß zweckmäßiger Weise z. B. ein möglichst inniges, vorzugsweise feinteiliges Trockengemisch hergestellt (z. B. wasserlösliche Salze wie Halogenide, Nitrate, Acetate, Carbonate oder Hydroxide in einer wässrigen Lösung vereinen und anschließend die wässrige Lösung z. B. sprühtrocknen oder nicht wasserlösliche Salze, z. B. Oxide, in wässrigem Medium suspendieren und anschließend die Suspension z. B. sprühtrocknen), das hier als Ausgangsmasse 2 bezeichnet wird. Wesentlich ist nur, dass es sich bei den Bestandteilen der Ausgangsmasse 2 entweder bereits um Oxide handelt, oder um solche Verbindungen, die durch Erhitzen, gegebenenfalls in Anwesenheit von Sauerstoff und/oder Sauerstoffquellen, in Oxide überführbar sind. Anschließend werden die Ausgangsmasse 1 und die Ausgangsmasse 2 im gewünschten Mengenverhältnis sowie unter Zusatz von Formgebungshilfsmittel zum zum ringähnlichen Vollkatalysatorvorläuferformkörper formbaren Gemisch vermischt. Die Formung kann, wie bereits beschrieben, anwendungstechnisch zweckmäßig über die Stufe einer Zwischenkompaktierung erfolgen.

In einer weniger bevorzugten Ausführungsform kann das Vorgebildete Mischoxid $Y^1{}_{a'}Y^2{}_{b'}O_{x'}$ bzw. $Bi_{a''}Z^2{}_{b''}O_{x''}$ mit Quellen der übrigen Bestandteile der gewünschten Aktivmasse auch in flüssigem, vorzugsweise wässrigem, Medium innig vermischt werden. Dieses Gemisch wird anschließend z. B. zu einem innigen Trockengemisch getrocknet und sodann, wie bereits beschrieben, geformt und thermisch behandelt. Dabei können die Quellen der übrigen Konstituenten in diesem flüssigen Medium gelöst und/oder suspendiert vorliegen, wohingegen das Vorgebildete Mischoxid in diesem flüssigen Medium im wesentlichen unlöslich sein sollte, d. h., suspendiert vorliegen muss.

Die vorgebildeten Mischoxidpartikel sind im fertig gestellten ringförmigen Vollkatalysator in der durch die Klassierung eingestellten Längsausdehnung im wesentlichen unverändert enthalten. Im Übrigen kann wie in der Deutschen Anmeldung mit dem Aktenzeichen 102007003778.5 ausgeführt verfahren werden. Die in der Deutschen Anmeldung mit dem Aktenzeichen 102007003778.5 bezüglich ringförmiger Multimetalloxid-Vollkatalysatorvorläuferformkörper sowie den aus diesen resultierenden ringförmigen Multimetalloxidvollkatalysatoren gemachten Ausführungen gelten für die Gegenstände dieser Anmeldung in entsprechender Weise.

In typischer Weise betragen die Seitendruckfestigkeiten von wie beschrieben erhältlichen ringähnlichen Multimetalloxid (XII bis XIV)- Vollkatalysatoren 5 bis 13 N, häufig 8 bis 11 N.

Wie bereits erwähnt, eignen sich die wie beschrieben erhältlichen ringähnlichen Vollkatalysatoren insbesondere als Katalysatoren für die Partialoxidation von

Propen zu Acrolein bzw. von iso-Buten und/oder teert. Butanol zu Methacrolein.

Die Partialoxidation kann dabei z. B. wie in den Schriften WO 00/53557, WO 00/53558,
DE-A 199 10 506, EP-A 1 106 598, WO 01/36364, DE-A 199 27 624,
DE-A 199 48 248, DE-A 199 48 523, DE-A 199 48 241, EP-A 700 714,
DE-A 10313213, DE-A 103 13 209, DE-A 102 32 748, DE-A 103 13 208,
WO 03/039744. EP-A 279 374, DE-A 33 38 380, DE-A 33 00 044, EP-A 575 897, DE-A 10 2004 003 212, DE-A 10 2005 013 039, DE-A 10 2005 009 891, die Deutsche Anmeldung mit dem Aktenzeichen 102007003778.5, DE-A 10 2005 010 111, DE-A 10 2005 009 885 sowie DE-A 44 07 020 für ringförmige Vollkatalysatoren beschrieben durchgeführt werden, wobei die Katalysatorbeschickung z. B. nur wie beschrieben erhältliche ringähnliche Vollkatalysatoren oder z. B. mit inerten Formkörpern verdünnte ringähnliche Vollkatalysatoren umfassen kann. Im letzteren Fall wird die Katalysatorbeschickung vorteilhaft in der Regel so gestaltet, dass ihre volumenspezifische Aktivität in Strömungsrichtung des Reaktionsgasgemisches kontinuierlich, sprunghaft und/oder stufenförmig zunimmt.

Für das Verfahren der Propylenpartialoxidation zu Acrolein besonders vorteilhafte Multimetalloxidstöchiomet- rien sind:

a) $[Bi_2W_2O_9 \times 2WO_3]_{0,4}[Mo_{12}Co_{5,5}Fe_{2,94}Si_{1,59}K_{0,08}O_x]_1$;
b) $Mo_{12}Ni_{6,5}Zn_2Fe_2Bi_1P_{0,0065}K_{0,06}O_x \cdot 10SiO_2$;
c) $Mo_{12}CO_7Fe_{2,94}Bi_{0,6}Si_{1,59}K_{0,08}O_x$;
d) wie Multimetalloxid II-Vollkatalysator gemäß Beispiel 1 der DE-A 197 46 210; und
e) wie Beispiel 1c aus der EP-A 015 565.

Das in dieser Schrift Gesagte hat aber auch dann Gültigkeit, wenn das katalytisch wirksame (aktive) Multimetalloxid des ringähnlichen Multimetalloxid-Vollkatalysators eine Stöchiometrie der allgemeinen Formel XV,

$$Mo_{12}P_aV_bX^1_cX^2_dX^3_eSb_fRe_gS_hO_n \qquad (XV),$$

aufweist, in der Variablen folgende Bedeutung haben:

$X^1$ = Kalium, Rubidium und/oder Cäsium,
$X^2$ = Kupfer und/oder Silber,
$X^3$ = Cer, Bor, Zirkonium, Mangan und/oder Wismut,
a = 0,5 bis 3,
b = 0,01 bis 3,
c = 0,2 bis 3,
d = 0,01 bis 2,
e = 0 bis 2,
f = 0 bis 2, vorzugsweise 0,01 bis 2,
g = 0 bis 1,
h = 0 bis 0,5, vorzugsweise 0,001 bis 0,5, und
n = eine Zahl, die durch die Wertigkeit und Häufigkeit der von Sauerstoff verschiedenen Elemente in XV bestimmt wird.

Bevorzugt sind Multimetalloxide XV, in denen h 0,03 bis 0,5 ist.

Besonders bevorzugte Stöchiometrien der allgemeinen Formel XV sind jene der Ausführungsbeispiele B1 bis B15 aus der EP-A 467 144 und dies auch dann, wenn diese beispielhaften Multimetalloxide kein K und/oder kein Re enthalten.

Vorgenannte EP-A 467 144 und die Deutsche Anmeldung mit dem Aktenzeichen 102007003778.5 beschreiben auch die Herstellung von ringförmigen Multimetalloxid (XV)-Vollkatalysatorformkörpern und deren Verwendung als Katalysatoren für die heterogen katalysierte Gasphasenpartialoxidation von Methacrolein zu Methacrylsäure. Diese Beschreibungen sind auch im in der vorliegenden Anmeldung gegebenen Kontext zutreffend.

D.h., ringähnliche Multimetalloxid (XV)-Vollkatalysatorvorläuferformkörper können erfindungsgemäß dadurch hergestellt werden, dass man als Ausgangsverbindungen geeignete Salze der sie konstituierenden elementaren Bestandteile, gegebenenfalls bei erhöhter Temperatur und unter Zusatz von Säuren oder Basen, in wässrigem Medium durch Lösen und/oder Suspendieren fein verteilt und, zur Vermeidung unerwünschter Oxidationsprozesse ggf. unter Inertgas, mischt, das Gemisch trocknet (z. B. eindampft oder sprühtrocknet), der resultierenden, feinteilige Form aufweisenden oder in feinteilige Form überführten Trockenmasse z. B. Graphit als Gleitmittel sowie gegebenenfalls sonstige der bereits genannten Formgebungshilfsmittel zusetzt, und die dabei erhaltene feinteilige Masse erfindungsgemäß zur gewünschten ringähnlichen Geometrie formt (verdichtet). Die dabei resultierenden Katalysatorvorläuferformkörper werden anschließend zur Überführung in die aktiven ringähnlichen Katalysatorformkörper thermisch behandelt. Bevorzugt wird die thermische Behandlung bei Temperaturen von 180 bis 480°C, besonders bevorzugt bei Temperaturen von 250 bis 450 °C durchgeführt. Die thermische Behandlung kann dabei unter den bereits beschriebenen Gasatmosphären erfolgen. Beispielhaft erwähnt seien nochmals strömende Luft, strömende Inertgasatmosphäre (z. B. $N_2$, oder $CO_2$, oder Edelgase) oder Vakuum. Die thermische Behandlung kann in mehreren Temperaturstufen und/oder in verschiedenen Atmosphären durchgeführt werden. So kann z. B. in einer ersten Stufe bei 200 bis 260 °C in Luft, in einer zweiten Stufe bei 420 bis 460 °C in Stickstoff und in einer dritten Stufe bei 350 bis 410 °C wiederum in Luft thermisch behandelt werden. Im Regelfall ist strömende Luft die bevorzugte Atmosphäre für die thermische Behandlung.

Im Übrigen gilt das in dieser Schrift bei der Herstellung von ringähnlichen Vollkatalysatorformkörpern von Multimetalloxiden XII bis XIV Gesagte hier in entsprechender Weise, jedoch mit dem Unterschied, dass hier die erhöhten Seitendruckfestigkeiten für die ringförmigen Vollkatalysatorvorläuferformkörper bevorzugt werden.

Ebenso gelten die in der Deutschen Anmeldung mit dem Aktenzeichen 102007003778.5 bezüglich der Herstellung von ringförmigen Vollkatalysatorformkörpern von Multimetalloxiden XV gemachten Aussagen hier in entsprechender Weise.

D. h., z. B. bevorzugtes Trocknungsverfahren für die wässrige Lösung oder Suspension der Quellen der elementaren Konstituenten des gewünschten aktiven Multimetalloxids XV ist die Sprühtrocknung. Das resultierende Sprühpulver mit einem Partikeldurchmesser $d_{50}$ zwischen 10 bis 50 $\mu$m wird vorteilhaft nach Zusatz von feinteiligem Graphit als Hilfsmittel zwischenkompaktiert, um das Pulver zu vergröbern. Bevorzugt erfolgt die Zwischenkompaktierung hier auf Partikeldurchmesser von 100 bis 2000 $\mu$m, bevorzugt von 150 bis 1500 $\mu$m und besonders bevorzugt von 400 bis 1000 $\mu$m. Anschließend erfolgt auf der Grundlage des vergröberten Pulvers die erfindungsgemäße Formgebung, wobei bei Bedarf zuvor nochmals feinteiliger Graphit (sowie gegebenenfalls weitere Formgebungshilfsmittel) zugegeben werden kann.

Bei der beschriebenen Herstellungsweise von ringähnlichen Vollkatalysatorformkörpern von aktiven Multimetalloxiden der allgemeinen Formel XV wird Antimon üblicherweise als Antimontrioxid, Rhenium z. B. als Rhenium(VII)oxid, Molybdän vorzugsweise als Ammoniumsalz der Molybdän- oder Phosphormolybdänsäure, Bor z. B. als Borsäure, Vanadin in der Regel als Ammoniumvanadat oder Vanadinoxalat, Phosphor mit Vorteil als ortho-Phosphorsäure oder Di-AmmoniumPhosphat, Schwefel z.B. als Ammoniumsulfat und die kationischen Metalle normalerweise als Nitrate, Oxide, Hydroxide, Carbonate, Chloride, Formiate, Oxalate und/oder Acetate bzw. deren Hydrate eingesetzt.

Das erfindungsgemäße Verfahren eignet sich ferner zur Herstellung von ringähnlichen Multimetalloxid-Vollkatalysatorvorläuferformkörpern mit gekrümmter und/oder nicht gekrümmter Stirnfläche von ringähnlichen Multimetalloxidvollkatalysatoren, deren aktives Multimetalloxid ein Vanadium, Phosphor und Sauerstoff enthaltendes Multimetalloxid ist, und die sich als Katalysatoren für die heterogen katalysierte Gasphasenoxidation wenigstens eines Kohlenwasserstoffs mit mindestens vier Kohlenstoffatomen (insbesondere n-Butan, n-Butene und/oder Benzol) zu Maleinsäureanhydrid eignen. Die Stöchiometrie des aktiven Multimetalloxids kann dabei z. B. eine solche der allgemeinen Formel XVI sein,

$$V_1P_bFe_cX^1_dX^2_eO_n \qquad (XVI),$$

in der die Variablen folgende Bedeutung aufweisen:

$X^1$ = Mo, Bi, Co, Ni, Si, Zn, Hf, Zr, Ti, Cr, Mn, Cu, B, Sn und/oder Nb,
$X^2$ = K, Na, Rb, Cs und/oder Tl,
b = 0,9 bis 1,5,
c = 0 bis 0,1,
d = 0 bis 0,1,
e = 0 bis 0,1, und
n = eine Zahl, die durch die Wertigkeit und Häufigkeit der von Sauerstoff verschiedenen Elemente in XVI bestimmt wird.

Zur Herstellung von diesbezüglich geeigneten und erfindungsgemäß zu ringähnlichen Vorläuferformkörpern (insbesondere F bzw. $F^{LII}$) zu verdichtenden pulverförmigen Haufwerken sei an dieser Stelle auf die WO 03/078310 und Deutschen Anmeldung mit dem Aktenzeichen 102007003778.5, die WO 01/68245 und die DE-A 10 2005 035 978 verwiesen, die die Herstellung entsprechender ringförmiger Systeme betreffen.

Beispielsweise kann wie folgt vorgegangen werden:

a) Umsetzung einer fünfwertigen Vanadiumverbindung (z. B. $V_2O_5$) mit einem organischen, reduzierenden Lösungsmittel (z. B. iso-Butanol) in Gegenwart einer fünfwertigen Phosphorverbindung (z. B. Ortho- und/oder Pyrophosphorsäure) unter Erwärmen auf 75 bis 205 °C, bevorzugt auf 100 bis 120 °C;

b) Abkühlen des Reaktionsgemisches auf vorteilhaft 40 bis 90 °C;

c) Zugabe von Eisen(III)phosphat;

d) Erneutes Erwärmen auf 75 bis 205°C, bevorzugt 100 bis 120 °C;

e) Isolierung der gebildeten festen Vanadium-, Phosphor-, Eisen- und Sauerstoff enthaltenden Vorläufermasse (z. B. durch Filtrieren);

f) Trocknung und/oder thermische Vorbehandlung der Vorläufermasse (gegebenenfalls bis zu beginnender Vorformierung durch Wasserabspaltung aus der Vorläufermasse);

g) Zugabe von feinteiligem Graphit und anschließend erfindungsgemäße Formgebung zum ringähnlichen Multimetalloxid-Vollkatalysatorvorläuferformkörper;

daran anschließend thermische Behandlung der gebildeten Katalysatorvorläuferformkörper durch Erhitzen in einer Atmosphäre, welche Sauerstoff, Stickstoff, Edelgase, Kohlendioxid, Kohlenmonoxid und/oder Wasserdampf enthält (z. B. wie in der WO 03078310 auf Seite 20, Zeile 16 bis Seite 21, Zeile 35 beschrieben).

Das erfindungsgemäße Verfahren umfasst weiterhin Verfahren zur Herstellung von ringähnlichen Multimetalloxid-Vollkatalysatorvorläuferformkörpern mit gekrümmter und/oder nicht gekrümmter Stirnfläche von ringähnlichen Multimetalloxid-Vollkatalysatoren, deren aktives Multimetalloxid ein Mo, V und wenigstens eines der Elemente Te und Sb enthaltendes Multimetalloxid ist, wie es z.B. die Schriften EP-A 962 253, DE-A 101 22 027, EP-A 608 838, DE-A 198 35 247, EP-A 895 809, EP-A 1 254 709, EP-A 1 192 987, EP-A 1 262 235, EP-A 1 193 240, JP-A 11-343261, JP-A 11-343262, EP-A 1 090 684, EP-A 1 301 457, EP-A 1 254 707, EP-A 1 335 793, DE-A 100 46 672, DE-A 100 34 825, EP-A 1 556 337, DE-A 100 33 121, WO 01/98246 und EP-A 1 558 569 beschreiben.

Häufig enthalten die vorgenannten Mo, V und wenigstens eines der Elemente Te und Sb enthaltenden Multimetalloxide noch das Element Nb. Die vorgenannten resultierenden ringähnlichen Multimetalloxidvollkatalysatoren eignen sich für alle in den vorgenannten Schriften aufgeführten heterogen katalysierten Gasphasenreaktionen (insbesondere partiellen Oxidationen). Dies sind im besonderen die heterogen katalysierte partielle Gasphasenoxidation von Propan zu Acrylsäure sowie von Acrolein zu Acrylsäure, von Methacrolein zu Methacrylsäure und von iso-Butan zu Methacrylsäure.

Das erfindungsgemäße Verfahren eignet sich aber auch, wie in dieser Schrift bereits mehrfach angesprochen, zur Herstellung von ringähnlichen Vorläuferformkörpern (z. B. von ringähnlichen Vorläuferformkörpern F oder von ringähnlichen Vorläuferformkörpern $F^{LII}$), aus denen durch thermische Behandlung ringähnliche (oxidische) Trägerformkörper erhältlich sind, die z. B. zur Herstellung ringähnlicher Schalenkatalysatoren oder zur Herstellung ringähnlicher Tränkkatalysatoren verwendet werden können. Derartige ringähnliche Trägerformkörper können aber selbstredend auch als inerte Formkörper zum Verdünnen eines Katalysatorfestbetts verwendet werden.

Zur erfindungsgemäßen Herstellung solcher ringähnlicher Träger-Vorläuferformkörper wird als erfindungsgemäß zu verdichtendes pulverförmiges Haufwerk in der Regel ein solches verwendet, das aus (unter Normalbedingungen üblicherweise festen) Metalloxiden und/oder aus solchen Metallverbindungen (z. B. Salzen) besteht, die durch Erhitzen (thermisches Behandeln) in (unter Normalbedingungen üblicherweise feste) Oxide überführbar sind (wenigstens durch thermisches Behandeln in Anwesenheit von gasförmigem molekularem Sauerstoff und/oder von gasförmigen Sauerstoff freisetzenden Komponenten). Zusätzlich kann das pulverförmige Haufwerk die in dieser Schrift bereits erwähnten Formgebungshilfsmittel wie z. B. Gleitmittel, Porosierungsmittel und Verstärkungsmittel zugesetzt enthalten.

Die zur erfindungsgemäßen Herstellung von ringähnlichen Träger-Vorläuferformkörpern zu verwendenden pulverförmigen Haufwerke werden daher in der Regel ebenfalls Haufwerk HW* oder Haufwerke HW** sein. Insbesondere aber werden sie pulverförmige Haufwerke O sein. Alle bezüglich der erfindungsgemäßen Verdichtung von pulverförmigen Haufwerken O, HW* und HW** in dieser Schrift gemachten Ausführungen gelten daher in entsprechender Weise. Bei erfindungsgemäß bevorzugten ringähnlichen Träger-Vorläuferformkörpern handelt es sich in der Regel um ringähnliche Vorläuferformkörper F, vorzugsweise um ringähnliche Vorläuferformkörper $F^{LII}$.

Die thermische Behandlung der ringähnlichen Träger-Vorläuferformkörper zur Überführung derselben in den ringähnlichen Träger erfolgt in der Regel bei Temperaturen ≥ 500 °C, häufig ≥ 600 °C und vielfach ≥ 700 °C. In der Regel wird die vorgenannte thermische Behandlung jedoch bei Temperaturen ≤ 1500 °C durchgeführt. Die thermische Behandlung kann dabei sowohl unter Inertgas als auch unter einer oxidativen Atmosphäre wie z. B. Luft (Gemisch aus Inertgas und Sauerstoff) erfolgen. Selbstredend kann die thermische Behandlung auch unter Vakuum oder reduzierender Atmosphäre erfolgen.

Üblicherweise erfolgt die thermische Behandlung unter oxidierender Atmosphäre (im allgemeinen unter Luft).

Von den katalytisch aktiven Multimetalloxiden unterscheiden sich die Trägeroxide normalerweise dadurch, dass die thermische Behandlung zu ihrer Erzeugung bei wesentlich höheren Temperaturen und/oder über eine längere Zeitdauer erfolgt (wodurch sie häufig weitgehend unporös sind) und/oder dass das in ihnen numerisch (molar gerechnet) am häufigsten enthaltene, von Sauerstoff verschiedene, Element kein Übergangsmetall der 5. bis 11. Nebengruppe (das sind die Vanadiumgruppe, die Chromgruppe, die Mangangruppe, die Eisengruppe, die Kobaltgruppe und die Nickelgruppe) und nicht Phosphor ist. Vielfach ist das in ihnen numerisch (molar gerechnet) am häufigsten enthaltene, von Sauerstoff verschiedene, Metall ein Element aus der Gruppe bestehend aus Erdalkali (z.B. Mg, Ca), Zn, Zr, Al, Si und Ti.

Die bei der Herstellung der ringähnlichen Träger-Vorläuferformkörper mitverwendeten organischen (einschließlich Graphit) Formgebungshilfsmittel zersetzen sich bei der vorgenannten thermischen Behandlung in der Regel zu gasförmig entweichenden Verbindungen (und/oder setzen sich zu gasförmig entweichenden chemischen Verbindungen um). Häufig besteht der ringähnliche Trägerformkörper aus einem keramischen Werkstoff. Beispielhaft genannt seien Silikatkeramiken und sonstige Metalloxidkeramiken. In dem entsprechender Weise enthält das zur Herstellung eines ringähnlichen Träger-Vorläuferformkörpers erfindungsgemäß zu verdichtende pulverförmige Haufwerk als mineralische Ausgangsrohstoffe vielfach pulverförmige Silikate wie z. B. Zirkonsilikat, Aluminiumsilikat (z. B. Mullit), Magnesium-Silikat (z. B. Steatit) und sonstige pulverförmige Metalloxide wie z. B. Aluminiumoxid, Magnesiumoxid und Zirkonoxid.

Beispielhaft näher ausgeführt sei an dieser Stelle die Herstellung der in der WO 99/48606 in Ringform ausgeführten Trägerformkörper, die sich zur Herstellung von Trägerkatalysatoren für die Umsetzung von Ethylen und Chlorwasserstoff im Beisein von molekularem Sauerstoff zu 1,2-Dichlorethan eigenen ("Oxychlorierung").

Als feinteiliger mineralischer Rohstoff wird ein feinteiliges Gemisch von Pseudoböhmit und $\gamma$-Al$_2$C$_3$ im Gewichtsverhältnis 4:1 bis 1:4, vorzugsweise 1:1 bis 1:3 verwendet. Diesem Gemisch, dessen d$_{50}$ Partikeldurchmesser anwendungstechnisch zweckmäßig 10 bis 100 $\mu$m beträgt, werden, bezogen auf sein Gewicht, 0,5 bis 7 Gew.-% (vorzugsweise 2 bis 5 Gew.-%) Magnesiumstearat sowie 0,5 bis 3 Gew.-% (vorzugsweise 1 bis 1,5 Gew.-%) feinteiliger Graphit (dso Partikeldurchmesser 15 bis 30 $\mu$m) als Formgebungshilfsmittel zugesetzt.

Das dabei resultierende pulverförmige Haufwerk wird anschließend in erfindungsgemäßer Weise zu den ringähnlichen Träger-Vorläuferformkörpern verdichtet (der Lehre der EP-A 184790 folgend ist die obere Stirnfläche des unteren Stempels und die untere Stirnfläche des oberen Stempels dabei vorteilhaft konkav gestaltet (d. h., sie weisen vorzugsweise eine kreisförmige Rille auf)). Diese werden danach in oxidierender Atmosphäre (vorzugsweise unter Luft) bei Temperaturen von 500 bis 800 °C, vorzugsweise 700 bis 750 °C thermisch behandelt (in der Regel 0,5 bis 10 h).

Der so erhaltene ringähnliche Trägerformkörper wird anschließend mit einer wässrigen CuCl$_2$/KCl-Lösung getränkt. Nach der Tränkung werden die ringähnlichen Formkörper zu den aktiven ringähnlichen Katalysatoren getrocknet (in der Regel bei Temperaturen von 80 bis 300 °C, vorzugsweise 100 bis 200 °C). Die Trocknung erfolgt normalerweise an Luft.

Typische Trocknungsdauern betragen 0,2 bis 10 h, im Bereich erhöhter Temperatur 0,5 bis 2 h. Die Konzentration und das Volumen der Tränklösung werden bei der Tränkung anwendungstechnisch zweckmäßig so gewählt, dass die resultierenden Trägerkatalysatoren einen Cu-Gehalt von 1 bis 15 Gew.-%, vorzugsweise von 2 bis 10 Gew.-%, und einen K-Gehalt von 0,1 bis 8 Gew.-%, vorzugsweise von 0,3 bis 3 Gew.-% aufweisen. Im übrigen kann wie in der WO 99/48606 beschrieben verfahren werden.

Das in dieser Schrift Gesagte hat aber auch dann Gültigkeit, wenn das katalytisch wirksame (aktive) Multimetalloxid des ringähnlichen Multimetalloxid-Vollkatalysators die Stöchiometrie (Fe$_2$O$_3$)$_1$·(MoO$_3$)$_{5,25}$ aufweist. Als Ausgangsverbindungen zur Herstellung derselben eignen sich z. B. Eisen-(III)-nitrat und Molybdäntrioxid. Besonders bevorzugte Fe-Quelle ist Eisen-(III)-nitrat-nonahydrat-Schmelze gemäß der Lehre der PCT/EP2008/050341. Bevorzugt werden beide in wässriger ammoniakalischer Lösung miteinander vermischt. Selbige wird anschließend sprühgetrocknet und das resultierende Sprühpulver in erfindungsgemäßer Weise zu ringähnlichen Vorläuferformkörpern verdichtet. Der Endabstand E ist dabei bevorzugt 5 mm, die Länge der Umrisslinie des Kreiszylinders Z ist vorzugsweise 2·$\pi$ mm ($\pi$ ist dabei das Verhältnis von Kreisumfang zu Kreisdurchmesser) und der Durchmesser DD der Deckfläche des Kegelstumpfes KS ca. 5 mm. Abschließend werden die ringähnlichen Vorläuferformkörper im Temperaturbereich von 400 bis 500 °C unter Luft thermisch behandelt. Die resultierenden ringähnlichen Multimetalloxid-Vollkatalysatorformkörper eignen sich z. B. als Katalysatoren für die Partialoxidation von Methanol zu Formaldehyd.

Damit umfasst die vorliegende Patentanmeldung insbesondere die nachfolgenden erfindungsgemäßen Ausführungsformen:

1. Verfahren zur Herstellung eines ringähnlichen oxidischen Formkörpers, umfassend das mechanische Verdichten eines in den Füllraum einer Matrize eingebrachten pulverförmigen Haufwerks aus Bestandteilen, die wenigstens eine Metallverbindung, die durch thermische Behandlung bei einer Temperatur $\geq$ 100 °C in ein Metalloxid überführbar ist, oder wenigstens ein Metalloxid, oder wenigstens ein Metalloxid und wenigstens eine solche Metallverbindung umfassen, zu einem ringähnlichen Vorläuferformkörper, bei dem sich der Füllraum in einer mit einer vertikalen Bohrachse B von oben nach unten durch das Matrizenmaterial hindurchgeführten Matrizenbohrung befindet und durch

- die Innenwand der Matrizenbohrung,

- die obere Stirnfläche eines von unten entlang der Bohrachse B in die Matrizenbohrung hub- und senkbeweglich eingeführten unteren Stempels, auf der das in den Füllraum eingebrachte pulverförmige Haufwerk aufliegt,

- die längs der Bohrachse B in einem axialen Ausgangsabstand A oberhalb der oberen Stirnfläche des unteren Stempels befindliche untere Stirnfläche eines entlang der Bohrachse B hub- und senkbeweglich angebrachten oberen Stempels, dessen untere Stirnfläche das in den Füllraum eingebrachte pulverförmige Haufwerk von oben berührt, und

- die Mantelfläche eines aus der geometrischen Mitte der oberen Stirnfläche des unteren Stempels heraus entlang der Bohrachse B in der Matrizenbohrung von unten nach oben geführten Mittelstiftes MF, der wenigstens bis zur geometrischen Mitte der unteren Stirnfläche des oberen Stempels hinaufreicht, begrenzt wird,

indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt, wobei

- die geometrische Form der Mantelfläche des unteren Stempels derjenigen der Mantelfläche eines Kreiszylinders I entspricht;

- die geometrische Form der Mantelfläche des oberen Stempels derjenigen der Mantelfläche eines Kreiszylinders II entspricht;

- in der geometrischen Mitte der oberen Stirnfläche des unteren Stempels eine von oben nach unten durch den unteren Stempel hindurchgeführte Mittelbohrung $MB^U$ ausgebildet ist;

- im Ausgangsabstand A der beiden Stirnflächen der Mittelstift MF von unten durch die Mittelbohrung $MB^U$ hindurch bis wenigstens zur geometrischen Mitte der unteren Stirnfläche des oberen Stempels hinaufragt;

- der Mittelstift MF von unten nach oben die geometrische Form eines Kreiszylinders Z mit einer kreiszylindrischen Mantelfläche MZ aufweist;

- die Länge der Umrisslinie des Kreiszylinders Z kleiner als die Länge der Umrisslinie des Kreiszylinders I sowie kleiner als die Länge der Umrisslinie des Kreiszylinders II ist;

- die Position des Mittelstiftes MF und die Position der Matrize einschließlich der Matrizenbohrung längs der Bohrachse B während des Verfahrens relativ zueinander fixiert sind;

- in der geometrischen Mitte der unteren Stirnfläche des oberen Stempels eine in den oberen Stempel hineinführende und mit wenigstens einem Auslass aus dem oberen Stempel in Verbindung stehende Mittelbohrung $MB^O$ ausgebildet ist, die den Mittelstift MF bei der Verringerung des Ausgangsabstands A auf den Endabstand E im erforderlichen Umfang aufzunehmen vermag und in die der Mittelstift MF bereits im Ausgangsabstand A hineinragen kann;

- die Symmetrieachsen der Matrizenbohrung, des Kreiszylinders I, des Kreiszylinders II, der Mittelbohrung $MB°$, des Mittelstiftes MF und der Mittelbohrung $MB^U$ auf einer gemeinsamen, durch die Matrizenbohrung vertikal verlaufenden geraden Linie L liegen;

- die Matrizenbohrung längs ihrer Bohrachse einen Längsabschnitt I aufweist, auf dessen Länge I die geometrische Form der Innenwand der Matrizenbohrung derjenigen der Mantelfläche eines Kreiszylinders KZ entspricht, und an dessen oberem Ende sich unmittelbar ein nach oben gerichteter Längsabschnitt II der Matrizenbohrung anschließt, der die Länge II aufweist;

- die Ausmaße des Längsabschnitts I der Matrizenbohrung und des Kreiszylinders I so beschaffen sind, dass der untere Stempel während des Verfahrens stets jeweils wenigstens auf einer Teillänge des Längsabschnitts I mit seiner Mantelfläche auf der Innenwand der Matrizenbohrung gleitend in die Matrizenbohrung geführt ist;

- die Ausmaße der Mittelbohrung MB$^U$ und des Kreiszylinders Z so beschaffen sind, dass der untere Stempel während des Verfahrens stets wenigstens im Bereich des Eingangs seiner Mittelbohrung MB$^U$ in seine obere Stirnfläche mit der Innenwand der Mittelbohrung MB$^U$ auf der kreiszylindrischen Mantelfläche MZ des Mittelstiftes MF gleitend in die Matrizenbohrung geführt ist; und

- nach beendeter Verdichtung der obere Stempel vom gebildeten ringähnlichen Vorläuferformkörper abgehoben und der ringähnliche Vorläuferformkörper durch Anheben des unteren Stempels aus der Matrizenbohrung entfernt wird,

dadurch gekennzeichnet, dass ein sich daran anschließendes Verfahren der thermischen Behandlung des ringähnlichen Vorläuferformkörpers bei einer Temperatur ≥ 100 °C, bei dem sich wenigstens eine Teilmenge seiner Bestandteile unter Ausbildung wenigstens einer gasförmigen Verbindung zersetzt und/oder chemisch umsetzt und der ringähnliche oxidische Formkörper sich ausbildet, und

dass die geometrische Form der Innenwand der Matrizenbohrung auf der Länge II des Längsabschnitts II von unten nach oben derjenigen der Mantelfläche eines sich von unten nach oben erweiternden Kegelstumpfes KS entspricht, dessen Querschnittsfläche an seinem unteren Ende der Querschnittsfläche des Kreiszylinders KZ an dessen oberem Ende entspricht, mit der Maßgabe, dass beim Erreichen des Endabstands E die untere Stirnfläche des oberen Stempels sich im Längsabschnitt II und die obere Stirnfläche des unteren Stempels sich nicht unterhalb des Längsabschnitts I befindet, so dass sich der durch das mechanische Verdichten des pulverförmigen Haufwerks zwischen den beiden Stirnflächen ausgebildete ringähnliche Vorläuferformkörper beim Erreichen des Endabstands E wenigstens teilweise im Längsabschnitt II befindet.

2. Verfahren gemäß Ausführungsform 1, dadurch gekennzeichnet, dass sich beim Erreichen des Endabstands E zwischen der oberen Stirnfläche des unteren Stempels und der unteren Stirnfläche des oberen Stempels wenigstens 20 % der Abstandsstrecke zwischen den beiden Stirnflächen im Längsabschnitt II befinden.

3. Verfahren gemäß Ausführungsform 1, dadurch gekennzeichnet, dass sich beim Erreichen des Endabstands E zwischen der oberen Stirnfläche des unteren Stempels und der unteren Stirnfläche des oberen Stempels wenigstens 60 % der Abstandsstrecke zwischen den beiden Stirnflächen im Längsabschnitt II befinden.

4. Verfahren gemäß Ausführungsform 1, dadurch gekennzeichnet, dass sich beim Erreichen des Endabstands E zwischen der oberen Stirnfläche des unteren Stempels und der unteren Stirnfläche des oberen Stempels wenigstens 90 % der Abstandsstrecke zwischen den beiden Stirnflächen im Längsabschnitt II befinden.

5. Verfahren gemäß Ausführungsform 1, dadurch gekennzeichnet, dass sich beim Erreichen des Endabstands E zwischen der oberen Stirnfläche des unteren Stempels und der unteren Stirnfläche des oberen Stempels der gesamte durch das mechanische Verdichten des pulverförmigen Haufwerks zwischen den beiden Stirnflächen ausgebildete ringähnliche Vorläuferformkörper im Längsabschnitt II befindet.

6. Verfahren gemäß einer der Ausführungsformen 1 bis 5, dadurch gekennzeichnet, dass die Umrisslinie des Kreiszylinders II länger oder gleich lang wie die Umrisslinie des Kreiszylinders I ist.

7. Verfahren gemäß einer der Ausführungsformen 1 bis 6, dadurch gekennzeichnet, dass die obere Stirnfläche des unteren Stempels und die untere Stirnfläche des oberen Stempels in zueinander parallelen Ebenen liegen, auf denen die Bohrachse B senkrecht steht.

8. Verfahren gemäß einer der Ausführungsformen 1 bis 7, dadurch gekennzeichnet, dass der Endabstand E 2 bis 10 mm beträgt.

9. Verfahren gemäß einer der Ausführungsformen 1 bis 7, dadurch gekennzeichnet, dass der Endabstand E 2 bis 8 mm beträgt.

10. Verfahren gemäß einer der Ausführungsformen 1 bis 7, dadurch gekennzeichnet, dass der Endabstand E 3 bis 8 mm beträgt.

11. Verfahren gemäß einer der Ausführungsformen 1 bis 7, dadurch gekennzeichnet, dass der Endabstand E 3 bis 7 mm beträgt.

12. Verfahren gemäß einer der Ausführungsformen 1 bis 11, dadurch gekennzeichnet, dass der Quotient Q aus der Länge der Umrisslinie des Kreiszylinders Z als Zähler und der Umrisslinie des Kreiszylinders I als Nenner 0,3 bis 0,7 beträgt.

13. Verfahren gemäß einer der Ausführungsformen 1 bis 11, dadurch gekennzeichnet, dass der Quotient Q aus der Länge der Umrisslinie des Kreiszylinders Z als Zähler und der Umrisslinie des Kreiszylinders I als Nenner 0,4 bis 0,6 beträgt.

14. Verfahren gemäß einer der Ausführungsformen 1 bis 12, dadurch gekennzeichnet, dass die Differenz, gebildet durch Subtraktion des Radius der Umrisslinie des Kreiszylinders Z vom Radius der Umrisslinie des Kreiszylinders I 1 bis 3 mm beträgt.

15. Verfahren gemäß einer der Ausführungsformen 1 bis 14, dadurch gekennzeichnet, dass der Durchmesser der Umrisslinie des Kreiszylinders I 2 bis 10 mm beträgt.

16. Verfahren gemäß einer der Ausführungsformen 1 bis 14, dadurch gekennzeichnet, dass der Durchmesser der Umrisslinie des Kreiszylinders I 2 bis 8 mm beträgt.

17. Verfahren gemäß einer der Ausführungsformen 1 bis 14, dadurch gekennzeichnet, dass der Durchmesser der Umrisslinie des Kreiszylinders I 4 bis 8 mm beträgt.

18. Verfahren gemäß einer der Ausführungsformen 1 bis 17, dadurch gekennzeichnet, dass der Kegelstumpf KS so beschaffen ist, dass zwischen dem Durchmesser DD der Deckfläche, dem Durchmesser DG der Grundfläche und der Höhe H des Kegelstumpfs KS die nachfolgende Beziehung erfüllt ist:

$$0{,}003 \cdot H \leq DG - DD \leq 0{,}050 \cdot H.$$

19. Verfahren gemäß einer der Ausführungsformen 1 bis 17, dadurch gekennzeichnet, dass der Kegelstumpf KS so beschaffen ist, dass zwischen dem Durchmesser DD der Deckfläche, dem Durchmesser DG der Grundfläche und der Höhe H des Kegelstumpfs KS die nachfolgende Beziehung erfüllt ist:

$$0{,}005 \cdot H \leq DG - DD \leq 0{,}025 \cdot H.$$

20. Verfahren gemäß einer der Ausführungsformen 1 bis 17, dadurch gekennzeichnet, dass der Kegelstumpf KS so beschaffen ist, dass zwischen dem Durchmesser DD der Deckfläche, dem Durchmesser DG der Grundfläche und der Höhe H des Kegelstumpfs KS die nachfolgende Beziehung erfüllt ist:

$$0{,}007 \cdot H \leq DG - DD \leq 0{,}015 \cdot H.$$

21. Verfahren gemäß einer der Ausführungsformen 1 bis 20, dadurch gekennzeichnet, dass sowohl die (für das pulverförmige Haufwerk zugänglich) obere Stirnfläche des unteren Stempels als auch die (für das pulverförmige Haufwerk zugängliche) untere Stirnfläche des oberen Stempels die geometrische Form eines Kreisrings aufweist.

22. Verfahren gemäß einer der Ausführungsformen 1 bis 20, dadurch gekennzeichnet, dass sowohl die obere Stirnfläche des unteren Stempels als auch die untere Stirnfläche des oberen Stempels die geometrische Form eines ins Stempelinnere einwärts gewölbten Kreisrings aufweist.

23. Verfahren gemäß einer der Ausführungsformen 1 bis 22, dadurch gekennzeichnet, dass die Matrizenbohrung nur die Längsabschnitte I und II aufweist.

24. Verfahren gemäß einer der Ausführungsformen 1 bis 22, dadurch gekennzeichnet, dass die Matrizenbohrung der Matrize so beschaffen ist, dass sich an ihren Längsabschnitt I nicht nur an dessen oberem Ende unmittelbar ein nach oben gerichteter Längsabschnitt II, sondern auch an dessen unterem Ende unmittelbar ein nach unten gerichteter Längsabschnitt II* der Länge II* anschließt, und die geometrische Form der Innenwand der Matrizenbohrung auf der Länge II* des Längsabschnitts II* der Mantelfläche eines Kegelstumpfes KS* entspricht, dessen Querschnittsfläche an seinem oberen Ende der Querschnittsfläche des Kreiszylinders KZ an dessen unterem Ende entspricht.

25. Verfahren gemäß Ausführungsform 24, dadurch gekennzeichnet, dass die Matrizenbohrung nur die Längsabschnitte I, II und II* aufweist.

26. Verfahren gemäß Ausführungsform 24 oder 25, dadurch gekennzeichnet, dass die geometrischen Ausmaße des Längsabschnitts II denen des Längsabschnitts II* entsprechen.

27. Verfahren gemäß einer der Ausführungsformen 1 bis 26, dadurch gekennzeichnet, dass zwischen der Höhe H des Kegelstumpfs KS und dem Endabstand E nachfolgende Beziehung erfüllt ist:

$$4 \cdot \text{Endabstand E} \geq H \geq 1 \cdot \text{Endabstand E.}$$

28. Verfahren gemäß einer der Ausführungsformen 1 bis 26, dadurch gekennzeichnet, dass zwischen der Höhe H des Kegelstumpfs KS und dem Endabstand E nachfolgende Beziehung erfüllt ist:

$$3 \cdot \text{Endabstand E} \geq H \geq 1 \cdot \text{Endabstand E.}$$

29. Verfahren gemäß einer der Ausführungsformen 1 bis 26, dadurch gekennzeichnet, dass zwischen der Höhe H des Kegelstumpfs KS und dem Endabstand E nachfolgende Beziehung erfüllt ist:

$$3 \cdot \text{Endabstand E} \geq H \geq 1{,}5 \cdot \text{Endabstand E.}$$

30. Verfahren gemäß einer der Ausführungsformen 1 bis 29, dadurch gekennzeichnet, dass die Länge des Längsabschnitts I größer ist als die Länge II des Längsabschnitts II.

31. Verfahren gemäß einer der Ausführungsformen 1 bis 29, dadurch gekennzeichnet, dass die Länge des Längsabschnitts I kleiner ist als die Länge II des Längsabschnitts II.

32. Verfahren gemäß einer der Ausführungsformen 1 bis 29, dadurch gekennzeichnet, dass die Länge des Längsabschnitts I nicht mehr als das Dreifache und nicht weniger als das 0,1-fache der Länge des Längsabschnitts II beträgt.

33. Verfahren gemäß einer der Ausführungsformen 1 bis 32, dadurch gekennzeichnet, dass wenigstens der Eingang in die Mittelbohrung $MB^O$ kreiszylindrisch so gestaltet ist, dass die Mantelfläche des Kreiszylinders Z bei dessen Aufnahme in die Mittelbohrung $MB^O$ wenigstens im Eingangsbereich derselben auf deren Innenwand gleitet.

34. Verfahren gemäß einer der Ausführungsformen 1 bis 33, dadurch gekennzeichnet, dass sich der Mittelstift MF innerhalb des Längsabschnitts II nach oben hin konisch verjüngt.

35. Verfahren gemäß einer der Ausführungsformen 1 bis 34, dadurch gekennzeichnet, dass das obere Ende des Längsabschnitts II der Matrizenbohrung, die obere Stirnfläche des Mittelstiftes MF und die obere Stirnfläche der Matrize miteinander bündig abschließen.

36. Verfahren gemäß einer der Ausführungsformen 1 bis 35, dadurch gekennzeichnet, dass das erfindungsgemäße Verfahren maschinell mit Hilfe eines Rundläufers durchgeführt wird.

37. Verfahren gemäß einer der Ausführungsformen 1 bis 36, dadurch gekennzeichnet, dass das mechanische Verdichten aus einer Vorverdichtung und aus einer dieser nachfolgenden Hauptverdichtung besteht, wobei der axiale Ausgangsabstand A im Rahmen der Vorverdichtung zunächst auf einen vorläufigen Endabstand $E^V$ verringert wird, und im Rahmen der Hauptverdichtung der vorläufige Endabstand $E^V$ auf den Endabstand E verringert wird.

38. Verfahren gemäß einer der Ausführungsformen 1 bis 37, dadurch gekennzeichnet, dass das pulverförmige Haufwerk wenigstens ein Metalloxid, Metallhydroxid, Metallcarbonat, Metallhydrogencarbonat, Metallhydrogenphosphat und/oder Metallnitrat umfasst.

39. Verfahren gemäß einer der Ausführungsformen 1 bis 38, dadurch gekennzeichnet, dass das pulverförmige Haufwerk wenigstens ein Metallnitrat aus der Gruppe bestehend aus Kobaltnitrat, Eisennitrat, Wismutnitrat, Nickelnitrat, Cäsiumnitrat, Kupfernitrat, Calciumnitrat und Magnesiumnitrat enthält.

40. Verfahren gemäß einer der Ausführungsformen 1 bis 39, dadurch gekennzeichnet, dass die Matrize aus einem Werkstoffverbund gefertigt ist, der auf seiner die Matrizenbohrung berührenden Seite aus einem Hartmetall und auf seiner von der Matrizenbohrung abgewandten Seite aus einem Werkzeugstahl besteht, der folgende Elementzusammensetzung aufweist:

| | |
|---|---|
| 1,50 bis 1,80 Gew.-% | C, |
| 0,10 bis 0,40 Gew.-% | Si, |
| 0,10 bis 0,50 Gew.-% | Mn, |
| $\geq 0$ bis 0,05 Gew.-% | P, |
| $\geq 0$ bis 0,05 Gew.-% | S, |
| 10 bis 13 Gew.-% | Cr, |
| 0,50 bis 0,80 Gew.-% | Mo, |
| 0,10 bis 1,10 Gew.-% | V, |
| $\geq 0$ bis 0,60 Gew.-% | W, und |
| $\geq 0$ bis 0,10 Gew.-% | eines oder mehrere seltene Erdmetalle, |
| und im | übrigen Fe und herstellungsbedingte Vergungen. |
| unreini- | |

41. Verfahren gemäß Ausführungsform 40, dadurch gekennzeichnet, dass das Hartmetall zu $\geq 90$ Gew.-% aus Wolframcarbid und zu wenigstens 5 Gew.-% aus Nickel oder aus Nickel und Chrom besteht.

42. Verfahren gemäß Ausführungsform 40, dadurch gekennzeichnet, dass das Hartmetall aus

| | |
|---|---|
| 90 bis 95 Gew.-% | WC, |
| $\geq 0$ bis 1 Gew.-% | TiC und/oder TaNbC, und |
| 5 bis 10 Gew.-% | Ni oder Ni und Cr |

besteht.

43. Verfahren gemäß einer der Ausführungsformen 1 bis 42, dadurch gekennzeichnet, dass das pulverförmige Haufwerk Salpetersäure, ein Ammoniumsalz und/oder ein Nitratsalz enthält.

44. Verfahren gemäß einer der Ausführungsformen 1 bis 43, dadurch gekennzeichnet, dass der Mittenrauhwert $R_a$ der Innenwand der Matrizenbohrung $\leq 0,2$ $\mu$m beträgt.

45. Verfahren gemäß einer der Ausführungsformen 1 bis 43, dadurch gekennzeichnet, dass der Mittenrauhwert $R_a$ der Innenwand der Matrizenbohrung $\leq 0,1$ $\mu$m beträgt.

46. Verfahren gemäß einer der Ausführungsformen 1 bis 45, dadurch gekennzeichnet, dass im Endabstand E von beiden Stempeln ein Pressdruck ausgeübt wird, der im Bereich 50 bis 5000 kg/cm$^2$ liegt.

47. Verfahren gemäß einer der Ausführungsformen 1 bis 45, dadurch gekennzeichnet, dass im Endabstand E von beiden Stempeln ein Pressdruck ausgeübt wird, der im Bereich 500 bis 2500 kg/cm$^2$ liegt.

48. Verfahren gemäß einer der Ausführungsformen 1 bis 47, dadurch gekennzeichnet, dass das Verfahren der thermischen Behandlung der hergestellten ringähnlichen Vorläuferformkörper bei einer Temperatur $\geq 200°$Cerfolgt.

49. Verfahren gemäß einer der Ausführungsformen 1 bis 47, dadurch gekennzeichnet, dass das Verfahren der thermischen Behandlung der hergestellten ringähnlichen Vorläuferformkörper bei einer Temperatur $\geq 300$ °C erfolgt.

50. Verfahren gemäß einer der Ausführungsformen 1 bis 49, dadurch gekennzeichnet, dass mit der thermischen Behandlung der ringähnlichen Vorläuferformkörper ein auf ihr Ausgangsgewicht bezogener Gewichtsverlust von 0,5 bis 40 Gew.-% einhergeht.

51. Verfahren gemäß einer der Ausführungsformen 1 bis 50, dadurch gekennzeichnet, dass die sich bei der thermischen Behandlung ausbildende wenigstens eine gasförmige Verbindung Ammoniak, $H_2O$, CO, $CO_2$ und/oder ein Stickstoffoxid ist.

52. Verfahren gemäß einer der Ausführungsformen 1 bis 51, dadurch gekennzeichnet, dass das pulverförmige Haufwerk wenigstens eine Substanz aus der Gruppe bestehend aus $NH_4OH$, $(NH_4)_2CO_3$, $NH_4HCO_3$, $NH_4NO_3$, $NH_4CHO_2$, $NH_4HSO_4$, $(NH_4)_2SO_4$, $NH_4CH_3CO_2$, Ammoniumoxalat und den Hydraten der vorgenannten Ammoniumsalze zugesetzt enthält.

53. Verfahren gemäß einer der Ausführungsformen 1 bis 52, dadurch gekennzeichnet, dass das pulverförmige Haufwerk Graphit, Stärke, gemahlene Nussschale, feinteiliges Kunststoffgranulat, Cellulose, Stearinsäure, Malonsäure, Salz der Stearinsäure und/oder Salz der Malonsäure zugesetzt enthält.

54. Verfahren gemäß einer der Ausführungsformen 1 bis 53, dadurch gekennzeichnet, dass bei dem anschließenden Verfahren der thermischen Behandlung der hergestellten ringähnlichen Vorläuferformkörper in den ring-ähnlichen Vorläuferformkörpern ein Multimetalloxid ausgebildet wird, das die Elemente Mo und Fe, oder die Elemente Mo, Fe und Bi, oder die Elemente Mo und V, oder die Elemente Mo, V und P, oder die Elemente V und P enthält.

55. Verfahren gemäß einer der Ausführungsformen 1 bis 54, dadurch gekennzeichnet, dass bei dem anschließenden Verfahren der thermischen Behandlung der hergestellten ringähnlichen Vorläuferformkörper in selbigen ein Multimetalloxid ausgebildet wird, in welchem das Element Mo, oder das Element V, oder das Element P dasjenige von Sauerstoff verschiedene Element ist, das molar gerechnet das numerisch am häufigsten enthaltene ist.

56. Verfahren gemäß einer der Ausführungsformen 1 bis 55, dadurch gekennzeichnet, dass bei dem anschließenden Verfahren der thermischen Behandlung der hergestellten ringähnlichen Vorläuferformkörper in selbigen ein Multimetalloxid der allgemeinen Formel XII,

$$MO_{12}Bi_aFe_bX^1_cX^2_dX^3_eX^4_fO_n \qquad (XII),$$

mit

X$^1$ = Nickel und/oder Kobalt,
X$^2$ = Thallium, Samarium, ein Alkalimetall und/oder ein Erdalkalimetall,
X$^3$ = Zink, Phosphor, Arsen, Bor, Antimon, Zinn, Cer, Blei, Vanadium, Chrom, Niob und/oder Wolfram,
X$^4$ = Silicium, Aluminium, Titan und/oder Zirkonium,

a = 0,2 bis 5,
b = 0,01 bis 5,
c = 0 bis 10,
d = 0 bis 2,
e = 0 bis 8,
f = 0 bis 10, und

n = eine Zahl, die durch die Wertigkeit und Häufigkeit der von Sauerstoff verschiedenen Elemente in XII bestimmt wird,

ausgebildet wird.

57. Verfahren gemäß einer der Ausführungsformen 1 bis 55, dadurch gekennzeichnet, dass bei dem anschließenden Verfahren der thermischen Behandlung der hergestellten ringähnlichen Vorläuferformkörper in selbigen ein Multimetalloxid der allgemeinen Formel XIII,

$$[Y^1{}_{a'}Y^2{}_{b'}O_{x'}]_p[Y^3{}_{c'}Y^4{}_{d'}Y^5{}_{e'}Y^6{}_{f'}Y^7{}_{g'}Y^8{}_{h'}O_{y'}]_q \qquad (XIII),$$

mit

| | | |
|---|---|---|
| $Y^1$ = | nur Wismut oder Wismut und wenigstens eines der Elemente Tellur, Antimon, Zinn und Kupfer, | |
| $Y^2$ = | Molybdän oder Wolfram, oder Molybdän und Wolfram, | |
| $Y^3$ = | ein Alkalimetall, Thallium und/oder Samarium, | |
| $Y^4$ = | ein Erdalkalimetall, Nickel, Kobalt, Kupfer, Mangan, Zink, Zinn, Cadmium und/oder Quecksilber, | |
| $Y^5$ = | Eisen oder Eisen und wenigstens eines der Elemente Vanadium, Chrom und Cer, | |
| $Y^6$ = | Phosphor, Arsen, Bor und/oder Antimon, | |
| $Y^7$ = | ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran, | |
| $Y^8$ = | Molybdän oder Wolfram, oder Molybdän und Wolfram, | |
| a' = | 0,01 bis 8, | |
| b' = | 0,1 bis 30, | |
| c' = | 0 bis 4, | |
| d' = | 0 bis 20, | |
| e' > | 0 bis 20, | |
| f' = | 0 bis 6, | |
| g' = | 0 bis 15, | |
| h' = | 8 bis 16, | |

x', y' = Zahlen, die durch die Wertigkeit und Häufigkeit der von Sauerstoff verschiedenen Elemente in XIII bestimmt werden, und

p, q = Zahlen, deren Verhältnis p/q 0,1 bis 10 beträgt,

ausgebildet wird.

58. Verfahren gemäß einer der Ausführungsformen 1 bis 55, dadurch gekennzeichnet, dass bei dem anschließenden Verfahren der thermischen Behandlung der hergestellten ringähnlichen Vorläuferformkörper in selbigen ein Multimetalloxid der allgemeinen Formel XIV,

$$[Bi_{a''}Z^2{}_{b''}O_{x''}]_{p''}[Z^8{}_{12}Z^3{}_{c''}Z^4{}_{d''}Fe_{e''}Z^5{}_{f''}Z^6{}_{g''}Z^7{}_{h''}O_{y''}]_{q''} \qquad (XIV),$$

mit

| | | |
|---|---|---|
| $Z^2$ = | Molybdän oder Wolfram, oder Molybdän und Wolfram, | |
| $Z^3$ = | Nickel und/oder Kobalt, | |
| $Z^4$ = | Thallium, ein Alkalimetall und/oder ein Erdalkalimetall, vorzugsweise K, Cs und/oder Sr, | |
| $Z^5$ = | Phosphor, Arsen, Bor, Antimon, Zinn, Cer, Vanadium, Chrom und/oder Bi, | |
| $Z^6$ = | Silicium, Aluminium, Titan und/oder Zirkonium, vorzugsweise Si, | |
| $Z^7$ = | Kupfer, Silber und/oder Gold, | |
| $Z^8$ = | Molybdän oder Wolfram, oder Wolfram und Molybdän | |
| a" = | 0,1 bis 1, | |
| b" = | 0,2 bis 2, | |
| c" = | 3 bis 10, | |
| d" = | 0,02 bis 2, | |
| e" = | 0,01 bis 5, vorzugsweise 0,1 bis 3, | |
| f" = | 0 bis 5, | |
| g" = | 0 bis 10, vorzugsweise > 0 bis 10, besonders bevorzugt 0,2 bis 10 und ganz besonders bevorzugt 0,4 bis 3, | |
| h" = | 0 bis 1, | |

x", y" = Zahlen, die durch die Wertigkeit und Häufigkeit der von Sauerstoff verschiedenen Elemente in XIV bestimmt werden, und

p", q"= Zahlen, deren Verhältnis p" / q" 0,1 bis 5 beträgt,

ausgebildet wird.

59. Verfahren gemäß einer der Ausführungsformen 1 bis 55, dadurch gekennzeichnet, dass bei dem anschließenden Verfahren der thermischen Behandlung der hergestellten ringähnlichen Vorläuferformkörper in selbigen ein Multimetalloxid der allgemeinen Formel XV,

$$Mo_{12}P_aV_bX_c^1X_d^2X_e^3Sb_fRe_gS_hO_n \qquad (XV),$$

mit

$X^1 =$ Kalium, Rubidium und/oder Cäsium,
$X^2 =$ Kupfer und/oder Silber,
$X^3 =$ Cer, Bor, Zirkonium, Mangan und/oder Wismut,
$a =$ 0,5 bis 3,
$b =$ 0,01 bis 3,
$c =$ 0,2 bis 3,
$d =$ 0,01 bis 2,
$e =$ 0 bis 2,
$f =$ 0 bis 2, vorzugsweise 0,01 bis 2,
$g =$ 0 bis 1,
$h =$ 0 bis 0,5, vorzugsweise 0,001 bis 0,5, und
$n =$ eine Zahl, die durch die Wertigkeit und Häufigkeit der von Sauerstoff verschiedenen Elemente in XV bestimmt wird,

ausgebildet wird.

60. Verfahren gemäß einer der Ausführungsformen 1 bis 55, dadurch gekennzeichnet, dass bei dem anschließenden Verfahren der thermischen Behandlung der hergestellten ringähnlichen Vorläuferformkörper in selbigen ein Multimetalloxid der allgemeinen Formel XVI,

$$V_1P_bFe_cX_d^1X_e^2O_n \qquad (XVI),$$

mit

$X^1 =$ Mo, Bi, Co, Ni, Si, Zn, Hf, Zr, Ti, Cr, Mn, Cu, B, Sn und/oder Nb,
$X^2 =$ Li, K, Na, Rb, Cs und/oder Tl,
$b =$ 0,9 bis 1,5,
$c =$ 0 bis 0,1,
$d =$ 0 bis 0,1,
$e =$ 0 bis 0,1, und
$n =$ eine Zahl, die durch die Wertigkeit und Häufigkeit der von Sauerstoff verschiedenen Elemente in XVI bestimmt wird,

ausgebildet wird.

61. Verfahren gemäß einer der Ausführungsformen 1 bis 53, dadurch gekennzeichnet, dass bei dem anschließenden Verfahren der thermischen Behandlung der hergestellten ringähnlichen Vorläuferformkörper in selbigen ein bei Normalbedingungen festes Oxid ausgebildet wird, in welchem kein Übergangsmetall der 5. bis 11. Nebengruppe und auch nicht Phosphor dasjenige von Sauerstoff verschiedene Element ist, das molar gerechnet das numerisch am häufigsten enthaltene ist.

62. Verfahren gemäß einer der Ausführungsformen 1 bis 53, dadurch gekennzeichnet, dass das pulverförmige Haufwerk wenigstens ein Metalloxid aus der Gruppe bestehend aus Aluminiumoxid, Wolframoxid, Antimonoxid, Zirkoniumoxid, Wismutoxid, Molybdänoxid, Siliciumoxid, Magnesiumoxid und Mischoxiden, die wenigstens zwei der in den vorgenannten Metalloxiden enthaltenen Metallelemente enthalten, enthält.

63. Verfahren gemäß einer der Ausführungsformen 1 bis 62, dadurch gekennzeichnet, dass sich bereits im Ausgangsabstand A sowohl die obere Stirnfläche des unteren Stempels als auch die untere Stirnfläche des oberen Stempels im Längsabschnitt II der Matrizenbohrung befinden.

64. Verfahren der heterogen katalysierten partiellen Gasphasenoxidation wenigstens einer organischen Verbindung an einem Katalysatorfestbett, dadurch gekennzeichnet, dass das Katalysatorfestbett einen ringähnlichen oxidischen Formkörper, der nach einem Verfahren gemäß einer der Ausführungsformen 1 bis 63 erhalten wurde, enthält.

65. Verfahren gemäß Ausführungsform 64, dadurch gekennzeichnet, dass die heterogen katalysierte partielle Gasphasenoxidation diejenige

a) von Propylen zu Acrolein und/oder Acrylsäure oder
b) von Acrolein zu Acrylsäure, oder
c) von Methacrolein zu Methacrylsäure, oder
d) von iso-Buten zu Methacrolein und/oder Methacrylsäure, oder
e) von Propan zu Acrolein und/oder Acrylsäure, oder
f) von iso-Butan zu Methacrolein und/oder Methacrylsäure, oder
g) von wenigstens einem $C_4$-Kohlenwasserstoff und/oder Benzol zu Maleinsäureanhydrid, oder
h) von Methanol zu Formaldehyd oder
i) die Oxichlorierung von Ethylen zu 1,2-Dichlorethan ist.

66. Rohrbündelreaktor, dessen Reaktionsrohre wenigstens einen ringähnlichen oxidischen Formkörper, der nach einem Verfahren gemäß einer der Ausführungsformen 1 bis 63 erhalten wurde, enthalten.

Beispiele und Vergleichsbeispiele

I. Herstellung von ringähnlichen Träger-Vorläuferformkörpern

Aus 220 kg feinteiligem $\gamma$-$Al_2O_3$ (Puralox® SCF a-230 der Fa. Sasol in D-25534 Brunsbüttel), 148 kg feinteiligem Pseudoböhmit (Pural® SCF 55 der Fa. Sasol), 12 kg Magnesiumstearat (der Fa. Peter Greven Fett-Chemie GmbH, Schmelzpunkt 145-160 °C, Schüttgewicht 200-300 g/h, Asche 6,8-8,3 Gew.-%, Feuchte < 2,0 Gew.-%) und 4,0 kg feinteiligem Graphit (Timrex® T44 der Firma Timcal AG Ltd., 6743 Bodio, Schweiz, mit $d_{10}$ = 6,4 $\mu$m, $d_{50}$ = 20,8 $\mu$m, $d_{90}$ = 56,8 $\mu$m) wurde mit Hilfe eines Mischers ein homogenes pulverförmiges Haufwerk erzeugt.
Die Spezifikation des Puralox war:

| | | |
|---|---|---|
| spezifische Oberfläche | = | 212 $m^2$/g, |
| $Al_2O_3$-Gehalt | = | 99,3 Gew.-%, |
| Schüttdichte | = | 0,61 g/ml, |
| Schütteldichte | = | 0,80 g/ml, |
| Si-Gehalt | = | 45 Gew.ppm, |
| Fe-Gehalt | = | 96 Gew.-ppm, |
| Na-Gehalt | = | 19 Gew.-ppm, |
| $\geq$ 12 $\mu$m | = | 85,5 Gew.-%, |
| < 25 $\mu$m | = | 34,2 Gew.-%, |
| < 45 $\mu$m | = | 68,5 Gew.-%, |
| > 64 $\mu$m | = | 12,6 Gew.-%, |
| < 90 $\mu$m | = | 96,9 Gew.-%, |
| $d_{50}$ | = | 33,8 $\mu$m. |

Die Spezifikation des Pural war:

| | | |
|---|---|---|
| spezifische Oberfläche | = | 239 $m^2$/g, |
| $Al_2O_3$-Gehalt | = | 75,1 Gew.-%, |
| Schüttdichte | = | 0,62 g/ml, |
| Schütteldichte | = | 0,90 g/ml, |
| Kohlenstoff | = | 0,14 Gew.-%, |
| > 12 $\mu$m | = | 72,6 Gew.-%, |
| < 25 $\mu$m | = | 68,1 Gew.-%, |
| < 45 $\mu$m | = | 95,5 Gew.-%, |
| > 48 $\mu$m | = | 3,0 Gew.-%, |
| < 90 $\mu$m | = | 100 Gew.-%, |
| $d_{50}$ | = | 18,8 $\mu$m. |

Anschließend wurde das pulverförmige Haufwerk mit Hilfe eines Kilian Synthesis 700 Rundläufers erfindungsgemäß verdichtet (Einfachwerkszeug, 77 Matrizen). Der prinzipielle Vorrichtungsaufbau war wie in Figur 6. Der Durchmesser der Vordruckrolle betrug 210 mm und der Durchmesser der Hauptdruckrolle betrug ebenfalls 210 mm. Der Abstand zweier auf der Matrizenscheibe einander gegenüberliegender Matrizen betrug 720 mm.

Die verwendeten Matrizen waren Matrizen mit kongruentem doppeltem Kegelstumpf wie in den Figuren 3a, 3b schematisch gezeigt.

Die Länge I des Längsabschnitts I betrug 6,2 mm.

Die Länge II (die Länge II*) des Längsabschnitts II (des Längsabschnitts II*) betrug 8 mm.

Die Umrisslinie des Kreiszylinders I und des Kreiszylinders II betrugen 15,7 mm. Der Durchmesser DD der Deckfläche des Kegelstumpfes KS betrug 5,1 mm. Der Durchmesser DG der Grundfläche des Kegelstumpfes KS betrug 5,0 mm. Die Länge der Umrisslinie des Kreiszylinders Z (des durchgehend kreiszylindrischen Mittelstiftes) betrug $2,5 \cdot \pi$ mm. Die obere plane Stirnfläche des Mittelstiftes MF schloss mit der planen oberen Matrizenstirnfläche bündig ab.

Beide Mittelbohrungen, $MB^U$ und $MB^O$ (die letztere stand mit zwei gasdurchlässigen Auslässen in Verbindung (vgl. Figur 4d)), wiesen im Eingangsbereich in die zugehörige Stirnfläche eine kreiszylindrische Geometrie mit identischem Radius auf. Der Kontakt ihrer Innenwände zur äußeren Mantelfläche des Mittelstiftes MF war im möglichen Kontaktbereich aufeinander gleitend.

Es wurden nur ringähnliche Vorläuferformkörper $F^{LII}$ gefertigt, wobei der Endabstand E stets 5 mm betrug.

Die obere Stirnfläche des unteren Stempels und die untere Stirnfläche des oberen Stempels waren gemäß EP-A 184790 in gleicher (kongruenter) Weise konkav gestaltet. Die Bohrachse B verlief senkrecht zu beiden Stempelquerschnitten. Die Rillentiefe betrug 0,8 mm.

Die einzelne Matrize war aus einem Werkstoffverbund gefertigt. Dieser bestand auf seiner die Matrizenbohrung berührenden Seite aus dem Hartmetall G 10-Ni (6,9 bis 7,0 mm Wanddicke) mit $R_a = 0,1 \, \mu m$ und auf seiner von der Matrizenbohrung abgewandten Seite aus DIN-Werkzeugstahl 1.2379 (6 mm Wanddicke) mit $R_a = 0,8 \, \mu m$. Der obere Stempel und der untere Stempel waren aus DIN-Werkstoff 1.2601 gefertigt. Der auf seiner Gesamtlänge kreiszylindrische Mittelstift MF war aus DIN-Werkzeugstahl 1.2343 gefertigt ($R_a = 0,4 \, \mu m$). $R_a$ der beiden Stirnflächen betrug ebenfalls 0,4 $\mu m$.

Die in den Füllraum eingebrachte Menge an pulverförmigem Haufwerk betrug 118 mg.

Beim Beginn des Verfahrens schloss die untere Stirn des oberen Stempels im Zustand des Ausgangsabstands A mit dem oberen Ende des Längsabschnitts II bündig ab.

Mit zunehmender Abnutzung der Innenwand des oberen Teils des Längsabschnitts II der Matrizenbohrung wurden im Zustand des Ausgangsabstands A die Positionen beider Stirnflächen innerhalb des Längsabschnitts II nach unten verschoben.

Die angewandte Vordruckkraft (Presskraft) betrug bei jedem der beiden Stempel 0,5 kN, die angewandte Hauptdruckkraft (Presskraft) betrug bei jedem der beiden Stempel 8,5 kN (Presskraftangaben beziehen sich stets auf den Zustand des Endabstands E).

Die Seitendruckfestigkeiten der resultierenden ringähnlichen Träger-Vorläuferformkörper lagen im Bereich von 19 bis 23 N.

Die Umdrehungsrate des Rundläufers lag bei 25 bis 30 UPM.

Hinsichtlich des Materials von Matrizenscheibenzunge, Matrizenscheibenstirn und Matrizenscheibenkinn gilt das in der Beschreibung Gesagte.

Anschließend wurden die erhaltenen ringähnlichen Träger-Vorläuferformkörper auf einem Bandcalcinierer (vgl. DE-A 10046957 sowie WO 02/24620) thermisch behandelt. Die Schüttguthöhe auf dem umlaufenden Band betrug 80 mm. Die Temperatur in der ersten Calcinierkammer betrug 690 °C, diejenige in der zweiten Calcinierkammer betrug 700 °C. Das grobmaschige Band wurde von unten mit Zuluft durchströmt, die mittels rotierender Ventilatoren angesaugt wurde, so dass die zeitliche und örtliche Abweichung der Temperatur vom Sollwert stets $\leq$ 2 °C war. Die Verweilzeit in der ersten Kammer war 2 h und die Verweilzeit in der zweiten Kammer war Ebenfalls 2 h.

Anschließend wurden die gebildeten ringähnlichen Trägerformkörper einer Siebung unterworfen.

Bei den verwendeten Sieben handelte es sich um Langlochsiebe. Bei der Überkornabsiebung betrug ihre geradlinige Kantenlänge 20 mm und der Abstand der beiden Kanten war 8 mm. Bei der sich daran anschließenden Unterkornsiebung betrug ihre geradlinige Kantenlänge 4 mm und der Abstand der beiden Kanten war 2 mm.

Bezogen auf das Gewicht des insgesamt zur Siebung aufgegebenen Siebgutes betrug der anfallende Unterkornanteil 2 Gew.-%.

Wurde in gleicher Weise wie vorstehend beschrieben verfahren, die Verdichtung des pulverförmigen Haufwerks jedoch mittels einer Matrize durchgeführt, deren

Matrizenbohrung ideal kreiszylindrisch war (Durchmesser = 5 mm; der Durchmesser der oberen und der unteren Stirnfläche betrug 4,95 mm), lag der bei der Siebung anfallende Unterkornanteil bei 7 Gew.-%.

Alternativ zur beschriebenen erfindungsgemäßen Verdichtung mit Hilfe eines Kilian Synthesis 700 Rundläufers kann die erfindungsgemäße Verdichtung auch mit einem Kirsch PH 800 Rundläufer ausgeführt werden. In diesem Fall wird ohne Vordruck verdichtet. Als Hauptdruckkraft (Presskraft) können bei jedem der beiden Stempel z.B. 8,2 kN angewendet werden.

Die wie beschrieben hergestellten ringähnlichen Trägerformkörper eignen sich z.B. für den in der WO 99/48606 beschriebenen Verwendungszweck.

II. Herstellung von ringähnlichen Multimetalloxid-Vollkatalysatorvorläuferformkörpern, wobei das aktive Multimetalloxid die Stöchiometrie

$[Bi_2W_2O_9 \cdot 2\,WO_3]_{0,40}[Mo_{12}Co_{5,4}Fe_{3,1}Si_{1,5}K_{0,08}O_x]_1$, aufweist

1. Herstellung einer Ausgangsmasse 1

In 780 kg einer 25 °C aufweisenden wässrigen salpetersauren Wismutnitratlösung (11,2 Gew.-% Bi; freie Salpetersäure 3 bis 5 Gew.-%; Massendichte: 1,22 bis 1,27 g/ml, hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni, Ag, Fe, je < 3 mg/kg Cu, Sb und < 1 mg/kg Cd, Zn) wurden bei 25°C innerhalb von 20 min por-tionsweise 214,7 kg einer 25 °C aufweisenden Wolframsäure (74,1 Gew.-% W, H.C. Starck, D-38615 Goslar, Reinheit > 99,9 Gew.-% $WO_3$ nach Glühen bei 750 °C, 0,4 $\mu m$ < $d_{50}$ < 0,8 $\mu m$) eingerührt (70 U/min oder "70 UPM"). Das resultierende wässrige Gemisch wurde anschließend noch 3 h bei 25°C gerührt und dann sprühgetrocknet.

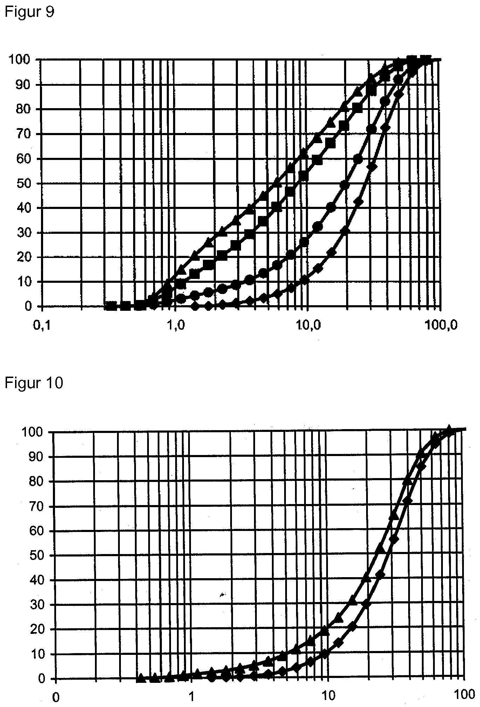

Die Sprühtrocknung erfolgte in einem Drehscheibensprühturm im Heißluftgleichstrom bei einer Gaseintrittstemperatur von 300 ± 10 °C, einer Gasaustrittstemperatur von 100 ± 10°C, einer Scheibendrehzahl von 18000 U/min und einem Durchsatz von 200 I/h. Das resultierende Sprühpulver wies einen Glühverlust von 12,8 Gew.-% (3 h bei 600°C im Porzellantiegel (der bei 900 °C bis zur Gewichtskonstanz geglüht worden war) unter Luft glühen) und (bei einem Dispergierdruck von 1,1 bar absolut) einen $d_{50}$ von 28,0 $\mu m$ ($d_{10}$ = 9,1 $\mu m$, $d_{90}$ = 55,2 $\mu m$) auf. Figur 9 zeigt die Partikeldurchmesserverteilung des resultierenden Sprühpulvers in Abhängigkeit vom angewendeten Dispergierdruck.

Die Abszisse zeigt die Partikeldurchmesser in logarithmischer Auftragung in $\mu m$.

Die Ordinate zeigt den Volumenanteil in % des Gesamtpartikelvolumens, der den entsprechenden Partikeldurchmesser aufweist in Abhängigkeit vom angewandten Dispergierdruck:

▲ : Dispergierdruck = 2 bar abs..

■ : Dispergierdruck = 1,5 bar abs

● : Dispergierdruck = 1,2 bar abs

♦ : Dispergierdruck = 1,1 bar abs

Die nachfolgende Tabelle gibt einen Überblick über repräsentative $d_x$-Werte in Abhängigkeit vom angewandten absoluten Dispergierdruck:

| | 2 bar | 1,5 bar | 1,2 bar | 1,1 bar |
|---|---|---|---|---|
| $d_{10}$ ($\mu m$) | 0,91 | 1,17 | 3,4 | 9,1 |
| $d_{50}$ ($\mu m$) | 5,8 | 8,5 | 19,7 | 28,0 |
| $d_{90}$ ($\mu m$) | 27,5 | 34,3 | 47,2 | 55,2 |

Das erhaltene Sprühpulver wurde anschließend mit 16,7 Gew.-% (bezogen auf das Pulver) an 25 °C aufweisendem Wasser in einem Kneter (20 U/min) für 30 min angeteigt und mittels eines Extruders (Drehmoment: ≤ 50 Nm) zu Strängen des Durchmessers 6 mm extrudiert. Diese wurden in Abschnitte von 6 cm geschnitten, auf einem 3-zonigen Bandtrockner bei einer Verweilzeit von 120 min je Zone bei Temperaturen von 90-95°C (Zone 1), 115 °C (Zone 2) und 125 °C (Zone 3) an Luft getrocknet und dann bei einer Temperatur im Bereich um 830°C thermisch behandelt (calciniert; im luftdurchströmten Drehrohrofen (0,3 mbar Unterdruck, 1,54 m³ Innenvolumen, 200 Nm³/h Luft, 50 kg/h Extrudat, Drehzahl: 1 U/min, bei 4 m Länge des Drehrohres 7 cm Neigung)). Wesentlich bei der genauen Einstellung der Calcinationstemperatur ist, dass sie an der angestrebten Phasenzusammensetzung des Calcinationsprodukts orientiert zu erfolgen hat. Gewünscht sind die Phasen $WO_3$ (monoklin) und $Bi_2W_2O_9$ (orthorhombisch), unerwünscht ist das Vorhan-densein von $\gamma$-$Bi_2WO_6$ (Russellit). Sollte daher nach der Calcination die Verbindung $\gamma$-$Bi_2WO_6$ anhand eines Reflexes im Röntgenpulverdiffraktogramm bei einem Reflexwinkel von $2\Theta$ = 28,4° (Cu$K\alpha$-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde mit einer Biplexmühle BQ500 mit 2500 U/min gemahlen, so dass der dso-Wert 2,45 $\mu m$ ($d_{10}$ = 1,05 $\mu m$, $d_{90}$ = 5,9 $\mu m$, gemessen bei einem Dispergierdruck von 2 bar absolut) und die BET-Oberfläche 0,8 m$^2$/g betrug.

Das Mahlgut wurde dann in Portionen von 20 kg in einem Schräglagen-Mischer (Typ VIS, Füllvolumen: 60 l, Aachener Misch- und Knetmaschinenfabrik) mit Misch- und Schneidflügel (Drehzahl Mischflügel:60 U/min, Drehzahl Schneidflügel: 3000 U/min) innerhalb von 5 min homogen mit 0,5 Gew.-% (bezogen auf das Mahlgut) feinteiligem SiO$_2$ der Fa. Degussa vom Typ Sipernat$^®$ D17 (Rüttelgewicht 150 g/l; d$_{50}$-Wert der SiO$_2$-Partikel (Laserbeugung nach ISO 13320-1) betrug 10 $\mu$m, die spezifische Oberfläche (Stickstoffadsorption nach ISO 5794-1, Annex D) betrug 100 m$^2$/g) vermischt.

2. Herstellung einer Ausgangsmasse 2

Eine Lösung A wurde hergestellt, indem man bei 60°C unter Rühren (70 U/min) zu 660 l eine Temperatur von 60°C aufweisendem Wasser innerhalb einer Minute 1,075 kg einer eine Temperatur von 60°C aufweisenden wässrigen Kaliumhydroxidlösung (47,5 Gew.-% KOH) und anschließend mit einer Dosiergeschwindigkeit von 600 kg/h 237,1 kg Ammoniumheptamolybdat-tetrahydrat (weiße Kristalle mit einer Körnung d < 1 mm, 81,5 Gew.-% MoO$_3$, 7,0-8,5 Gew.-% NH$_3$, max. 150 mg/kg Alkalimetalle, H.C. Starck, D-38642 Goslar) dosierte und die resultierende leicht trübe Lösung bei 60°C 60 min rührte.

Eine Lösung B wurde hergestellt, indem man bei 60°C in 282,0 kg einer eine Temperatur von 60 °C aufweisenden wässrigen Kobalt(-II)-nitratlösung (12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte und zu dieser unter Rühren (70 U/min) 142,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-% Sulfat, Dr. Paul Lohmann GmbH, D-81857 Emmerthal) dosierte. Anschließend wurde unter Aufrechterhaltung der 60°C 30 Minuten nachgerührt. Dann wurde unter Beibehalt der 60 °C die Lösung B in die vorgelegte Lösung A abgelassen und weitere 15 Minuten bei 60°C gerührt. Anschließend wurden dem resultierenden wässrigen Gemisch 19,9 kg eines Kieselgels der Fa. Grace GmbH in D-67547 Worms vom Typ Ludox$^®$ TM-50 (50 Gew.-% SiO$_2$; stabilisierendes Gegen-Ion: Na$^+$; Partikelladung: negativ; Verhältnis Si : Na als Gewichtsverhältnis SiO$_2$/Na$_2$O : 225; SiO$_2$-Gehalt: 50 Gew.-%; pH-Wert: 9,0; Massendichte (25 °C, 1 atm) : 1,40 g/cm$^3$; Sulfate (als Na$_2$SO$_4$) : 0,08 Gew.-%; titrierbares Alkali (als Na$_2$O) : 0,21 Gew.-%; Viskosität (25 °C, 1 atm) : 40 cP; spezifische Oberfläche der SiO$_2$-Partikel: 140 m$^2$/g) zugegeben und danach noch weitere 15 Minuten bei 60 °C gerührt.

Anschließend wurde in einem Drehscheibensprühturm vom Typ FS-15 der Firma Niro im Heißluftgegenstrom sprühgetrocknet (Gaseintrittstemperatur: 350 $\pm$ 10°C, Gasaustrittstemperatur: 140 $\pm$ 5°C, Scheibendrehzahl: 18000 U/min, Durchsatz:

270 kg/h). Das resultierende Sprühpulver wies einen Glühverlust von 30,5 Gew.-% (3 h bei 600°C im Porzellantiegel (der bei 900 °C bis zur Gewichtskonstanz geglüht worden war) unter Luft glühen) und (bei einem Dispergierdruck von 2,0 bar absolut) einen d$_{50}$ von 23,6 $\mu$m (d$_{10}$ = 5,2 $\mu$m, d$_{90}$ = 49,5 $\mu$m) auf. Figur 10 zeigt die Partikeldurchmesserverteilung des resultierenden Sprühpulvers in Abhängigkeit vom angewendeten Dispergierdruck.

Die Abszisse zeigt die Partikeldurchmesser in logarithmischer Auftragung in $\mu$m. Die Ordinate zeigt den Volumenanteil in % des Gesamtpartikelvolumens, der den entsprechenden Partikeldurchmesser aufweist in Abhängigkeit vom angewandten Dispergierdruck:

▲ : Dispergierdruck = 2 bar abs..

♦ : Dispergierdruck = 1,1 bar abs

Die nachfolgende Tabelle gibt einen Überblick über repräsentative d$_x$-Werte in Abhängigkeit vom angewandten absoluten Dispergierdruck:

|  | 2 bar | 1,1 bar |
|---|---|---|
| d$_{10}$ ($\mu$m) | 5,2 | 9,9 |
| d$_{50}$ ($\mu$m) | 23,6 | 28,5 |
| d$_{90}$ ($\mu$m) | 49,5 | 56,3 |

3. Herstellung der Multimetalloxidkatalysatorformkörper und ihrer Vorläufer

110 kg der Ausgangsmasse 2 wurden dann in einem Schräglagen-Mischer (Typ VIL, Füllvolumen: 200 l, Aachener Misch- und Knetmaschinenfabrik) mit Misch- und Schneidflügel (Drehzahl Mischflügel: 39 U/min, Drehzahl Schneidflügel: 3000 U/min) vorgelegt und 1 min vorgemischt. Innerhalb von 10 min wurde hierzu bei fortgesetztem Mischen über eine Zellenradschleuse die Ausgangsmasse 1 in der für eine Multimetalloxidaktivmasse der Stöchiometrie:

$$[Bi_2W_2O_9 \cdot 2\,WO_3]_{0,40}[Mo_{12}CO_{5,4}Fe_{3,1}Si_{1,5}K_{0,08}O_x]_1$$

erforderlichen Menge innerhalb von 10 min zudosiert. Dann wurde der Mischvorgang weitere 15 min fortgesetzt um eine (zur Erreichung einer hohen Aktivität und Acroleinselektivität erforderliche) intensive und vollständige Homogenisierung (einschließlich des Zerschlagens evtl. vorhandener Agglomerate) der beiden Ausgangsmassen zu erreichen. Bezogen auf die vorgenannte Gesamtmasse wurde innerhalb von weiteren 2 min 1 Gew.-% Graphit TIMREX T44 der Firma Timcal AG untergemischt.

Das resultierende Gemisch wurde dann in einem Kompaktor Typ K200/100 der Fa. Hosokawa Bepex GmbH) mit konkaven, geriffelten Glattwalzen (Spaltweite: 2,8 mm, Walzendrehzahl: 9 UPM, Presskraftsollwert: ca. 75 kN) verdichtet. Über integrierte Schwingsiebe der Fa. Allgaier (Siebweite Überkorn: 1,5 mm, Siebweite Unterkorn: 400 $\mu$m) mit Kugel-Siebhilfen (Durchmesser 22 mm) wurde ein Kompaktat mit einer großteils zwischen 400 $\mu$m und 1,5 mm liegenden Partikelgröße isoliert.

Für die Tablettierung wurden dem Kompaktat in einem Turbulent-Mischer der Firma Drais innerhalb von 2 min weitere 2,5 Gew.-% des Graphits TIMREX T44 der Firma Timcal AG zugemischt.

Anschließend wurde das wie beschrieben erzeugte pulverförmige Haufwerk mit Hilfe eines Korsch PH 865 Rundläufers unter Luftatmosphäre erfindungsgemäß verdichtet (Einfachwerkzeug, 65 Matrizen). Der grundsätzliche Vorrichtungsaufbau war wie in Figur 6. Der Durchmesser der Vordruckrolle betrug 100 mm und der Durchmesser der Hauptdruckrolle betrug 300 mm. Der Abstand zweier auf der Matrixenscheibe einander gegenüberliegender Matrizen betrug 780 mm.

Die verwendeten Matrizen waren Matrizen mit kongruentem doppeltem Kegelstumpf wie in den Figuren 3a, 3b schematisch gezeigt.

Die Länge I des Längsabschnitts I betrug 6,22 mm.

Die Länge II (die Länge II*) des Längsabschnitts II (des Längsabschnitts II*) betrug 8 mm.

Die Umrisslinie des Kreiszylinders I und des Kreiszylinders II betrugen 15,7 mm. Der Durchmesser DD der Deckfläche des Kegelstumpfes KS betrug 5,1 mm. Der Durchmesser DG der Grundfläche des Kegelstumpfes KS betrug 5,0 mm. Die Länge der Umrisslinie des Kreiszylinders Z (des durchgehend kreiszylindrischen Mittelstiftes) betrug 2,5 · $\pi$ mm. Die plane obere Stirnfläche des Mittelstiftes MF schloss mit der planen oberen Matrizenstirnfläche bündig ab.

Es wurden nur ringähnliche Vorläuferformkörper $F^{LII}$ gefertigt, wobei der Endabstand E stets 3 mm betrug. Die obere Stirnfläche des unteren Stempels und die untere Stirnfläche des oberen Stempels waren beide plan gestaltet. Die Bohrachse B stand auf beiden Stirnflächen senkrecht.

Beide Mittelbohrungen, $MB^U$ und $MB^O$ (die letztere stand mit zwei gasdurchlässigen Auslässen in Verbindung (vgl. Figur 4d)), wiesen im Eingangsbereich in die zugehörige Stirnfläche eine kreiszylindrische Geometrie mit identischem Radius auf. Der Kontakt ihrer Innenwände zur äußeren Mantelfläche des Mittelstiftes MF war im möglichen Kontaktbereich aufeinander gleitend.

Die einzelne Matrize war aus einem Werkstoffverbund gefertigt. Dieser bestand auf seiner die Matrizenbohrung berührenden Seite aus dem Hartmetall G10-Ni (6,9 bis 7 mm Wanddicke) mit $R_a$ = 0,1 $\mu$m und auf seiner von der Matrizenbohrung abgewandten Seite aus DIN-Werkzeugstahl 1.2379 (9 mm Wanddicke) mit $R_a$ = 0,8 $\mu$m. Der obere Stempel und der untere Stempel waren aus DIN-Werkstoff 1.2601 gefertigt. Der auf seiner Gesamtlänge kreiszylindrische Mittelstift MF war aus DIN-Werkzeugstahl 1.2343 gefertigt ($R_a$ = 0,4 $\mu$m). $R_a$ der beiden Stirnflächen betrug ebenfalls 0,4 $\mu$m.

Die in den Füllraum eingebrachte Menge an pulverförmigem Haufwerk betrug 129 mg.

Beim Beginn des Verfahrens schloss die untere Stirnfläche des oberen Stempels im Zustand des Ausgangsabstands A mit dem oberen Ende des Längsabschnitts II bündig ab. Mit zunehmender Abnutzung der Innenwand des oberen Teils des Längsabschnitts II der Matrizenbohrung wurden im Zustand des Ausgangsabstands A die Positionen beider Stirnflächen innerhalb des Längsabschnitts II nach unten verschoben.

Die angewandte Vordruckkraft (Presskraft) betrug bei jedem der beiden Stempel 0,3 kN, die angewandte Hauptdruckkraft (Presskraft) betrug bei jedem der beiden Stempel 4,2 kN.

Die Seitendruckfestigkeiten der resultierenden ringähnlichen Multimetalloxid-Vollkatalysatorvorläuferformkörper lagen im Bereich von 21 bis 23 N.

Die Umdrehungsrate des Rundläufers lag bei 35 bis 45 UPM.

Hinsichtlich des Materials von Matrizenscheibenzunge, Matrizenscheibenstirn und Matrizenscheibenkinn gilt das in der Beschreibung Gesagte.

Um Staubfreisetzung zu vermeiden wurde die Tablettiermaschine abgesaugt (300 bis 400 Nm$^3$/h). Die Abluft wurde über einen Filter geführt, der periodisch abgereinigt wurde.

Anschließend wurden die hergestellten ringähnlichen Multimetalloxid-Vollkatalysatorvorläuferformkörper wie in Beispiel 1 der DE-A 100 46 957 (die Schütthöhe in der Zersetzung (Kammern 1 bis 4) belief sich dabei jedoch auf 53 mm bei einer Verweilzeit pro Kammer von 1,23 h und in der Kalzination (Kammern 5 bis 8) belief sie sich auf 153 mm bei einer Verweilzeit von 3,89 h) beschrieben mittels einer Bandkalziniervorrichtung thermisch behandelt; die Kammern besaßen eine Grundfläche (bei einer einheitlichen Kammerlänge von 1,40 m) von 1,29 m$^2$ (Zersetzung) und 1,40 m$^2$ (Kalzination) und wurden von unten durch das grobmaschige Band von 50-210 Nm$^3$/h auf 100 °C (Zersetzung) bzw. 450 °C (Kalzination) vorgeheizte Zuluft durchströmt; zusätzlich wurde die Luft durch rotierende Ventilatoren (900 bis 1450 U/min) umgewälzt. Innerhalb der Kammern war die zeitliche und örtliche Abweichung der Temperatur vom Sollwert (typische Werte für die Zonen 1-8 sind: 140 °C, 190 °C, 220 °C, 265 °C, 380 °C, 425 °C, 460 °C, 460 °C) stets ≤ 2°C. Hinter Kammer 8 schloss sich eine auf 70 °C temperierte 2m lange Kühlzone an. Im Übrigen wurde wie in Beispiel 1 der DE-A 100 46 957 beschrieben verfahren.

Anschließend wurden die gebildeten ringähnlichen Multimetalloxidvollkatalysatorformkörper einer Unterkornsiebung unterworfen. Bei den verwendeten Sieben handelte es sich um Langlochsiebe. Ihre geradlinige Kantenlänge betrug 20 mm und der Abstand der beiden Kanten war 1,8 mm. Bezogen auf das Gewicht des insgesamt zur Siebung aufgegebenen Siebgutes betrug der anfallende Unterkornanteil 0,4 Gew.-%.

Wurde in gleicher Weise wie vorstehend beschrieben verfahren, die Verdichtung des pulverförmigen Haufwerks jedoch mittels einer Matrize durchgeführt, deren Matrizenbohrung ideal kreiszylindrisch war (Durchmesser = 5 mm; der Durchmesser der oberen und der unteren Stirnfläche betrug 4,95 mm), lag der bei der Siebung anfallende Unterkornanteil bei 2,1 Gew.-%.

Die wie beschrieben hergestellten ringähnlichen Multimetalloxidvollkatalysatoren eignen sich z.B. für die heterogen katalysierte partielle Gasphasenoxidation von Propylen zu Acrolein.

Alternativ zur erfindungsgemäßen Verdichtung mit Hilfe eines Korsch PH 865 Rundläufers kann die erfindungsgemäße Verdichtung auch mit einem Kilian Synthesis 700-77 A Rundläufers ausgeführt werden. Als Vordruckkraft (Vordruckpresskraft) können dabei bei jedem der beiden Stempel 0,6 kN und als Hauptdruckkraft (Hauptdruckpresskraft) bei jedem der beiden Stempel 5,0 kN angewendet werden. Weiterhin kann die erfindungsgemäße Verdichtung auch in Stickstoffatmosphäre durchgeführt werden.

III. Herstellung von ringähnlichen Multimetalloxid-Vollkatalysatorvorläuferformkörpern, wobei das aktive Multimetalloxid die Stöchiometrie $Mo_{12}Co_7Fe_{2,94}Bi_{0,6}Si_{1,59}K_{0,08}O_x$ aufwies

Bei 60 °C wurden 213 kg Ammoniumheptamolybdattetrahydrat (81,5 Gew.-% $MoO_3$) in 600 l Wasser gelöst. In diese Lösung wurden unter Aufrechterhaltung der 60 °C 0,97 kg einer 46,8 gew.-%igen wässrigen Kaliumhydroxidlösung von 20 °C eingerührt (dabei wurde eine Lösung A erhalten).

Eine zweite Lösung B wurde hergestellt, indem man unter Rühren zu 333,7 kg einer wässrigen Kobalt-(II)-nitratlösung (12,4 Gew.-% Co) bei 30°C 116,25 kg einer 20°C aufweisenden wässrigen Eisen-(III)-nitratlösung (14,2 Gew.-% Fe) gab. Nach beendeter Zugabe wurde noch 30 min. bei 30°C gerührt. Danach wurden bei 60°C 112,3 kg einer 20°C aufweisenden wässrigen Wismutnitratlösung (11,2 Gew.-% Bi) unter Erhalt der Lösung B eingerührt. Innerhalb von 30 min. wurde bei 60°C die Lösung B in die Lösung A eingerührt. 15 min. nach beendetem Einrühren wurden bei 60°C 19,16 kg Kieselsol (vom Typ Ludox TM-50 der Fa. Grace GmbH in D-67547 Worms) in die erhaltene Maische gegeben. Unter Aufrechterhaltung der 60°C wurde noch 15 min. nachgerührt. Dann wurde die erhaltene Maische im Heißluftgegenstromverfahren sprühgetrocknet (Gaseingangstemperatur: 400 ± 10°C, Gasausgangstemperatur: 140 ± 5°C) wobei ein Sprühpulver erhalten wurde, dessen Glühverlust (3 h bei 600°C unter Luft) 30 % seines Gewichtes betrug. Das Sprühpulver wies einen $d_{50}$ von 20,3 μm sowie einen $d_{10}$ von 3,24 μm und einen $d_{90}$ von 53,6 μm (gemessen bei einem Dispergierdruck von 2 bar absolut) auf.

In das Sprühpulver wurden zusätzlich 1,0 Gew.-% (bezogen auf die Sprühpulvermenge) Graphit Asbury 3160 der Firma Asbury Graphite Mills, Inc. New Jersey 08802, USA eingemischt.

Das dabei resultierende Trockengemisch wurde mittels eines Kompaktors der Fa. Hosokawa Bepex GmbH (D-74211 Leingarten) vom Typ Kompaktor K 200/100 unter den Bedingungen von 2,8 mm Spaltbreite, 1,0 mm Siebweite, 200 μm Siebweite Unterkorn, 35 kN Presssollkraft und 65 bis 70 Upm Schneckendrehzahl durch Vorkompaktieren auf eine im wesentlichen einheitliche Korngröße von 200 μm bis 1 mm vergröbert.

Das Kompaktat wurde anschließend mit, bezogen auf sein Gewicht, weiteren 2 Gew.-% des selben Graphit vermischt und anschließend mit Hilfe eines Kilian Rundläufers vom Typ RX 73, der Fa. Kilian, D-50735 Köln, unter Luftatmosphäre zu ring-ähnlichen Multimetalloxid-Vollkatalysatorvorläuferformkörpern $F^{LII}$ mit nicht gekrümmter (d.h., mit planer) Stirnfläche verdichtet. Der grundsätzliche Vorrichtungsaufbau war wie in Figur 6. Das dabei verwendete Werkzeug (Matrize, Stempel etc.) sowie die Werkstoffe für Matrizenscheibenzunge, Matrizenscheibenstirn und Matrizenscheibenkinn entsprachen jenen aus Beispiel II. Dies gilt auch für die anderen Verdichtungsbedingungen einschließlich des Endabstands E von 3 mm. Die Seitendruckfestigkeit der resultierenden ringähnlichen Multimetalloxidvorläuferformkörper betrug 19 bis 21 N.

Zu ihrer nachfolgenden thermischen Behandlung wurden jeweils 1900 g der ringähnlichen Multimetalloxid-Vollkatalysatorvorläuferformkörper in einer beheizbaren Umluftkammer (0,12 m$^3$ Innenvolumen) aufgeschüttet (2 Nm$^3$ Luft/min.). Anschließend wurde die Temperatur in der Schüttung wie folgt verändert:

- mit 1°C/min. von 25°C auf 160°C erhöht;
- dann 100 min. bei 160°C gehalten;
- danach mit 3°C/min. von 160°C auf 200°C erhöht;
- dann 100 min. bei 200°C gehalten;
- danach mit 2°C/min. von 200°C auf 230°C erhöht;
- dann 100 min. bei 230°C gehalten;
- danach mit 3°C/min. von 230°C auf 270°C erhöht;
- dann 100 min. bei 270°C gehalten;
- danach mit 1°C/min. auf 380°C erhöht;
- dann 4,5 h bei 380°C gehalten;
- danach mit 1°C/min. auf 430°C erhöht;
- dann 4,5 h bei 430°C gehalten;
- danach mit 1°C/min. auf 500°C erhöht;
- dann 9 h bei 500°C gehalten;
- danach innerhalb von 4 h auf 25°C abgekühlt.

Dabei wurden aus den ringähnlichen Multimetalloxid-Vollkatalysatorvorläuferform-körpern ringähnliche Multimetalloxid-Vollkatalysatorformkörper erhalten. Diese eignen sich z.B. als Katalysatoren für die heterogen katalysierte partielle Gasphasenoxidation von Propylen zu Acrolein.

Diese wurden der Unterkornsiebung gemäß Beispiel II unterworfen. Bezogen auf das Gewicht des insgesamt zur Siebung aufgegebenen Siebgutes betrug der anfallende Unterkornanteil 0,6 Gew.-%.

Wurde in gleicher Weise wie vorstehend beschrieben verfahren, die Verdichtung des pulverförmigen Haufwerks jedoch mit einer Matrize durchgeführt, deren Matrizenbohrung ideal kreiszylindrisch war (Durchmesser = 5 mm; der Durchmesser der oberen und der unteren Stirnfläche betrug 4,95 mm) lag der bei der Siebung anfallende Unterkornanteil bei 3,2 Gew.-%.

IV. Herstellung von ringähnlichen Multimetalloxid-Vollkatalysatorvorläuferformkörpern, wobei das aktive Multimetalloxid die Stöchiometrie $Mo_{12}P_{1,5}V_{0,6}C_{0,6}Cu_{0,5}Sb_1S_{0,04}O_x$ aufwies

In 619 l auf 45°C temperiertes Wasser in einem wassertemperierten Doppelmantel-behälter wurden unter Rühren (70 Umdrehungen pro Minute (UPM)) 537,5 kg Ammoniumheptamolybdattetrahydrat $((NH_4)_6Mo_7O_{24} \cdot 4\ H_2O$ (81 Gew.-% $MoO_3$, 8 Gew.-% $NH_3$, $\leq$ 50 Gew.-ppm Na und $\leq$ 100 Gew.-ppm K) eindosiert. Die Temperatur der Lösung sank hierbei auf 37°C ab. Um ein sicheres Auflösen des Ammoniumheptamolybdats zu gewährleisten, wurde nach Ende der Zudosierung noch 15 Minuten nachgerührt, wobei die Temperatur von 37°C beibehalten wurde. Unter weiterem Rühren wurden bei derselben Temperatur innerhalb von 3 Minuten 17,82 kg Ammoniummetavanadat ($NH_4VO_3$, 77 Gew.-% $V_2O_5$, 14,5 Gew.-% $NH_3$, $\leq$ 150 Gew.-ppm Na und $\leq$ 500 Gew.-ppm K) zudosiert. Es wurde 2 Minuten nachgerührt. Dann wurde innerhalb einer Minute eine in einem separaten Lösebehälter hergestellte, farblose, klare, 60°C warme Lösung von 49,6 kg Cäsiumnitrat ($CsNO_3$ mit 72 Gew.-% $Cs_2O$ und $\leq$ 50 Gew.-ppm Na, $\leq$ 100 Gew.-ppm K, $\leq$ 10 Gew.-ppm Al sowie $\leq$ 20 Gew.-ppm Fe) in 106 l Wasser eingerührt. Hierbei stieg die Temperatur der resultierenden Suspension auf 39°C. Nach einminütigem Nachrühren wurden innerhalb einer weiteren Minute unter fortgesetztem Rühren 31,66 l 75 gew.-%ige Phosphorsäure (Dichte bei 25°C und 1 atm: 1,57 g/ml, Viskosität bei 25°C und 1 atm: 0,147 cm$^2$/S) zudosiert. Aufgrund der exothermen Reaktion stieg die Temperatur hierbei auf 42°C. Erneut wurde 1 Minute nachgerührt. Dann wurden innerhalb einer Minute 1,34 kg Ammoniumsulfat $((NH_4)_2SO_4$ (> 99 Gew.-%)) eingerührt und 1 weitere Minute nachgerührt. Unter fortgesetztem Rühren bei identischer Temperatur wurden innerhalb von 3 Minuten 37,04 kg Antimontrioxid ($Sb_2O_3$, Partikeldurchmesser $d_{50}$ = ca. 2μm, Kristallstruktur laut XRD: > 75 % Senarmontit, < 25 % Valentinit, Reinheit: > 99,3 Gew.-%, $\leq$ 0,3 Gew.-% $As_2O_3$, $\leq$ 0,3 Gew.-% PbO und $\leq$ 300 Gew.-ppm FeO) zugegeben (käuflich erhältlich als Triox White, Code No. 639000 der Fa. Antraco, D-10407 Berlin). Nun wurde die Rührerdrehzahl von 70 auf 50 UPM zurückgenommen. Anschließend wurde die gerührte Suspension mittels Dampf im Doppelmantel innerhalb von 30 Minuten linear auf 95°C aufgeheizt. Bei dieser Temperatur und 50 UPM wurden innerhalb von 4 Minuten 51,64 kg Kupfernitratlösung (wässrige $Cu(NO_3)_2$-Lösung mit 15,6 Gew.-% Cu) zugegeben. Nach 56-minütigem Nachrühren bei 95 °C wurde die Rührgeschwindigkeit weiter von 50 auf 35 UPM zurückgenommen. Anschließend wurde die gesamte Suspension innerhalb von 4 Minuten in einen mit Stickstoff überlagerten, auf 85 °C temperierten und mit 35 UPM gerührten Sprühturmvorlagenbehälter abgelassen und es wurde mit 20 l Wasser (25°C) nachgespült. Aus diesem heraus wurde die Suspension in einem Drehscheibensprühturm vom Typ FS-15 der Firma Niro im Heißluftgleichstrom sprühgetrocknet (Gaseintrittstemperatur: 285 ± 10°C, Gasaustrittstemperatur: 110 ± 5°C, Scheibendrehzahl: 18000 U/min, Durchsatz: 270 kg/h), wobei das resultierende Sprühpulver einen Glühverlust (1 h bei 500 °C in Luft) von 17,2 Gew.-% und einen $d_{50}$ von 35,9 $\mu$m aufwies ($d_{10}$ = 14,3 $\mu$m, $d_{90}$ = 65,6 $\mu$m, gemessen bei einem Dispergierdruck von 2 bar absolut) aufwies.

Das Sprühpulver wurde mit 1,5 Gew.-% des Graphits Timrex 44 der Firma Timcal homogen vermischt und kompaktiert (Kompaktor der Fa. Hosokawa Bepex GmbH, D-74211 Leingarten, Typ K200/100 mit konkaven, geriffelten Glattwalzen, Spaltweite: 2,8 mm, Siebweite: 1,25 mm, Siebweite Unterkorn: 400 $\mu$m, Schneckendrehzahl: 65 bis 70 UPM). Für die Tablettierung wurden dem Kompaktat weitere 1 Gew.-% desselben Graphits zugemischt.

Anschließend wurde das wie beschrieben erzeugte pulverförmige Haufwerk mit Hilfe eines Korsch PH 865 Rundläufers unter Luftatmosphäre erfindungsgemäß verdichtet (Einfachwerkzeug, 65 Matrizen). Der grundsätzliche Vorrichtungsaufbau war wie in Figur 6. Der Durchmesser der Vordruckrolle betrug 100 mm und der Durchmesser der Hauptdruckrolle betrug 300 mm. Der Abstand zweier auf der Matrizenscheibe einander gegenüber liegender Matrizen betrug 780 mm.

Die verwendeten Matrizen waren Matrizen mit kongruentem doppeltem Kegelstumpf wie in den Figuren 3a, 3b schematisch gezeigt. Die Länge I des Längsabschnitts I betrug 2,2 mm. Die Länge II (die Länge II*) des Längsabschnitts II (des Längsabschnitts II*) betrug 10 mm.

Die Umrisslinie des Kreiszylinders I und des Kreiszylinders II betrugen 22 mm. Der Durchmesser DD der Deckfläche des Kegelstumpfes KS betrug 7,1 mm. Der Durchmesser DG der Grundfläche des Kegelstumpfes KS betrug 7,0 mm. Die Länge der Umrisslinie des Kreiszylinders Z (des durchgehend kreiszylindrischen Mittelstiftes) betrug 3,0 · $\pi$ mm. Die plane obere Stirnfläche des Mittelstiftes MF schloss mit der planen oberen Matrizenstirnfläche bündig ab.

Es wurden nur ringähnliche Vorläuferformkörper $F^{LII}$ gefertigt, wobei der Endabstand E stets 7 mm betrug. Die obere Stirnfläche des unteren Stempels und die untere Stirnfläche des oberen Stempels waren beide plan gestaltet. Die Bohrachse B stand auf beiden Stirnflächen senkrecht.

Beide Mittelbohrungen, $MB^U$ und $MB^O$ (die letztere stand mit zwei gasdurchlässigen Auslässen in Verbindung (vgl. Figur 4d)), wiesen im Eingangsbereich in die zugehörige Stirnfläche eine kreiszylindrische Geometrie mit identischem Radius auf. Der Kontakt ihrer Innenwände zur äußeren Mantelfläche des Mittelstiftes MF war im möglichen Kontaktbereich aufeinander gleitend.

Die einzelne Matrize war aus einem Werkstoffverbund gefertigt. Dieser bestand auf seiner die Matrizenbohrung berührenden Seite aus dem Hartmetall G10-Ni (2,5 bis 2,6 mm Wanddicke) mit $R_a$ = 0,1 $\mu$m und auf seiner von der Matrizenbohrung abgewandten Seite aus DIN-Werkzeugstahl 1.2379 (9 mm Wanddicke) mit $R_a$ = 0,8 $\mu$m. Der obere Stempel und der untere Stempel waren aus DIN-Werkstoff 1.2601 gefertigt. Der auf seiner Gesamtlänge kreiszylindrische Mittelstift MF war aus DIN-Werkzeugstahl 1.2343 gefertigt ($R_a$ = 0,4 $\mu$m). $R_a$ der beiden Stirnflächen betrug ebenfalls 0,4 $\mu$m.

Die in den Füllraum eingebrachte Menge an pulverförmigem Haufwerk betrug 576 mg.

Beim Beginn des Verfahrens schloss die untere Stirnfläche des oberen Stempels im Zustand des Ausgangsabstands A mit dem oberen Ende des Längsabschnitts II bündig ab. Mit zunehmender Abnutzung der Innenwand des oberen Teils des Längsabschnitts II der Matrizenbohrung wurden im Zustand des Ausgangsabstands A die Positionen beider Stirnflächen innerhalb des Längsabschnitts II nach unter verschoben. Die bei jedem der beiden Stempel angewandte Vordruckkraft (Presskraft) betrug 0,3 kN, die bei jedem der beiden Stempel angewandte Hauptdruckkraft (Presskraft) betrug 3,5 kN.

Die Seitendruckfestigkeiten der resultierenden ringähnlichen Multimetalloxid-Vollkatalysatorvorläuferformkörper lagen im Bereich von 33 bis 37 N. Die Umdrehungszahl des Rundläufers lag bei 20 bis 25 UPM. Hinsichtlich des Materials von Matrizenscheibenzunge, Matrizenscheibenstirn und Matrizenscheibenkinn gilt das in der Beschreibung Gesagte.

Anschließend wurden 8 kg der ringähnlichen Multimetalloxid-Vollkatalysatorvorläufer-formkörper in einem Drahtbehälter der Grundfläche 33,0 cm x 49,5 cm gleichmäßig verteilt, wobei sich eine Schütthöhe von 4 cm ergab. Der Drahtbehälter wurde in einem Kammerofen (Fa. Elino Industrie-Ofenbau, Carl Hanf GmbH & Co, D-52355 Düren, Typ KA-040/006-08 EW.OH, Abmessungen: Länge = 57 cm, Breite = 57 cm, Höhe = 80 cm) so angeordnet, dass die Schüttung der Tabletten gleichmäßig durchströmbar war. Es wurden 2 Nm$^3$/h Frischluft zugeführt und die Luftumwälzung im Ofen so eingestellt, dass die Schüttung mit einer Geschwindigkeit von 0,9 m/s (bestimmt mittels Aerometer, Fa. Testo, Typ 445) durchströmt wurde. Der Ofen wurde nun mit der folgenden Temperaturrampe auf 380 °C aufgeheizt: innerhalb von 40 min auf 180 °C aufheizen, 30 min halten, innerhalb von 10 min auf 220 °C aufheizen, 30 min halten, innerhalb von 13 min auf 270 °C aufheizen, 30 min halten, innerhalb von 25 min auf 340 °C und dann innerhalb von 40 min auf 380 °C aufheizen. Diese Temperatur wurde dann 390 min gehalten. Währenddessen wurde der NH$_3$-Gehalt in der abgesaugten Atmosphäre der thermischen Behandlung kontinuierlich durch FTIR-Spektroskopie überwacht (Spektrometer der Fa. Nicolet, Typ "Impact", IR-Edelstahlzelle mit CaF$_2$-Fenster, 10 cm Schichtdicke, Temperierung auf 120 °C, Bestimmung der Konzentration anhand der Intensität der Bande bei 3.333 cm$^{-1}$). Der NH$_3$-Gehalt blieb während der gesamten thermischen Behandlung $\leq$ 2,4 Vol.-%. Dieser Maximalwert wurde bei 220 °C erreicht.

Die erhaltenen ringähnlichen Multimetalloxid-Vollkatalysatorformkörper eignen sich z.B. als Katalysatoren für die heterogen katalysierte partielle Gasphasenoxidation von Methacrolein zu Methacrylsäure.

Danach wurden die gebildeten ringähnlichen Multimetalloxidvollkatalysatorformkörper einer Unterkornsiebung unterworfen. Bei den verwendeten Sieben handelte es sich um Langlochsiebe. Ihre geradlinige Kantenlänge betrug 20 mm und der Abstand der beiden Kanten war 6 mm. Bezogen auf das Gewicht des insgesamt zur Siebung aufgegebenen Siebgutes betrug der anfallende Unterkornanteil 14 Gew.-%.

Wurde in gleicher Weise wie vorstehend beschrieben verfahren, die Verdichtung des pulverförmigen Haufwerks jedoch mittels einer Matrize durchgeführt, deren Matrizenbohrung ideal kreiszylindrisch war (Durchmesser = 7 mm; der Durchmesser der beiden Stirnflächen betrug 6,95 mm), lag der bei der Siebung anfallende Unterkornanteil bei 21,7 Gew.-%.

V. Herstellung von ringähnlichen Multimetalloxid-Vollkatalysatorvorläuferformkörpern wobei das aktive Multimetalloxid Vanadium, Phosphor, Eisen und Sauerstoff enthält

In einem mit Stickstoff inertisierten, über Druckwasser außenbeheizbaren 8 $m^3$-Stahl/Email-Rührkessel mit Strombrechern wurden 4602 kg iso-Butanol vorgelegt. Nach Inbetriebnahme des dreistufigen Impellerrührers wurde das iso-Butanol unter Rückfluss auf 90°C aufgeheizt. Bei dieser Temperatur wurde nun über eine Förderschnecke mit der Zugabe von 690 kg Vanadiumpentoxid begonnen. Nachdem nach ca. 20 Minuten etwa 2/3 der gewünschten Menge an Vanadiumpentoxid zugegeben waren, wurde bei weiterer Zugabe an Vanadiumpentoxid mit der Einpumpung von 805 kg einer eine Temperatur von 50 °C aufweisenden Polyphosphorsäure mit einem $P_2O_5$-Gehalt von 76 Gew.-% (entspricht 105 Gew.-% $H_3PO_4$) begonnen. Nach beendeter Zugabe der Phosphorsäure wurde das Reaktionsgemisch unter Rückfluss auf etwa 100 bis 108°C erhitzt und unter diesen Bedingungen 14 Stunden belassen. Im Anschluss daran wurde die heiße Suspension innerhalb von 70-80 Minuten auf 60°C abgekühlt und 22,7 kg Fe-(III)Phosphat (29,9 Gew.-% Fe) zugegeben. Nach erneutem Hochheizen innerhalb von 70 Minuten auf Rückfluss siedete die Suspension unter Rückfluss für eine weitere Stunde. Anschließend wurde die Suspension in eine zuvor mit Stickstoff inertisierte und beheizte Druckfilternutsche abgelassen und bei einer Temperatur von etwa 100 °C bei einem Druck oberhalb der Filternutsche von bis zu 0,35 MPa abs abfiltriert. Der Nutschkuchen wurde durch stetiges Einleiten von Stickstoff bei 100 °C und unter Rühren mit einem mittig angeordneten, in der Höhe verstellbaren Rührer innerhalb von etwa einer Stunde trockengeblasen. Nach dem Trockenblasen wurde auf ca. 155 °C aufgeheizt und auf einen Druck von 15 kPa abs (150 mbar abs) evakuiert. Die Trocknung wurde bis zu einem Restiso-butanolgehalt von < 2 Gew.-% in der getrockneten Katalysator-Vorläufermasse durchgeführt.

Das Fe/V-Verhältnis betrug 0,016.

Anschließend wurde das getrocknete Pulver 2 Stunden unter Luft in einem Drehrohr mit einer Länge von 6,5 m, einem Innendurchmesser von 0,9 m und innenliegenden spiralförmigen Wendeln (zu Durchmischungszwecken) behandelt. Die Drehzahl des Drehrohres betrug 0,4 U/min. Das Pulver wurde in einer Menge von 60 kg/h in das Drehrohr gefördert. Die Luftzufuhr betrug 100 $m^3$/h. Die direkt an der Außenseite des von außen beheizten Drehrohrs gemessenen Temperaturen der fünf gleichlangen Heizzonen betrugen in Richtung "vom Ausgang des Pulvers" zum "Eingang des Pulvers" ins Drehrohr 250°C, 300°C, 345°C, 345°C und 345°C.

Die dem Drehrohr entnommene Vorläufermasse wurde mit 1 Gew.-% des Graphits Timrex T44 der Firma Timcal AG innig und homogen vermischt. Das resultierende Gemisch wurde dann kompaktiert und anschließend mit weiteren 2 Gew.-% desselben Graphits vermischt.

Anschließend wurde das wie beschrieben erzeugte pulverförmige Haufwerk mit Hilfe eines Korsch PH 865 Rundläufers unter Stickstoffatmosphäre erfindungsgemäß verdichtet (Einfachwerkzeug, 65 Matrizen). Der grundsätzliche Vorrichtungsaufbau war wie in Figur 6. Der Durchmesser der Vordruckrolle betrug 100 mm und der Durchmesser der Hauptdruckrolle betrug 300 mm. Der Abstand zweier auf der Matrizenscheibe einander gegenüber liegender Matrizen betrug 780 mm.

Die verwendeten Matrizen waren Matrizen mit kongruentem doppeltem Kegelstumpf wie in den Figuren 3a, 3b schematisch gezeigt. Die Länge I des Längsabschnitts I betrug 6,2 mm. Die Länge II (die Länge II*) des Längsabschnitts II (des Längsabschnitts II*) betrug 8 mm.

Die Umrisslinie des Kreiszylinders I und des Kreiszylinders II betrugen 17,3 mm. Der Durchmesser DD der Deckfläche des Kegelstumpfes KS betrug 5,5 mm. Der Durchmesser DG der Grundfläche des Kegelstumpfes KS betrug 5,6 mm. Die Länge der Umrisslinie des Kreiszylinders Z (des durchgehend kreiszylindrischen Mittelstiftes) betrug 3,0 · $\pi$ mm. Die plane obere Stirnfläche des Mittelstiftes MF schloss mit der planen oberen Matrizenstirnfläche bündig ab.

Es wurden nur ringähnliche Formkörper $F^{LII}$ gefertigt, wobei der Endabstand E stets 3,2 mm betrug. Die obere Stirnfläche des unteren Stempels und die untere Stirnfläche des oberen Stempels waren beide plan gestaltet. Die Bohrachse B stand auf beiden Stirnflächen senkrecht.

Beide Mittelbohrungen, $MB^U$ und $MB^O$ (die letztere stand mit zwei gasdurchlässigen Auslässen in Verbindung (vgl. Figur 4d)), wiesen im Eingangsbereich in die zugehörige Stirnfläche eine kreiszylindrische Geometrie mit identischem Radius auf. Der Kontakt ihrer Innenwände zur äußeren Mantelfläche des Mittelstiftes MF war im möglichen Kontaktbereich aufeinander gleitend.

Die einzelne Matrize war aus einem Werkstoffverbund gefertigt. Dieser bestand auf seiner die Matrizenbohrung berührenden Seite aus dem Hartmetall G10-Ni (3,2-3,3 mm Wanddicke) mit $R_a$ = 0,1 μm und auf seiner von der Matrizenbohrung abgewandten Seite aus DIN-Werkzeugstahl 1.2379 (9,1 mm Wanddicke) mit $R_a$ = 0,8 μm. Der obere Stempel und der untere Stempel waren aus DIN-Werkstoff 1.2601 gefertigt. Der auf seiner Gesamtlänge kreiszylindrische Mittelstift MF war aus DIN-Werkzeugstahl 1.2343 gefertigt ($R_a$ = 0,4 μm). $R_a$ der beiden Stirnflächen betrug ebenfalls 0,4 μm.

Die in den Füllraum eingebrachte Menge an pulverförmigem Haufwerk betrug 90 mg.

Beim Beginn des Verfahrens schloss die untere Stirnfläche des oberen Stempels im Zustand des Ausgangsabstands A mit dem oberen Ende des Längsabschnitts II bündig ab. Mit zunehmender Abnutzung der Innenwand des oberen Teils des Längsabschnitts II der Matrizenbohrung wurden im Zustand des Ausgangsabstands A die Positionen beider Stirnflächen innerhalb des Längsabschnitts II nach unter verschoben. Die auf jeden der beiden Stempel angewandte Vordrucckraft (Presskraft) betrug 0,3 kN, die auf jeden der beiden Stempel angewandte Hauptdruckkraft (Presskraft) betrug 4,2 kN.

Die Seitendruckfestigkeiten der resultierenden ringähnlichen Multimetalloxid-Vollkatalysatorvorläuferformkörper lagen im Bereich von 9 bis 11 N. Die Umdrehungszahl des Rundläufers lag bei 20 bis 30 UPM. Hinsichtlich des Materials von Matrizenscheibenzunge, Matrizenscheibenstirn und Matrizenscheibenkinn gilt das in der Beschreibung Gesagte.

Aus den erhaltenen ringähnlichen Multimetalloxid-Vollkatalysatorvorläuferformkörpern wurden durch wie in der WO 03/78059, Seite 39 unter Beispiel 9 beschriebene thermische Behandlung die resultierenden ringähnlichen Multimetalloxidvollkatalysatoren erzeugt. Diese eignen sich z.B. als Katalysatoren für die heterogen katalysierte partielle Gasphasenoxidation von n-Butan zu Maleinsäureanhydrid.

Danach wurden die gebildeten ringähnlichen Multimetalloxidvollkatalysatorformkörper einer Unterkornsiebung unterworfen. Bei den verwendeten Sieben handelte es sich um Langlochsiebe. Ihre geradlinige Kantenlänge betrug 4 mm und der Abstand der beiden Kanten war 4 mm. Bezogen auf das Gewicht des insgesamt zur Siebung aufgegebenen Siebgutes betrug der anfallende Unterkornanteil 0,3 Gew.-%.

Wurde in gleicher Weise wie vorstehend beschrieben verfahren, die Verdichtung des pulverförmigen Haufwerks jedoch mittels einer Matrize durchgeführt, deren Matrizenbohrung ideal kreiszylindrisch war (Durchmesser = 5,5 mm; der Durchmesser der beiden Stirnflächen betrug 5,45 mm), lag der bei der Siebung anfallende Unterkornanteil bei 1,2 Gew.-%.


**Patentansprüche**

1.  Verfahren zur Herstellung eines ringähnlichen oxidischen Formkörpers, umfassend das mechanische Verdichten eines in den Füllraum einer Matrize eingebrachten pulverförmigen Haufwerks aus Bestandteilen, die wenigstens eine Metallverbindung, die durch thermische Behandlung bei einer Temperatur ≥ 100 °C in ein Metalloxid überführbar ist, oder wenigstens ein Metalloxid, oder wenigstens ein Metalloxid und wenigstens eine solche Metallverbindung umfassen, zu einem ringähnlichen Vorläuferformkörper, bei dem sich der Füllraum in einer mit einer vertikalen Bohrachse B von oben nach unten durch das Matrizenmaterial hindurchgeführten Matrizenbohrung befindet und durch

    - die Innenwand der Matrizenbohrung,
    - die obere Stirnfläche eines von unten entlang der Bohrachse B in die Matrizenbohrung hub- und senkbeweglich eingeführten unteren Stempels, auf der das in den Füllraum eingebrachte pulverförmige Haufwerk aufliegt,
    - die längs der Bohrachse B in einem axialen Ausgangsabstand A oberhalb der oberen Stirnfläche des unteren Stempels befindliche untere Stirnfläche eines entlang der Bohrachse B hub- und senkbeweglich angebrachten oberen Stempels, dessen untere Stirnfläche das in den Füllraum eingebrachte pulverförmige Haufwerk von oben berührt, und
    - die Mantelfläche eines aus der geometrischen Mitte der oberen Stirnfläche des unteren Stempels heraus entlang der Bohrachse B in der Matrizenbohrung von unten nach oben geführten Mittelstiftes MF, der wenigstens bis zur geometrischen Mitte der unteren Stirnfläche des oberen Stempels hinaufreicht, begrenzt wird,

    indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt, wobei

    - die geometrische Form der Mantelfläche des unteren Stempels derjenigen der Mantelfläche eines Kreiszylinders I entspricht;

- die geometrische Form der Mantelfläche des oberen Stempels derjenigen der Mantelfläche eines Kreiszylinders II entspricht;

- in der geometrischen Mitte der oberen Stirnfläche des unteren Stempels eine von oben nach unten durch den unteren Stempel hindurchgeführte Mittelbohrung $MB^U$ (37) ausgebildet ist;

- im Ausgangsabstand A der beiden Stirnflächen der Mittelstift MF (18) von unten durch die Mittelbohrung $MB^U$ (37) hindurch bis wenigstens zur geometrischen Mitte der unteren Stirnfläche des oberen Stempels hinaufragt;

- der Mittelstift MF (18) von unten nach oben die geometrische Form eines Kreiszylinders Z mit einer kreiszylindrischen Mantelfläche MZ aufweist;

- die Länge der Umrisslinie des Kreiszylinders Z kleiner als die Länge der Umrisslinie des Kreiszylinders I sowie kleiner als die Länge der Umrisslinie des Kreiszylinders II ist;

- die Position des Mittelstiftes MF (18) und die Position der Matrize einschließlich der Matrizenbohrung längs der Bohrachse B während des Verfahrens relativ zueinander fixiert sind;

- in der geometrischen Mitte der unteren Stirnfläche des oberen Stempels eine in den oberen Stempel hineinführende und mit wenigstens einem Auslass aus dem oberen Stempel in Verbindung stehende Mittelbohrung $MB^O$ (35) ausgebildet ist, die den Mittelstift MF (18) bei der Verringerung des Ausgangsabstands A auf den Endabstand E im erforderlichen Umfang aufzunehmen vermag und in die der Mittelstift MF (18) bereits im Ausgangsabstand A hineinragen kann;

- die Symmetrieachsen der Matrizenbohrung, des Kreiszylinders I, des Kreiszylinders II, der Mittelbohrung $MB^O$ (35), des Mittelstiftes MF (18) und der Mittelbohrung $MB^U$ (37) auf einer gemeinsamen, durch die Matrizenbohrung vertikal verlaufenden geraden Linie L liegen;

- die Matrizenbohrung längs ihrer Bohrachse einen Längsabschnitt I (31) aufweist, auf dessen Länge I die geometrische Form der Innenwand der Matrizenbohrung derjenigen der Mantelfläche eines Kreiszylinders KZ entspricht, und an dessen oberem Ende sich unmittelbar ein nach oben gerichteter Längsabschnitt II (32) der Matrizenbohrung anschließt, der die Länge II aufweist;

- die Ausmaße des Längsabschnitts I (31) der Matrizenbohrung und des Kreiszylinders I so beschaffen sind, dass der untere Stempel während des Verfahrens stets jeweils wenigstens auf einer Teillänge des Längsabschnitts I (31) mit seiner Mantelfläche auf der Innenwand der Matrizenbohrung gleitend in die Matrizenbohrung geführt ist; und

- die Ausmaße der Mittelbohrung $MB^U$ (37) und des Kreiszylinders Z so beschaffen sind, dass der untere Stempel während des Verfahrens stets wenigstens im Bereich des Eingangs seiner Mittelbohrung $MB^U$ (37) in seine obere Stirnfläche mit der Innenwand der Mittelbohrung $MB^U$ (37) auf der kreiszylindrischen Mantelfläche MZ des Mittelstiftes MF (18) gleitend in die Matrizenbohrung geführt ist; und

- nach beendeter Verdichtung der obere Stempel vom gebildeten ringähnlichen Vorläuferformkörper abgehoben und der ringähnliche Vorläuferformkörper durch Anheben des unteren Stempels aus der Matrizenbohrung entfernt wird,

**dadurch gekennzeichnet, dass** ein sich daran anschließendes Verfahren der thermischen Behandlung des ringähnlichen Vorläuferformkörpers bei einer Temperatur $\geq$ 100 °C, bei dem sich wenigstens eine Teilmenge seiner Bestandteile unter Ausbildung wenigstens einer gasförmigen Verbindung zersetzt und/oder chemisch umsetzt und der ringähnliche oxidische Formkörper sich ausbildet, und

dass die geometrische Form der Innenwand der Matrizenbohrung auf der Länge II des Längsabschnitts II (32) von unten nach oben derjenigen der Mantelfläche eines sich von unten nach oben erweiternden Kegelstumpfes KS entspricht, dessen Querschnittsfläche an seinem unteren Ende der Querschnittsfläche des Kreiszylinders KZ an dessen oberem Ende entspricht, mit der Maßgabe, dass beim Erreichen des Endabstands E die untere Stirnfläche des oberen Stempels sich im Längsabschnitt II (32) und die obere Stirnfläche des unteren Stempels sich nicht unterhalb des Längsabschnitts I (31) befindet, so dass sich der durch das mechanische Verdichten des pulverförmigen Haufwerks zwischen den beiden Stirnflächen ausgebildete ringähnliche Vorläuferformkörper beim Erreichen des Endabstands E wenigstens teilweise im Längsabschnitt II (32) befindet.

2. Verfahren nach Anspruch 1, **dadurch gekennzeichnet, dass** sich beim Erreichen des Endabstands E zwischen der oberen Stirnfläche des unteren Stempels und der unteren Stirnfläche des oberen Stempels wenigstens 20 % der Abstandsstrecke zwischen den beiden Stirnflächen im Längsabschnitt II (32) befinden

3. Verfahren nach Anspruch 1, **dadurch gekennzeichnet, dass** sich beim Erreichen des Endabstands E zwischen der oberen Stirnfläche des unteren Stempels und der unteren Stirnfläche des oberen Stempels der gesamte durch das mechanische Verdichten des pulverförmigen Haufwerks zwischen den beiden Stirnflächen ausgebildete ringähnliche Vorläuferformkörper im Längsabschnitt II (32) befindet.

4. Verfahren nach einem der Ansprüche 1 bis 3, **dadurch gekennzeichnet, dass** die Umrisslinie des Kreiszylinders II länger oder gleich lang wie die Umrisslinie des Kreiszylinders I ist.

5. Verfahren nach einem der Ansprüche 1 bis 4, **dadurch gekennzeichnet, dass** die obere Stirnfläche des unteren Stempels und die untere Stirnfläche des oberen Stempels in zueinander parallelen Ebenen liegen, auf denen die Bohrachse B senkrecht steht.

6. Verfahren nach einem der Ansprüche 1 bis 5, **dadurch gekennzeichnet, dass** der Endabstand E 2 bis 10 mm beträgt.

7. Verfahren nach einem der Ansprüche 1 bis 6, **dadurch gekennzeichnet, dass** der Quotient Q aus der Länge der Umrisslinie des Kreiszylinders Z als Zähler und der Umrisslinie des Kreiszylinders I als Nenner 0,3 bis 0,7 beträgt.

8. Verfahren nach einem der Ansprüche 1 bis 7, **dadurch gekennzeichnet, dass** die Differenz, gebildet durch Subtraktion des Radius der Umrisslinie des Kreiszylinders Z vom Radius der Umrisslinie des Kreiszylinders I 1 bis 3 mm beträgt.

9. Verfahren nach einem der Ansprüche 1 bis 8, **dadurch gekennzeichnet, dass** der Durchmesser der Umrisslinie des Kreiszylinders I 2 bis 10 mm beträgt.

10. Verfahren nach einem der Ansprüche 1 bis 9, **dadurch gekennzeichnet, dass** der Kegelstumpf KS so beschaffen ist, dass zwischen dem Durchmesser DD der Deckfläche, dem Durchmesser DG der Grundfläche und der Höhe H des Kegelstumpfs KS die nachfolgende Beziehung erfüllt ist:

$$0,003 \cdot H \leq DG - DD \leq 0,050 \cdot H.$$

11. Verfahren nach einem der Ansprüche 1 bis 10, **dadurch gekennzeichnet, dass** sowohl die obere Stirnfläche des unteren Stempels als auch die untere Stirnfläche des oberen Stempels die geometrische Form eines Kreisrings aufweist.

12. Verfahren nach einem der Ansprüche 1 bis 10, **dadurch gekennzeichnet, dass** die Matrizenbohrung der Matrize so beschaffen ist, dass sich an ihren Längsabschnitt I (31) nicht nur an dessen oberem Ende unmittelbar ein nach oben gerichteter Längsabschnitt II (32), sondern auch an dessen unterem Ende unmittelbar ein nach unten gerichteter Längsabschnitt II* (33) der Länge II* anschließt, und die geometrische Form der Innenwand der Matrizenbohrung auf der Länge II* des Längsabschnitts II* (33) der Mantelfläche eines Kegelstumpfes KS* entspricht, dessen Querschnittsfläche an seinem oberen Ende der Querschnittsfläche des Kreiszylinders KZ an dessen unterem Ende entspricht.

13. Verfahren nach einem der Ansprüche 1 bis 12, **dadurch gekennzeichnet, dass** zwischen der Höhe H des Kegelstumpfs KS und dem Endabstand E nachfolgende Beziehung erfüllt ist:

$$4 \cdot \text{Endabstand } E \geq H \geq 1 \cdot \text{Endabstand } E.$$

14. Verfahren nach einem der Ansprüche 1 bis 13, **dadurch gekennzeichnet, dass** die Länge des Längsabschnitts I (31) nicht mehr als das Dreifache und nicht weniger als das 0,1-fache der Länge des Längsabschnitts II (32) beträgt.

15. Verfahren nach einem der Ansprüche 1 bis 14, **dadurch gekennzeichnet, dass** wenigstens der Eingang in die Mittelbohrung $MB^O$ (35) kreiszylindrisch so gestaltet ist, dass die Mantelfläche des Kreiszylinders Z bei dessen Aufnahme in die Mittelbohrung $MB^O$ (35) wenigstens im Eingangsbereich derselben auf deren Innenwand gleitet.

16. Verfahren nach einem der Ansprüche 1 bis 15, **dadurch gekennzeichnet, dass** das mechanische Verdichten aus einer Vorverdichtung und aus einer dieser nachfolgenden Hauptverdichtung besteht, wobei der axiale Ausgangsabstand A im Rahmen der Vorverdichtung zunächst auf einen vorläufigen Endabstand $E^V$ verringert wird, und im Rahmen der Hauptverdichtung der vorläufige Endabstand $E^V$ auf den Endabstand E verringert wird.

17. Verfahren nach einem der Ansprüche 1 bis 16, **dadurch gekennzeichnet, dass** das pulverförmige Haufwerk wenigstens ein Metalloxid, Metallhydroxid, Metallcarbonat, Metallhydrogencarbonat, Metallhydrogenphosphat und/oder Metallnitrat umfasst

**18.** Verfahren nach einem der Ansprüche 1 bis 17, **dadurch gekennzeichnet, dass** die Matrize aus einem Werkstoffverbund gefertigt ist, der auf seiner die Matrizenbohrung berührenden Seite aus einem Hartmetall und auf seiner von der Matrizenbohrung abgewandten Seite aus einem Werkzeugstahl besteht, der folgende Elementzusammensetzung aufweist:

| | |
|---|---|
| 1,50 bis 1,80 Gew.-% | C, |
| 0,10 bis 0,40 Gew.-% | Si, |
| 0,10 bis 0,50 Gew.-% | Mn, |
| $\geq$ 0 bis 0,05 Gew.-% | P, |
| $\geq$ 0 bis 0,05 Gew.-% | S, |
| 10 bis 13 Gew.-% | Cr, |
| 0,50 bis 0,80 Gew.-% | Mo, |
| 0,10 bis 1,10 Gew.-% | V, |
| $\geq$ 0 bis 0,60 Gew.-% | W, und |
| $\geq$ 0 bis 0,10 Gew.-% | eines oder mehrere seltene Erdmetalle, und im übrigen Fe und herstellungsbedingte Verunreigungen. |

ni-

**19.** Verfahren nach Anspruch 18, **dadurch gekennzeichnet, dass** das Hartmetall zu $\geq$ 90 Gew.-% aus Wolframcarbid und zu wenigstens 5 Gew.-% aus Nickel oder aus Nickel und Chrom besteht.

**20.** Verfahren nach einem der Ansprüche 1 bis 19, **dadurch gekennzeichnet, dass** das pulverförmige Haufwerk Salpetersäure, ein Ammoniumsalz und/oder ein Nitratsalz enthält.

**21.** Verfahren nach einem der Ansprüche 1 bis 20, **dadurch gekennzeichnet, dass** im Endabstand E von beiden Stempeln ein Pressdruck ausgeübt wird, der im Bereich 50 bis 5000 kg/cm$^2$ liegt.

**22.** Verfahren nach einem der Ansprüche 1 bis 21, **dadurch gekennzeichnet, dass** das Verfahren der thermischen Behandlung der hergestellten ringähnlichen Vorläuferformkörper bei einer Temperatur $\geq$ 200°C erfolgt.

**23.** Verfahren nach einem der Ansprüche 1 bis 22, **dadurch gekennzeichnet, dass** mit der thermischen Behandlung der ringähnlichen Vorläuferformkörper ein auf ihr Ausgangsgewicht bezogener Gewichtsverlust von 0,5 bis 40 Gew.-% einhergeht.

**24.** Verfahren nach einem der Ansprüche 1 bis 23, **dadurch gekennzeichnet, dass** das pulverförmige Haufwerk wenigstens eine Substanz aus der Gruppe bestehend aus $NH_4OH$, $(NH_4)CO_3$, $NH_4HCO_3$, $NH_4NO_3$, Harnstoff, $NH_4CHO_2$, $NH_4CH_3CO_2$, $NH_4HSO_4$, $(NH_4)_2SO_4$, Ammoniumoxalat und den Hydraten der vorgenannten Ammoniumsalze zugesetzt enthält.

**25.** Verfahren nach einem der Ansprüche 1 bis 24, **dadurch gekennzeichnet, dass** das pulverförmige Haufwerk Graphit, Stärke, gemahlene Nussschale, feinteiliges Kunststoffgranulat, Cellulose, Stearinsäure, Malonsäure, Salz der Stearinsäure und/oder Salz der Malonsäure zugesetzt enthält.

**26.** Verfahren nach einem der Ansprüche 1 bis 25, **dadurch gekennzeichnet, dass** bei dem anschließenden Verfahren der thermischen Behandlung der hergestellten ringähnlichen Vorläuferformkörper in selbigen ein Multimetalloxid ausgebildet wird, das die Elemente Mo und Fe, oder die Elemente Mo, Fe und Bi, oder die Elemente Mo und V, oder die Elemente Mo, V und P, oder die Elemente V und P enthält.

27. Verfahren nach einem der Ansprüche 1 bis 25, **dadurch gekennzeichnet, dass** bei dem anschließenden Verfahren der thermischen Behandlung der hergestellten ringähnlichen Vorläuferformkörper in selbigen ein bei Normalbedingungen festes Oxid ausgebildet wird, in welchem kein Übergangsmetall der 5. bis 11. Nebengruppe und auch nicht Phosphor dasjenige von Sauerstoff verschiedene Element ist, das molar gerechnet das numerisch am häufigsten enthaltene ist.

28. Verfahren nach einem der Ansprüche 1 bis 27, **dadurch gekennzeichnet, dass** sich bereits im Ausgangsabstand A sowohl die obere Stirnfläche des unteren Stempels als auch die untere Stirnfläche des oberen Stempels im Längsabschnitt II (32) der Matrizenbohrung befinden.

29. Verfahren der heterogen katalysierten partiellen Gasphasenoxidation wenigstens einer organischen Verbindung an einem Katalysatorfestbett, **dadurch gekennzeichnet, dass** das Katalysatorfestbett einen ringähnlichen oxidischen Formkörper, der nach einem Verfahren gemäß einen der Ansprüche 1 bis 28 erhalten wurde, enthält.

30. Rohrbündelreaktor, dessen Reaktionsrohre wenigstens einen ringähnlichen oxidischen Formkörper, der nach einem Verfahren gemäß einem der Ansprüche 1 bis 28 erhalten wurde, enthalten.


## Claims

1. A process for producing a ringlike oxidic shaped body, comprising the mechanical compaction of a pulverulent aggregate which has been introduced into the fill chamber of a die and is composed of constituents which comprise at least one metal compound which can be converted to a metal oxide by thermal treatment at a temperature of $\geq$ 100°C, or at least one metal oxide, or at least one metal oxide and at least one such metal compound, to give a ringlike shaped precursor body, in which the fill chamber is disposed in a die bore conducted through the die material from the top downward with a vertical bore axis B and is delimited by

  - the inner wall of the die bore,
  - the upper end face of a lower punch introduced from below along the bore axis B into the die bore so as to be liftable and lowerable, on which the pulverulent aggregate introduced into the fill chamber rests,
  - the lower end face, disposed along the bore axis B at an axial starting distance A above the upper end face of the lower punch, of an upper punch mounted so as to be liftable and lowerable along the bore axis B, whose lower end face is in contact with the pulverulent aggregate introduced into the fill chamber from above, and
  - the outer face of a center pin MF conducted from the bottom upward in the die bore along the bore axis B from the geometric center of the upper end face of the lower punch, said center pin MF extending at least up to the geometric center of the lower end face of the upper punch,

  by reducing the axial starting distance A of the two end faces along the bore axis B to an axial end distance E predefined for the compaction by lowering the upper punch while maintaining the position of the lower punch or additionally lifting the lower punch, where

  - the geometric shape of the outer face of the lower punch corresponds to that of the outer face of a circular cylinder I;
  - the geometric shape of the outer face of the upper punch corresponds to that of the outer face of a circular cylinder II;
  - in the geometric center of the upper end face of the lower punch, a center bore $MB^U$ (37) conducted through the lower punch from the top downward is formed;
  - at the starting distance A of the two end faces, the center pin MF (18) projects from below through the center bore $MB^U$ (37) at least up to the geometric center of the lower end face of the upper punch;
  - the center pin MF (18), from the bottom upward, has the geometric shape of a circular cylinder Z with a circular cylindrical outer face MZ;
  - the length of the outline of the circular cylinder Z is less than the length of the outline of the circular cylinder I and less than the length of the outline of the circular cylinder II;
  - the position of the center pin MF (18) and the position of the die including the die bore along the bore axis B are fixed relative to one another during the process;

- in the geometric center of the lower end face of the upper punch, a center bore $MB^O$ (35) which is conducted into the upper punch and is connected to at least one outlet from the upper punch is formed, said center bore $MB^O$ (35) being capable of accommodating the center pin MF (18) to the necessary degree in the event of reduction of the starting distance A to the end distance E, and the center pin MF (18) being able to project into it even at the starting distance A;

- the axes of symmetry of the die bore, of the circular cylinder I, of the circular cylinder II, of the center bore $MB^O$ (35), of the center pin MF (18) and of the center bore $MB^U$ (37) are on a common straight line L running vertically through the die bore;

- the die bore, along its bore axis, has a longitudinal section I (31) over whose length I the geometric shape of the inner wall of the die bore corresponds to that of the outer face of a circular cylinder KZ, and which is adjoined at its upper end directly by a longitudinal section II (32) of the die bore which is directed upward and has the length II;

- the dimensions of the longitudinal section I (31) of the die bore and of the circular cylinder I are such that the lower punch, during the process, is always conducted in each case sliding into the die bore at least for part of the length of the longitudinal section I (31) with its outer face on the inner wall of the die bore; and

- the dimensions of the center bore $MB^U$ (37) and of the circular cylinder Z are such that the lower punch, during the process, is always conducted sliding into the die bore at least in the region of the entrance of its center bore $MB^U$ (37) into its upper end face with the inner wall of the center bore $MB^U$ (37) on the circular cylindrical outer face MZ of the center pin MF (18); and

- on completion of compaction, the upper punch is lifted from the ringlike shaped precursor body formed and the ringlike shaped precursor body is removed from the die bore by lifting the lower punch,

wherein a subsequent process for thermal treatment of the ringlike shaped precursor body at a temperature of $\geq 100°C$, in which at least a portion of its constituents is decomposed and/or converted chemically to form at least one gaseous compound and the ringlike oxidic shaped body forms, and

wherein the geometric shape of the inner wall of the die bore, over the length II of the longitudinal section II (32), from the bottom upward, corresponds to that of the outer face of a frustocone KS which widens from the bottom upward, whose cross-sectional area, at its lower end, corresponds to the cross-sectional area of the circular cylinder KZ at its upper end, with the proviso that, on attainment of the end distance E, the lower end face of the upper punch is in the longitudinal section II (32) and the upper end face of the lower punch is not below the longitudinal section I (31), such that the ringlike shaped precursor body formed by the mechanical compaction of the pulverulent aggregate between the two end faces is at least partly in the longitudinal section II (32) on attainment of the end distance E.

2. The process according to claim 1, wherein, on attainment of the end distance E between the upper end face of the lower punch and the lower end face of the upper punch, at least 20% of the distance between the two end faces is within the longitudinal section II (32).

3. The process according to claim 1, wherein, on attainment of the end distance E between the upper end face of the lower punch and the lower end face of the upper punch, all of the ringlike shaped precursor body formed by the mechanical compaction of the pulverulent aggregate between the two end faces is within the longitudinal section II (32).

4. The process according to any one of claims 1 to 3, wherein the outline of the circular cylinder II is longer than or is equal in length to the outline of the circular cylinder I.

5. The process according to any one of claims 1 to 4, wherein the upper end face of the lower punch and the lower end face of the upper punch are in planes parallel to one another, to which the bore axis B is at right angles.

6. The process according to any one of claims 1 to 5, wherein the end distance E is from 2 to 10 mm.

7. The process according to any one of claims 1 to 6, wherein the quotient Q of the length of the outline of the circular cylinder Z as the numerator and the outline of the circular cylinder I as the denominator is from 0.3 to 0.7.

8. The process according to any one of claims 1 to 7, wherein the difference formed by subtracting the radius of the outline of the circular cylinder Z from the radius of the outline of the circular cylinder I is from 1 to 3 mm.

9. The process according to any one of claims 1 to 8, wherein the diameter of the outline of the circular cylinder I is from 2 to 10 mm.

**10.** The process according to any one of claims 1 to 9, wherein the frustocone KS is such that the following relationship between the diameter DD of the top face, the diameter DG of the bottom face and the height H of the frustocone KS is satisfied:

$$0.003 \cdot H \leq DG - DD \leq 0.050 \cdot H.$$

**11.** The process according to any one of claims 1 to 10, wherein both the upper end face of the lower punch and the lower end face of the upper punch have the geometric shape of a circular ring.

**12.** The process according to any one of claims 1 to 10, wherein the die bore of the die is such that its longitudinal section I (31) is not only adjoined at its upper end directly by a longitudinal section II (32) directed upward, but also at its lower end directly by a longitudinal section II* (33) of length II* directed downward, and the geometric shape of the inner wall of the die bore, over the length II* of the longitudinal section II* (33), corresponds to the outer face of a frustocone KS* whose cross-sectional area at its upper end corresponds to the cross-sectional area of the circular cylinder KZ at its lower end.

**13.** The process according to any one of claims 1 to 12, wherein the following relationship between the height H of the frustocone KS and the end distance E is satisfied:

$$4 \cdot \text{end distance } E \geq H \geq 1 \cdot \text{end distance } E.$$

**14.** The process according to any one of claims 1 to 13, wherein the length of longitudinal section I (31) is not more than three times and not less than 0.1 times the length of longitudinal section II (32).

**15.** The process according to any one of claims 1 to 14, wherein at least the entrance into the center bore $MB^O$ (35) is configured in circular cylindrical form such that the outer face of the circular cylinder Z, when it is accommodated into the center bore $MB^O$ (35), slides along its inner wall at least in the entrance region thereof.

**16.** The process according to any one of claims 1 to 15, wherein the mechanical compaction consists of a preliminary compaction and of a main compaction following thereafter, the axial starting distance A, in the course of the preliminary compaction, first being reduced to a preliminary end distance $E^V$, and the preliminary end distance $E^V$ being reduced to the end distance E in the course of the main compaction.

**17.** The process according to any one of claims 1 to 16, wherein the pulverulent aggregate comprises at least one metal oxide, metal hydroxide, metal carbonate, metal hydrogencarbonate, metal hydrogenphosphate and/or metal nitrate.

**18.** The process according to any one of claims 1 to 17, wherein the die is manufactured from a material composite which consists of a cemented carbide on its side in contact with the die bore and of a tool steel on its side facing away from the die bore, said tool steel having the following element composition:

| | |
|---|---|
| 1.50 to 1.80% by wt. of | C, |
| 0.10 to 0.40% by wt. of | Si, |
| 0.10 to 0.50% by wt. of | Mn, |
| $\geq 0$ to 0.05% by wt. of | P, |
| $\geq 0$ to 0.05% by wt. of | S, |
| 10 to 13% by wt. of | Cr, |
| 0.50 to 0.80% by wt. of | Mo, |
| 0.10 to 1.10% by wt. of | V, |
| $\geq 0$ to 0.60% by wt. of | W, and |
| $\geq 0$ to 0.10% by wt. of | one or more rare earth metals, and apart from these Fe and impurities resulting from the production. |

19. The process according to claim 18, wherein the cemented carbide consists of tungsten carbide to an extent of $\geq$ 90% by weight and of nickel or of nickel and chromium to an extent of at least 5% by weight.

20. The process according to any one of claims 1 to 19, wherein the pulverulent aggregate comprises nitric acid, an ammonium salt and/or a nitrate salt.

21. The process according to any one of claims 1 to 20, wherein, at the end distance E, the two punches exert a pressure which is in the range from 50 to 5000 kg/cm$^2$.

22. The process according to any one of claims 1 to 21, wherein the process for thermal treatment of the ringlike shaped precursor bodies produced is effected at a temperature of $\geq$ 200°C.

23. The process according to any one of claims 1 to 22, wherein the thermal treatment of the ringlike shaped precursor bodies is accompanied by a weight loss of from 0.5 to 40% by weight based on its starting weight.

24. The process according to any one of claims 1 to 23, wherein the pulverulent aggregate comprises at least one added substance from the group consisting of $NH_4OH$, $(NH_4)_2CO_3$, $NH_4HCO_3$, $NH_4NO_3$, urea, $NH_4CHO_2$, $NH_4CH_3CO_2$, $NH_4HSO_4$, $(NH_4)_2SO_4$, ammonium oxalate and the hydrates of the aforementioned ammonium salts.

25. The process according to any one of claims 1 to 24, wherein the pulverulent aggregate comprises added graphite, starch, ground nutshell, fine polymer granule, cellulose, stearic acid, malonic acid, salt of stearic acid and/or salt of malonic acid.

26. The process according to any one of claims 1 to 25, wherein the subsequent process for thermal treatment of the ringlike shaped precursor bodies produced forms a multimetal oxide therein which comprises the elements Mo and Fe, or the elements Mo, Fe and Bi, or the elements Mo and V, or the elements Mo, V and P, or the elements V and P.

27. The process according to any one of claims 1 to 25, wherein the subsequent process for thermal treatment of the ringlike shaped precursor bodies produced forms an oxide therein which is solid under standard conditions and in which no transition metal of transition group 5 to 11 nor phosphorus is that element other than oxygen which, calculated in molar terms, is the numerically most common.

28. The process according to any one of claims 1 to 27, wherein, even at the starting distance A, both the upper end face of the lower punch and the lower end face of the upper punch are in the longitudinal section II (32) of the die bore.

29. A process for heterogeneously catalyzed partial gas phase oxidation of at least one organic compound over a fixed catalyst bed, wherein the fixed catalyst bed comprises a ringlike oxidic shaped body which has been obtained by a process according to any of claims 1 to 28.

30. A shell and tube reactor whose reaction tubes comprise at least one ringlike oxidic shaped body which has been obtained by a process according to any of claims 1 to 28.

**Revendications**

1. Procédé de fabrication d'un objet moulé annulaire de type oxyde,

comprenant la compression mécanique d'un mélange pulvérulent introduit dans la chambre de remplissage d'une matrice, constitué de constituants qui comprennent au moins un composé métallique, qui par traitement thermique à une température $\geq$ 100 °C peut être converti en un oxyde métallique, ou au moins un oxyde métallique, ou au moins un oxyde métallique et au moins un composé métallique de ce type, pour obtenir un précurseur annulaire d'objet moulé, procédé dans lequel la chambre de chargement se trouve dans un alésage de matrice, qui, par un axe d'alésage vertical B, est traversé de haut en bas par le matériau de matrice, et qui est limité par

- la paroi intérieure de l'alésage de matrice,
- la face frontale supérieure d'un poinçon inférieur, inséré par en bas, le long de l'axe d'alésage B, dans l'alésage de matrice par un déplacement ascendant et descendant, poinçon sur lequel est disposé le mélange pulvérulent introduit dans la chambre de remplissage,

- la face frontale inférieure, se trouvant le long de l'axe d'alésage B à une distance initiale axiale A au-dessus de la surface frontale supérieure du poinçon inférieur, d'un poinçon supérieur disposé le long de l'axe d'alésage B et pouvant subir un déplacement ascendant et descendant, poinçon dont la surface frontale inférieure est en contact par en haut avec le mélange pulvérulent introduit dans la chambre de remplissage, et

- la surface latérale d'une tige centrale MF, guidée de bas en haut dans l'alésage de matrice le long de l'axe d'alésage B, à partir du milieu géométrique de la surface frontale du poinçon inférieur, tige centrale qui s'étend jusqu'au milieu géométrique de la surface frontale inférieure du poinçon supérieur,

dans lequel on diminue la distance axiale initiale A des deux surfaces frontales à une distance axiale finale E, prédéfinie pour la compression, le long de l'axe d'alésage B, par le fait que l'on abaisse le poinçon supérieur et, à cette occasion, on maintient la position du tampon inférieur ou on élève en outre le tampon inférieur, dans lequel

- la forme géométrique de la surface latérale du poinçon inférieur correspond à celle de la surface latérale d'un cylindre circulaire I ;

- la forme géométrique de la surface latérale du poinçon supérieur correspond à celle de la surface latérale d'un cylindre circulaire II ;

- un alésage central $MB^U$ (37), passant de haut en bas à travers le poinçon inférieur, est réalisé au milieu géométrique de la surface frontale supérieure du poinçon inférieur ;

- à la distance initiale A des deux surfaces frontales, la tige centrale MF (18) dépasse vers le haut, à partir du bas, en passant par l'alésage central $MB^U$ (37) au moins jusqu'au milieu géométrique de la surface frontale inférieure du poinçon supérieur ;

- la tige centrale MF (18) présente, de bas en haut, la forme géométrique d'un cylindre circulaire Z ayant une surface latérale cylindrique circulaire MZ ;

- la longueur du périmètre du cylindre circulaire Z est inférieure à la longueur du périmètre du cylindre circulaire I, et aussi inférieure à la longueur du périmètre du cylindre circulaire II ;

- la position de la tige centrale MF (18) et la position de la matrice, y compris de l'alésage de matrice le long de l'axe d'alésage B, sont, au cours de l'opération, fixées l'une par rapport à l'autre ;

- un alésage central $MB^O$ (35), traversant le poinçon supérieur et en liaison avec au moins un orifice de sortie du poinçon supérieur, est réalisé au milieu géométrique de la face frontale inférieure du poinçon supérieur, alésage central qui peut recevoir la tige centrale MF (18) lors de la diminution, dans la mesure nécessaire, de la distance initiale A pour la faire arriver à la distance finale E, et dans lequel la tige centrale MF (18) peut pénétrer, déjà à la distance initiale A ;

- les axes de symétrie de l'alésage de matrice, du cylindre circulaire I, du cylindre circulaire II, de l'alésage central $MB^O$ (35), de la tige centrale MF (18) et de l'alésage central $MB^U$ (37) se trouvent sur une droite commune L, traversant verticalement l'alésage de matrice ;

- l'alésage de matrice présente le long de son axe d'alésage un segment longitudinal I (31), sur la longueur I duquel la forme géométrique de la paroi intérieure de l'alésage de matrice correspond à celle de la surface latérale d'un cylindre circulaire KZ, et dont l'extrémité supérieure se poursuit directement par un segment longitudinal II (32), dirigé vers le haut, de l'alésage de matrice, qui présente la longueur II ;

- les dimensions du segment longitudinal I (31) de l'alésage de matrice et du cylindre circulaire I sont telles que le poinçon inférieur, pendant l'opération, est toujours guidé en glissement dans l'alésage de matrice, par sa surface latérale, sur la paroi intérieure de l'alésage de matrice, au moins sur une longueur partielle du segment longitudinal I (31) ; et

- les dimensions de l'alésage central $MB^U$ (37) et du cylindre circulaire Z sont telles que le poinçon inférieur est guidé en glissement dans l'alésage de matrice, par la paroi intérieure de l'alésage central $MB^U$ (37) sur la surface latérale MZ cylindrique circulaire de la tige centrale MF (18), au moins dans la zone de l'entrée de son alésage central $MB^U$ (37) dans sa surface frontale supérieure ; et

- après la fin de la compression, le poinçon supérieur est abaissé, à partir du précurseur annulaire ainsi formé de l'objet moulé, et le précurseur annulaire de l'objet moulé est enlevé de l'alésage de matrice par soulèvement du poinçon inférieur,

**caractérisé en ce qu'**une opération effectuée ensuite pour le traitement thermique du précurseur annulaire de l'objet moulé à une température $\geq$ 100 °C, à laquelle au moins une quantité partielle de ces constituants se décompose et/ou subit une réaction chimique avec formation d'au moins un composé gazeux, l'objet moulé annulaire de type oxyde étant alors forme, et

**EP 2 307 192 B1**

**en ce que** la forme géométrique de la paroi intérieure de l'alésage de matrice, sur la longueur II du segment longitudinal II (32) de bas en haut, correspond à celle de la surface latérale d'un tronc de cône KS, s'élargissant de bas en haut, dont l'aire en section transversale correspond, en son extrémité inférieure, à l'aire en section transversale du cylindre circulaire KZ au niveau de son extrémité inférieure, à la condition que, lorsque la distance finale E est atteinte, la surface frontale inférieure du poinçon supérieur se trouve dans le segment longitudinal II (32) et que la surface frontale supérieure du poinçon inférieur ne se trouve pas en dessous du segment longitudinal I (31), de sorte que le précurseur annulaire de l'objet moulé, réalisé par la compression mécanique du mélange pulvérulent entre les deux surfaces frontales, se trouve, quand la distance finale E est atteinte, au moins partiellement dans le segment longitudinal II (32).

2. Procédé selon la revendication 1, **caractérisé en ce que**, lorsque la distance finale E est atteinte, au moins 20 % de la distance entre les deux faces frontales se trouvent dans le segment longitudinal II (32) entre la face frontale supérieure du poinçon inférieur et la surface frontale inférieure du poinçon supérieur.

3. Procédé selon la revendication 1, **caractérisé en ce que**, lorsque la distance finale E est atteinte, le précurseur annulaire de l'objet moulé, réalisé dans sa totalité par la compression mécanique du mélange pulvérulent entre les deux faces frontales, se trouve dans le segment longitudinal II (32) entre la face frontale supérieure du poinçon inférieur et la surface frontale inférieure du poinçon supérieur.

4. Procédé selon l'une des revendications 1 à 3, **caractérisé en ce que** le périmètre du cylindre circulaire II est plus long, ou a la même longueur que le périmètre du cylindre circulaire I.

5. Procédé selon l'une des revendications 1 à 4, **caractérisé en ce que** la surface frontale supérieure du poinçon inférieur et la surface frontale inférieure du poinçon supérieur se trouvent dans des plans parallèles l'un à l'autre, auxquels l'axe d'alésage B est perpendiculaire.

6. Procédé selon l'une des revendications 1 à 5, **caractérisé en ce que** la distance finale E est de 2 à 10 mm.

7. Procédé selon l'une des revendications 1 à 6, **caractérisé en ce que** le quotient Q de la longueur du périmètre du cylindre circulaire Z, considéré comme un numérateur, et du périmètre du cylindre circulaire I, considéré comme un dénominateur, est de 0,3 à 0,7.

8. Procédé selon l'une des revendications 1 à 7, **caractérisé en ce que** la différence obtenue en soustrayant le rayon du périmètre du cylindre circulaire Z du rayon du périmètre du cylindre circulaire I est de 1 à 3 mm.

9. Procédé selon l'une des revendications 1 à 8, **caractérisé en ce que** le diamètre du périmètre du cylindre circulaire I est de 2 à 10 mm.

10. Procédé selon l'une des revendications 1 à 9, **caractérisé en ce que** le tronc de cône KS est tel que, entre le diamètre DD de la surface supérieure, le diamètre DG de la surface inférieure et la hauteur H du tronc de cône KS, on ait la relation suivante :

$$0{,}003 \ H \leq DG - DD \leq 0{,}050 \ H.$$

11. Procédé selon l'une des revendications 1 à 10, **caractérisé en ce que** tant la surface frontale supérieure du poinçon inférieur que la surface frontale inférieure du poinçon supérieur présentent la forme géométrique d'un anneau circulaire.

12. Procédé selon l'une des revendications 1 à 10, **caractérisé en ce que** l'alésage de la matrice est tel que son segment longitudinal I (31) se poursuit, pas seulement au niveau de son extrémité supérieure, directement par un segment longitudinal II (32) dirigé vers le haut, mais aussi, en son extrémité inférieure, directement par un segment longitudinal II* (33) dirigé vers le bas et ayant la longueur II*, et la forme géométrique de la paroi intérieure de l'alésage de matrice, sur la longueur II* du segment longitudinal II* (33), correspond à la surface latérale d'un tronc de cône KS*, dont l'aire en section transversale correspond, au niveau de son extrémité supérieure, à l'aire en section transversale du cylindre circulaire KZ au niveau de son extrémité inférieure.

13. Procédé selon l'une des revendications 1 à 12, **caractérisé en ce que**, entre la hauteur H du tronc de cône KS et la distance finale E, on a la relation suivante :

$$4 \cdot \text{distance finale } E \geq H \geq 1 \cdot \text{distance finale } E.$$

14. Procédé selon l'une des revendications 1 à 13, **caractérisé en ce que** la longueur du segment longitudinal I (31) n'est pas supérieure au triple et n'est pas inférieure à 0,1 fois la longueur du segment longitudinal II (32).

15. Procédé selon l'une des revendications 1 à 14, **caractérisé en ce qu'**au moins l'entrée dans l'alésage central $MB^O$ (35) est conçue, d'une manière cylindrique circulaire, de telle sorte que la surface latérale du cylindre circulaire Z, lorsqu'il est logé dans l'alésage central $MB^O$ (35), glisse au moins dans sa zone d'entrée sur sa paroi intérieure.

16. Procédé selon l'une des revendications 1 à 15, **caractérisé en ce que** la compression mécanique est constituée d'une compression préalable et d'une compression principale suivant cette dernière, la distance initiale axiale A diminuant d'abord, dans le cadre de la compression préalable, jusqu'à une distance finale provisoire $E^V$ et, dans le cadre de la compression principale, la distance finale provisoire $E^V$ diminue jusqu'à la distance finale E.

17. Procédé selon l'une des revendications 1 à 16, **caractérisé en ce que** le mélange pulvérulent comprend au moins un oxyde métallique, un hydroxyde métallique, un carbonate métallique, un hydrogénocarbonate métallique, un hydrogénophosphate métallique et/ou un nitrate métallique.

18. Procédé selon l'une des revendications 1 à 17, **caractérisé en ce que** la matrice est fabriquée en un matériau composite, qui sur sa face en contact avec l'alésage de la matrice est constitué d'un métal dur et, sur sa face opposée à l'alésage de la matrice, d'un acier à outil, qui présente la composition élémentaire suivante :

| | |
|---|---|
| 1,50 à 1,80 % en poids | C, |
| 0,10 à 0,40 % en poids | Si, |
| 0,10 à 0,50 % en poids | Mn, |
| $\geq 0$ à 0,05 % en poids | P, |
| $\geq 0$ à 0,05 % en poids | S, |
| 10 à 13 % en poids | Cr, |
| 0,50 à 0,80 % en poids | Mo, |
| 0,10 à 1,10 % en poids | V, |
| $\geq 0$ à 0,60 % en poids | W, et |
| $\geq 0$ à 0,10 % en poids | d'un ou plusieurs métaux terreux, le reste étant constitué de Fe et des impuretés dues au procédé de fabrication. |

19. Procédé selon la revendication 18, **caractérisé en ce que** le métal dur est constitué à raison de $\geq 90$ % en poids de carbure de tungstène et à raison d'au moins 5 % en poids de nickel, ou de nickel et de chrome.

20. Procédé selon l'une des revendications 1 à 19, **caractérisé en ce que** le mélange pulvérulent contient de l'acide nitrique, un sel d'ammonium et/ou un sel nitrate.

21. Procédé selon l'une des revendications 1 à 20, **caractérisé en ce que**, à la distance finale E des deux poinçons, une pression de compression est exercée, qui est comprise dans la plage de 50 à 5 000 kg/cm².

22. Procédé selon l'une des revendications 1 à 21, **caractérisé en ce que** l'opération de traitement thermique des précurseurs annulaires fabriqués de l'objet moulé est réalisée à une température $\geq 200$ °C.

23. Procédé selon l'une des revendications 1 à 22, **caractérisé en ce que** le traitement thermique du précurseur annulaire de l'objet moulé provoque une perte de poids, par rapport au poids initial, de 0,5 à 40 % en poids.

24. Procédé selon l'une des revendications 1 à 23, **caractérisé en ce que** le mélange pulvérulent contient, ajoutée, une substance du groupe consistant en $NH_4OH$, $(NH_4)_2CO_3$, $NH_4HCO_3$, $NH_4NO_3$, urée, $NH_4CHO_2$, $NH_4CH_3CO_2$, $NH_4HSO_4$, $(NH_4)_2SO_4$, oxalate d'ammonium, et les hydrates des sels d'ammonium mentionnés ci-dessus.

**25.** Procédé selon l'une des revendications 1 à 24, **caractérisé en ce que** le mélange pulvérulent contient, ajoutés, du graphite, de l'amidon, de la coque de noix broyée, un granulé plastique finement divisé, de la cellulose, de l'acide stéarique, de l'acide malonique, un sel de l'acide stéarique et/ou un sel de l'acide malonique.

**26.** Procédé selon l'une des revendications 1 à 25, **caractérisé en ce que**, lors de l'opération ultérieure de traitement thermique des précurseurs annulaires fabriqués de l'objet moulé, il se forme, dans ce dernier, un oxyde multi-métallique, qui contient les éléments Mo et Fe, ou les éléments Mo, Fe et Bi, ou les éléments Mo et V, ou les éléments Mo, V et P, ou les éléments V et P.

**27.** Procédé selon l'une des revendications 1 à 25, **caractérisé en ce que**, lors du traitement ultérieur de traitement thermique des précurseurs annulaires fabriqués de l'objet moulé, il se forme dans ce dernier un oxyde solide dans les conditions ambiantes, dans lequel aucun métal de transition du 5e au 11e groupe, ni du phosphore, n'est l'élément différent de l'oxygène, qui en moles est numériquement présent en la plus grande quantité.

**28.** Procédé selon l'une des revendications 1 à 27, **caractérisé en ce que**, déjà à la distance initiale A, tant la surface frontale supérieure du poinçon inférieur que la surface frontale inférieure du poinçon supérieur se trouvent dans le segment longitudinal II (32) de l'alésage de matrice.

**29.** Procédé d'oxydation partielle en phase gazeuse, catalysé par un catalyseur hétérogène, d'au moins un composé organique sur un lit fixe de catalyseur, **caractérisé en ce que** le lit fixe de catalyseur contient un objet moulé annulaire de type oxyde, qui a été obtenu par un procédé selon l'une des revendications 1 à 28.

**30.** Réacteur tubulaire, dont les tubes de réaction contiennent au moins un objet moulé annulaire de type oxyde, qui a été obtenu par un procédé selon l'une des revendications 1 à 28.

Fig. 1

Fig. 2a

Fig. 2b

Fig. 3a

Fig. 3b

14

34

4

35

13

Fig. 4a

Fig. 4b

14

4

34

13

35

Fig. 4c

Fig. 4d

Fig. 4e

Fig. 4f

Fig. 5a

Fig. 5b

Fig. 5c

Fig. 5d

37

12

5

Fig. 5e

Fig. 6

18

27

Fig. 7

—30

—18

—27

Fig. 8

Figur 9

Figur 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 184790 A
- US 20050263926 A
- JP 10029097 A
- US 2005263926 A1
- US 7147011 B
- DE 102007028332
- DE 19714430 A
- DE 102007017080
- DE 2624853 A
- DE 19733969 A
- DE 2435777 A
- AT 358833
- EP 1364732 A
- AT 362943
- WO 2005115733 A
- DE 102005037678 A
- DE 102007025869
- DE 102007003778
- WO 2005030393 A
- EP 467144 A
- EP 1060792 A
- DE 19855913 A
- WO 0168245 A
- DE 102005035978 A
- WO 0378059 A
- WO 03078310 A
- DE 19922113 A
- WO 0224620 A
- WO 02062737 A
- US 20050131253 A
- DE 102007003778 A
- DE 102007028332 A
- DE 10046957 A
- DE 4407020 A
- EP 835 A
- EP 575897 A
- DE 3338380 C
- DE 10325487 A
- WO 0053557 A
- WO 0053558 A
- DE 19910506 A
- EP 1106598 A

- WO 0136364 A
- DE 19927624 A
- DE 19948248 A
- DE 19948523 A
- DE 19948241 A
- EP 700714 A
- DE 10313213 A
- DE 10313209 A
- DE 10232748 A
- DE 10313208 A
- WO 03039744 A
- EP 279374 A
- DE 3338380 A
- DE 3300044 A
- DE 102004003212 A
- DE 102005013039 A
- DE 102005009891 A
- DE 102005010111 A
- DE 102005009885 A
- DE 19746210 A
- EP 015565 A
- EP 962253 A
- DE 10122027 A
- EP 608838 A
- DE 19835247 A
- EP 895809 A
- EP 1254709 A
- EP 1192987 A
- EP 1262235 A
- EP 1193240 A
- JP 11343261 A
- JP 11343262 A
- EP 1090684 A
- EP 1301457 A
- EP 1254707 A
- EP 1335793 A
- DE 10046672 A
- DE 10034825 A
- EP 1556337 A
- DE 10033121 A
- WO 0198246 A
- EP 1558569 A
- WO 9948606 A
- EP 2008050341 W

**In der Beschreibung aufgeführte Nicht-Patentliteratur**

- Tabellenbuch Metall. Verlag Europa Lehrmittel, 1999

- **FRANK HEBNER.** Ermüdungsverhalten des Hartmetalls G55 Co bei Raumtemperatur. Friedrich-Alexander-Universität, 07. September 2003